# EUROPEAN PATENT APPLICATION

(11) **EP 3 995 178 A1**
(43) Date of publication of application: **11.05.2022**
(21) Application number: 20206545.4
(22) Date of filing: 09.11.2020
(51) Int. Cl.: A61P 35/04, C12Q 1/6809, C12Q 1/6881, G01N 33/50

(54) **METHOD**

(71) Applicant: Université de Genève, 1211 Genève (CH)
(72) Inventor: The designation of the inventor has not yet been filed
(74) Representative: J A Kemp LLP

(57) **Abstract**

The present invention relates to novel methods for identifying a cluster of cells associated with a phenotype. The invention also relates to methods for preventing cancer metastasis. The invention also relates to methods for determining the efficacy of a medicament. The invention also relates to a heterogeneous population of cells spiked with a phenotypically characterised and tagged single cell.

SEQUENCE LISTING

<110>  Université de Genève

<120>  METHOD

<130>  N420497EP

<140>  tbc
<141>  2020-11-09

<160>  122

<170>  PatentIn version 3.5

<210>  1
<211>  20
<212>  DNA
<213>  Artificial Sequence

<220>
<223>  XRCC5 fw

<400>  1
cagagttggg ctgtccgatt                                                   20


<210>  2
<211>  20
<212>  DNA
<213>  Artificial Sequence

<220>
<223>  XRCC5 rv

<400>  2
cctagcccca accaacaact                                                   20


<210>  3
<211>  19
<212>  DNA
<213>  Artificial Sequence

<220>
<223>  GBF1 fw

<400>  3
ctgcagtcag aggcgatcc                                                    19


<210>  4
<211>  23
<212>  DNA
<213>  Artificial Sequence

<220>
<223>  GBF1 rv

<400>  4
gactcttttt gataaagctg ggc                                               23

<210>  5
<211>  20
<212>  DNA
<213>  Artificial Sequence

<220>
<223>  GDF11fw

<400>  5
cttatcagcg gacccagcag                                                   20


<210>  6
<211>  21
<212>  DNA
<213>  Artificial Sequence

<220>
<223>  GDF11 rv

<400>  6
tatctcctca gggccaggtt a                                                 21


<210>  7
<211>  20
<212>  DNA
<213>  Artificial Sequence

<220>
<223>  AXL fw

<400>  7
cttgcatgca gtgaacagcc                                                   20


<210>  8
<211>  21
<212>  DNA
<213>  Artificial Sequence

<220>
<223>  AXL rv

<400>  8
acacatgtgc acgtaactca c                                                 21


<210>  9
<211>  22
<212>  DNA
<213>  Artificial Sequence

<220>
<223>  NEO1fw

<400>  9
tttttgtagg agccagcatt cg                                                22


<210>  10
<211>  22
<212>  DNA

<213>  Artificial Sequence

<220>
<223>  NEO1 rv

<400>  10
aacagtggcc acacactgat aa                                                22


<210>  11
<211>  20
<212>  DNA
<213>  Artificial Sequence

<220>
<223>  ROR2 fw

<400>  11
ctcacattgc tcactgggct                                                   20


<210>  12
<211>  20
<212>  DNA
<213>  Artificial Sequence

<220>
<223>  ROR2 rv

<400>  12
cggcaagttc tccatcgact                                                   20


<210>  13
<211>  25
<212>  DNA
<213>  Artificial Sequence

<220>
<223>  DNAH5 fw

<400>  13
cacagggaat aaaaggagag aaact                                             25


<210>  14
<211>  20
<212>  DNA
<213>  Artificial Sequence

<220>
<223>  DNAH5 rv

<400>  14
ttgcctaacc cagcctacac                                                   20


<210>  15
<211>  20
<212>  DNA
<213>  Artificial Sequence

<220>

<223>  CH9 fw

<400>  15
tccttcagca cacttccaca                                                    20


<210>  16
<211>  22
<212>  DNA
<213>  Artificial Sequence

<220>
<223>  CH9 rv

<400>  16
tggggtttag gatatgctga gt                                                 22


<210>  17
<211>  20
<212>  DNA
<213>  Artificial Sequence

<220>
<223>  AKAP12 FW

<400>  17
ccgctaagct gatctcctgt                                                    20


<210>  18
<211>  24
<212>  DNA
<213>  Artificial Sequence

<220>
<223>  AKAP12 RV

<400>  18
catcttcaga gtctctctgt ccaa                                               24


<210>  19
<211>  23
<212>  DNA
<213>  Artificial Sequence

<220>
<223>  CAV1 FW

<400>  19
caggcttgta acctttacag gac                                                23


<210>  20
<211>  23
<212>  DNA
<213>  Artificial Sequence

<220>
<223>  CAV1 RV

<400>  20

catagatgct tagtccctca tgc                                                23

<210>  21
<211>  20
<212>  DNA
<213>  Artificial Sequence

<220>
<223>  GLI1 FW

<400>  21
agcgtgagcc tgaatctgtg                                                     20

<210>  22
<211>  21
<212>  DNA
<213>  Artificial Sequence

<220>
<223>  GLI1 RV

<400>  22
cagcatgtac tgggctttga a                                                   21

<210>  23
<211>  23
<212>  DNA
<213>  Artificial Sequence

<220>
<223>  IL18 FW

<400>  23
tcttcattga ccaaggaaat cgg                                                 23

<210>  24
<211>  21
<212>  DNA
<213>  Artificial Sequence

<220>
<223>  IL18 RV

<400>  24
   tccggggtgc attatctcta c                                                    21

<210>  25
<211>  21
<212>  DNA
<213>  Artificial Sequence

<220>
<223>  TGFB2 FW

<400>  25
cagcacactc gatatggacc a                                                   21

<210>  26
<211>  20
<212>  DNA
<213>  Artificial Sequence

<220>
<223>  TGFB2 RV

<400>  26
cctcgggctc aggatagtct                                                    20


<210>  27
<211>  18
<212>  DNA
<213>  Artificial Sequence

<220>
<223>  CAV2 FW

<400>  27
gccctctttg aaatcagc                                                      18


<210>  28
<211>  20
<212>  DNA
<213>  Artificial Sequence

<220>
<223>  CAV2 RV

<400>  28
caagtattca atcctggctc                                                    20


<210>  29
<211>  22
<212>  DNA
<213>  Artificial Sequence

<220>
<223>  CSAG1 FW

<400>  29
acactggtct ggtgaagatg tc                                                 22


<210>  30
<211>  20
<212>  DNA
<213>  Artificial Sequence

<220>
<223>  CSAG1 RV

<400>  30
tgttgatggg tggttgttgg                                                    20


<210>  31
<211>  21
<212>  DNA

<213>  Artificial Sequence

<220>
<223>  MAGEA2 FW

<400>  31
tccagcaacc aagaagagga g                                                21


<210>  32
<211>  20
<212>  DNA
<213>  Artificial Sequence

<220>
<223>  MAGEA2 RV

<400>  32
tgattgctgc ttggaactcg                                                  20


<210>  33
<211>  23
<212>  DNA
<213>  Artificial Sequence

<220>
<223>  MAGEA12 FW

<400>  33
gtggtcctaa gatctaccaa gca                                              23


<210>  34
<211>  19
<212>  DNA
<213>  Artificial Sequence

<220>
<223>  MAGEA12 RV

<400>  34
agggcagcag gtaggagtg                                                   19


<210>  35
<211>  20
<212>  DNA
<213>  Artificial Sequence

<220>
<223>  LGR5 FW

<400>  35
ggagcattca ctggccttta                                                  20


<210>  36
<211>  20
<212>  DNA
<213>  Artificial Sequence

<220>

<223>  LGR5 RV

<400>  36
ctggacgggg atttctgtta                                                    20


<210>  37
<211>  20
<212>  DNA
<213>  Artificial Sequence

<220>
<223>  MAGEA3 FW

<400>  37
   agaaatgctg gggagtgtcg                                                     20


<210>  38
<211>  20
<212>  DNA
<213>  Artificial Sequence

<220>
<223>  MAGEA3 RV

<400>  38
aagaccagct gcaaggaact                                                    20


<210>  39
<211>  20
<212>  DNA
<213>  Artificial Sequence

<220>
<223>  IL8 FW

<400>  39
gtgcagtttt gccaaggagt                                                    20


<210>  40
<211>  20
<212>  DNA
<213>  Artificial Sequence

<220>
<223>  IL8 RV

<400>  40
ctctgcaccc agttttcctt                                                    20


<210>  41
<211>  22
<212>  DNA
<213>  Artificial Sequence

<220>
<223>  SCEL FW

<400>  41

gtgctcaacc gacataattc cc                                                22

<210>  42
<211>  23
<212>  DNA
<213>  Artificial Sequence

<220>
<223>  SCEL RV

<400>  42
tgtcatcaga actgtaccga cta                                               23

<210>  43
<211>  19
<212>  DNA
<213>  Artificial Sequence

<220>
<223>  SDCBP2 FW

<400>  43
ggggctcttt gtgcagttg                                                    19

<210>  44
<211>  19
<212>  DNA
<213>  Artificial Sequence

<220>
<223>  SDCBP2 RV

<400>  44
agtcacgccc gtcaatctg                                                    19

<210>  45
<211>  19
<212>  DNA
<213>  Artificial Sequence

<220>
<223>  ADM2 FW

<400>  45
ctgagcccca tctgaagcc                                                    19

<210>  46
<211>  20
<212>  DNA
<213>  Artificial Sequence

<220>
<223>  ADM2 RV

<400>  46
cagcactgcg tgtagaccag                                                   20

<210>  47
<211>  21
<212>  DNA
<213>  Artificial Sequence

<220>
<223>  RBPM2 FW

<400>  47
aagacagcct gttggttttg t                                                21


<210>  48
<211>  20
<212>  DNA
<213>  Artificial Sequence

<220>
<223>  RBPM2 RV

<400>  48
cgaataccgt tcagcgcatt                                                  20


<210>  49
<211>  21
<212>  DNA
<213>  Artificial Sequence

<220>
<223>  sXBP1 FW

<400>  49
ctgagtccga atcaggtgca g                                                21


<210>  50
<211>  21
<212>  DNA
<213>  Artificial Sequence

<220>
<223>  sXBP1 RV

<400>  50
atccatgggg agatgttctg g                                                21


<210>  51
<211>  20
<212>  DNA
<213>  Artificial Sequence

<220>
<223>  IL32 FW

<400>  51
gaagactgcg tgcagaaggt                                                  20


<210>  52
<211>  18
<212>  DNA

<213>  Artificial Sequence

<220>
<223>  IL32RV

<400>  52
ctctgccagg ctcgacat                                                    18


<210>  53
<211>  21
<212>  DNA
<213>  Artificial Sequence

<220>
<223>  EREG FW

<400>  53
ttgtttgcat ggacagtgca t                                                21


<210>  54
<211>  23
<212>  DNA
<213>  Artificial Sequence

<220>
<223>  EREG RV

<400>  54
cccacttcac acctgcagta gtt                                              23


<210>  55
<211>  21
<212>  DNA
<213>  Artificial Sequence

<220>
<223>  ANXA13 FW

<400>  55
gctaaagcga gcagtcctca g                                                21


<210>  56
<211>  22
<212>  DNA
<213>  Artificial Sequence

<220>
<223>  ANXA13 RV

<400>  56
gtcctgcccg ataagatttc aa                                               22


<210>  57
<211>  21
<212>  DNA
<213>  Artificial Sequence

<220>

<223>  VGLL1 FW

<400>  57
ccaaaggcaa acagaagcct a                                                21


<210>  58
<211>  22
<212>  DNA
<213>  Artificial Sequence

<220>
<223>  VGLL1 RV

<400>  58
catcacacct tcactctgac tc                                               22


<210>  59
<211>  18
<212>  DNA
<213>  Artificial Sequence

<220>
<223>  WWTR1 FW

<400>  59
attcgaatgc gccaagag                                                    18


<210>  60
<211>  20
<212>  DNA
<213>  Artificial Sequence

<220>
<223>  WWTR1 RV

<400>  60
aactggggca agagtctcag                                                  20


<210>  61
<211>  20
<212>  DNA
<213>  Artificial Sequence

<220>
<223>  HSPA5 FW

<400>  61
catcacgccg tcctatgtcg                                                  20


<210>  62
<211>  20
<212>  DNA
<213>  Artificial Sequence

<220>
<223>  HSPA5 RV

<400>  62

cgtcaaagac cgtgttctcg                                                    20

<210>  63
<211>  20
<212>  DNA
<213>  Artificial Sequence

<220>
<223>  DNAJC15 FW

<400>  63
ttgcaggtcg ctacgcattt                                                    20

<210>  64
<211>  22
<212>  DNA
<213>  Artificial Sequence

<220>
<223>  DNAJC15 RV

<400>  64
ccagcttctc gcctactcat tt                                                 22

<210>  65
<211>  20
<212>  DNA
<213>  Artificial Sequence

<220>
<223>  BGN FW

<400>  65
acacaccgga cagatagacg                                                    20

<210>  66
<211>  20
<212>  DNA
<213>  Artificial Sequence

<220>
<223>  BGN RV

<400>  66
cagggtgaag tcccagaagc                                                    20

<210>  67
<211>  20
<212>  DNA
<213>  Artificial Sequence

<220>
<223>  VSIG1 FW

<400>  67
agcagataag ccaggcaaac                                                    20

<210>  68
<211>  20
<212>  DNA
<213>  Artificial Sequence

<220>
<223>  VSIG1 RV

<400>  68
gcaaggcagc ttaggatcag                                                    20


<210>  69
<211>  22
<212>  DNA
<213>  Artificial Sequence

<220>
<223>  VSIG1_V1 FW

<400>  69
gctggacctc tgagatttac tt                                                 22


<210>  70
<211>  20
<212>  DNA
<213>  Artificial Sequence

<220>
<223>  VSIG1_V1 RV

<400>  70
tacctggatc gttggaccct                                                    20


<210>  71
<211>  26
<212>  DNA
<213>  Artificial Sequence

<220>
<223>  VSIG1_V2 FW

<400>  71
aggagatgga gccaatttct atttac                                             26


<210>  72
<211>  20
<212>  DNA
<213>  Artificial Sequence

<220>
<223>  VSIG1_V2 RV

<400>  72
tacctggatc gttggaccct                                                    20


<210>  73
<211>  20
<212>  DNA

<213>  Artificial Sequence

<220>
<223>  HES6 FW

<400>  73
aacttttagg gaccctgcag                                                    20


<210>  74
<211>  22
<212>  DNA
<213>  Artificial Sequence

<220>
<223>  HES6 RV

<400>  74
cgctggcagc tctacaaaat tc                                                 22


<210>  75
<211>  19
<212>  DNA
<213>  Artificial Sequence

<220>
<223>  SPINK4 FW

<400>  75
cagtgggtaa tcgccctgg                                                     19


<210>  76
<211>  23
<212>  DNA
<213>  Artificial Sequence

<220>
<223>  SPINK4 RV

<400>  76
cacagatggg cattcttgag aaa                                                23


<210>  77
<211>  20
<212>  DNA
<213>  Artificial Sequence

<220>
<223>  TFF3 FW

<400>  77
ctccagctct gctgaggagt                                                    20


<210>  78
<211>  20
<212>  DNA
<213>  Artificial Sequence

<220>

<223>  TFF3 RV

<400>  78
ctccagctct gctgaggagt                                                   20


<210>  79
<211>  21
<212>  DNA
<213>  Artificial Sequence

<220>
<223>  HDAC9 FW

<400>  79
agtagagagg catcgcagag a                                                 21


<210>  80
<211>  21
<212>  DNA
<213>  Artificial Sequence

<220>
<223>  HDAC9 RV

<400>  80
ggagtgtctt tcgttgctga t                                                 21


<210>  81
<211>  21
<212>  DNA
<213>  Artificial Sequence

<220>
<223>  KIF19 FW

<400>  81
accctcatcg cccataaagt g                                                 21


<210>  82
<211>  21
<212>  DNA
<213>  Artificial Sequence

<220>
<223>  KIF19 RV

<400>  82
tcgaacaggt aggacttctc c                                                 21


<210>  83
<211>  20
<212>  DNA
<213>  Artificial Sequence

<220>
<223>  MAP2 FW

<400>  83

ccaatggatt cccatacagg                                                    20


<210>  84
<211>  20
<212>  DNA
<213>  Artificial Sequence

<220>
<223>  MAP2 RV

<400>  84
ctgctacagc ctcagcagtg                                                    20


<210>  85
<211>  20
<212>  DNA
<213>  Artificial Sequence

<220>
<223>  ENOX FW

<400>  85
cgatagacac gacccagctc                                                    20


<210>  86
<211>  20
<212>  DNA
<213>  Artificial Sequence

<220>
<223>  ENOX RV

<400>  86
cgcatcctct gcttgcattc                                                    20


<210>  87
<211>  21
<212>  DNA
<213>  Artificial Sequence

<220>
<223>  PDGFA FW

<400>  87
gcaagaccag gacggtcatt t                                                  21


<210>  88
<211>  19
<212>  DNA
<213>  Artificial Sequence

<220>
<223>  PDGFA RV

<400>  88
ggcacttgac actgctcgt                                                     19

<210>  89
<211>  19
<212>  DNA
<213>  Artificial Sequence

<220>
<223>  NKD1 FW

<400>  89
actttcggct ggaagtggc                                                    19


<210>  90
<211>  20
<212>  DNA
<213>  Artificial Sequence

<220>
<223>  NKD1 RV

<400>  90
cagggttcgc tcactctctc                                                   20


<210>  91
<211>  20
<212>  DNA
<213>  Artificial Sequence

<220>
<223>  TUBA1A FW

<400>  91
gcaacaacct ctcctcttcg                                                   20


<210>  92
<211>  20
<212>  DNA
<213>  Artificial Sequence

<220>
<223>  TUBA1A RV

<400>  92
gaatcatctc ctcccccaat                                                   20


<210>  93
<211>  22
<212>  DNA
<213>  Artificial Sequence

<220>
<223>  REG4 FW

<400>  93
ccttgcacta gctacatcct ca                                                22


<210>  94
<211>  20
<212>  DNA

<213>  Artificial Sequence

<220>
<223>  REG4 RV

<400>  94
aaaaccatcc aggagcacag                                                   20


<210>  95
<211>  20
<212>  DNA
<213>  Artificial Sequence

<220>
<223>  RCOR2 FW

<400>  95
cactcgcacg acagcatgat                                                   20


<210>  96
<211>  22
<212>  DNA
<213>  Artificial Sequence

<220>
<223>  RCOR2 RV

<400>  96
catcgcaatg tacttgtcaa gc                                                22


<210>  97
<211>  23
<212>  DNA
<213>  Artificial Sequence

<220>
<223>  ZEB1 FW

<400>  97
ttacaccttt gcatacagaa ccc                                               23


<210>  98
<211>  22
<212>  DNA
<213>  Artificial Sequence

<220>
<223>  ZEB1 RV

<400>  98
tttacgatta cacccagact gc                                                22


<210>  99
<211>  22
<212>  DNA
<213>  Artificial Sequence

<220>

<223>  SYT2L FW

<400>  99
ggaggtcttc gtattggctt tg                                                22


<210>  100
<211>  21
<212>  DNA
<213>  Artificial Sequence

<220>
<223>  SYT2L RV

<400>  100
ccatcttctc ccagagagca a                                                 21


<210>  101
<211>  22
<212>  DNA
<213>  Artificial Sequence

<220>
<223>  MSX1 FW

<400>  101
ggaaccatat cttcacctgc gt                                                22


<210>  102
<211>  20
<212>  DNA
<213>  Artificial Sequence

<220>
<223>  MSX1 RV

<400>  102
agttctccag ctcgctcagc                                                   20


<210>  103
<211>  20
<212>  DNA
<213>  Artificial Sequence

<220>
<223>  BMP4 FW

<400>  103
gatccacagc actggtcttg                                                   20


<210>  104
<211>  20
<212>  DNA
<213>  Artificial Sequence

<220>
<223>  BMP4 RV

<400>  104

gggatgctgc tgaggttaaa                                                    20

<210>  105
<211>  21
<212>  DNA
<213>  Artificial Sequence

<220>
<223>  GLRX FW

<400>  105
cccatcaaac aagggcttct g                                                  21

<210>  106
<211>  22
<212>  DNA
<213>  Artificial Sequence

<220>
<223>  GLRX RV

<400>  106
ctgcatccgc ctatacaatc tt                                                 22

<210>  107
<211>  20
<212>  DNA
<213>  Artificial Sequence

<220>
<223>  DKK1 FW

<400>  107
tccgaggaga aattgaggaa                                                    20

<210>  108
<211>  20
<212>  DNA
<213>  Artificial Sequence

<220>
<223>  DKK1 RV

<400>  108
cctgaggcac agtctgatga                                                    20

<210>  109
<211>  22
<212>  DNA
<213>  Artificial Sequence

<220>
<223>  EPCAM FW

<400>  109
aatcgtcaat gccagtgtac tt                                                 22

<210>  110
<211>  23
<212>  DNA
<213>  Artificial Sequence

<220>
<223>  EPCAM RV

<400>  110
tctcatcgca gtcaggatca taa                                               23


<210>  111
<211>  19
<212>  DNA
<213>  Artificial Sequence

<220>
<223>  S100A14 FW

<400>  111
gagacgctga ccccttctg                                                    19


<210>  112
<211>  20
<212>  DNA
<213>  Artificial Sequence

<220>
<223>  S100A14 RV

<400>  112
cttggccgct tctccaatca                                                   20


<210>  113
<211>  19
<212>  DNA
<213>  Artificial Sequence

<220>
<223>  RAB25 FW

<400>  113
atcggcgaat caggtgtgg                                                    19


<210>  114
<211>  21
<212>  DNA
<213>  Artificial Sequence

<220>
<223>  RAB25 RV

<400>  114
taggtctggt gcttggttag g                                                 21


<210>  115
<211>  1269
<212>  DNA

<213>  Artificial Sequence

<220>
<223>  VSIG1 ORF_1

<400>  115
atggtgttcg cattttggaa ggtctttctg atcctaagct gccttgcagg tcaggttagt      60

gtggtgcaag tgaccatccc agacggtttc gtgaacgtga ctgttggatc taatgtcact     120

ctcatctgca tctacaccac cactgtggcc tcccgagaac agctttccat ccagtggtct     180

ttcttccata agaaggagat ggagccaatt tctcacagct cgtgcctcag tactgagggt     240

atggaggaaa aggcagtcag tcagtgtcta aaaatgacgc acgcaagaga cgctcgggga     300

agatgtagct ggacctctga gatttacttt tctcaaggtg gacaagctgt agccatcggg     360

caatttaaag atcgaattac agggtccaac gatccaggta atgcatctat cactatctcg     420

catatgcagc cagcagacag tggaatttac atctgcgatg ttaacaaccc cccagacttt     480

ctcggccaaa accaaggcat cctcaacgtc agtgtgttag tgaaaccttc taagcccctt     540

tgtagcgttc aaggaagacc agaaactggc cacactattt ccctttcctg tctctctgcg     600

cttggaacac cttcccctgt gtactactgg cataaacttg agggaagaga catcgtgcca     660

gtgaaagaaa acttcaaccc aaccaccggg attttggtca ttggaaatct gacaaatttt     720

gaacaaggtt attaccagtg tactgccatc aacagacttg gcaatagttc ctgcgaaatc     780

gatctcactt cttcacatcc agaagttgga atcattgttg gggccttgat tggtagcctg     840

gtaggtgccg ccatcatcat ctctgttgtg tgcttcgcaa ggaataaggc aaaagcaaag     900

gcaaaagaaa gaaattctaa gaccatcgcg gaacttgagc caatgacaaa gataaaccca     960

aggggagaaa gcgaagcaat gccaagagaa gacgctaccc aactagaagt aactctacca    1020

tcttccattc atgagactgg ccctgatacc atccaagaac cagactatga gccaaagcct    1080

actcaggagc ctgccccaga gcctgcccca ggatcagagc ctatggcagt gcctgacctt    1140

gacatcgagc tggagctgga gccagaaacg cagtcggaat tggagccaga gccagagcca    1200

gagccagagt cagagcctgg ggttgtagtt gagcccttaa gtgaagatga aaagggagtg     1260

gttaaggca                                                            1269

<210>  116
<211>  1161
<212>  DNA
<213>  Artificial Sequence

<220>
<223>  VSIG1 ORF_2

<400>  116
atggtgttcg cattttggaa ggtctttctg atcctaagct gccttgcagg tcaggttagt      60

gtggtgcaag tgaccatccc agacggtttc gtgaacgtga ctgttggatc taatgtcact     120

ctcatctgca tctacaccac cactgtggcc tcccgagaac agctttccat ccagtggtct 180

ttcttccata agaaggagat ggagccaatt tctatttact tttctcaagg tggacaagct 240

gtagccatcg ggcaatttaa agatcgaatt acagggtcca acgatccagg taatgcatct 300

atcactatct cgcatatgca gccagcagac agtggaattt acatctgcga tgttaacaac 360

cccccagact ttctcggcca aaaccaaggc atcctcaacg tcagtgtgtt agtgaaacct 420

tctaagcccc tttgtagcgt tcaaggaaga ccagaaactg gccacactat ttccctttcc 480

tgtctctctg cgcttggaac accttcccct gtgtactact ggcataaact tgagggaaga 540

gacatcgtgc cagtgaaaga aaacttcaac ccaaccaccg ggattttggt cattggaaat 600

ctgacaaatt ttgaacaagg ttattaccag tgtactgcca tcaacagact tggcaatagt 660

tcctgcgaaa tcgatctcac ttcttcacat ccagaagttg gaatcattgt tggggccttg 720

attggtagcc tggtaggtgc cgccatcatc atctctgttg tgtgcttcgc aaggaataag 780

gcaaaagcaa aggcaaaaga aagaaattct aagaccatcg cggaacttga gccaatgaca 840

aagataaacc caaggggaga aagcgaagca atgccaagag aagacgctac ccaactagaa 900

gtaactctac catcttccat tcatgagact ggccctgata ccatccaaga accagactat 960

gagccaaagc ctactcagga gcctgcccca gagcctgccc caggatcaga gcctatggca 1020

gtgcctgacc ttgacatcga gctggagctg gagccagaaa cgcagtcgga attggagcca 1080

gagccagagc cagagccaga gtcagagcct ggggttgtag ttgagccctt aagtgaagat 1140

gaaaagggag tggttaaggc a 1161

<210> 117
<211> 59
<212> DNA
<213> Artificial Sequence

<220>
<223> shVSIG1(26)

<400> 117
ccggacgtga ctgttggatc taatgctcga gcattagatc caacagtcac gtttttttg 59

<210> 118
<211> 59
<212> DNA
<213> Artificial Sequence

<220>
<223> shVSIG(95)

<400> 118
ccggccaacg atccaggtaa tgcatctcga gatgcattac ctggatcgtt ggtttttg 59

<210> 119

<211>  57
<212>  DNA
<213>  Artificial Sequence

<220>
<223>  shCAV1

<400>  119
ccgggacgtg gtcaagattg actttctcga gaaagtcaat cttgaccacg tcttttt          57


<210>  120
<211>  20
<212>  DNA
<213>  Artificial Sequence

<220>
<223>  CRISPR gRNA

<400>  120
ctcaactaac ctccacacaa                                                   20


<210>  121
<211>  20
<212>  DNA
<213>  Artificial Sequence

<220>
<223>  VSIG KO V-P F1

<400>  121
atcaaggaaa cacgccccat                                                   20


<210>  122
<211>  27
<212>  DNA
<213>  Artificial Sequence

<220>
<223>  VSIG KO V-P R1

<400>  122
tgagaaaatt ttcattcaga agctcca                                           27

## Description

### Field of the Invention

The present invention relates to novel methods for identifying a cluster of cells associated with a phenotype. The invention also relates to methods for preventing cancer metastasis. The invention also relates to methods for determining the efficacy of a medicament. The invention also relates to a heterogeneous population of cells spiked with a phenotypically characterised and tagged single cell.

### Background of the Invention

Defining and understanding the role of heterogeneity in complex cellular systems, such as a growing tumor, are central questions in biology and medicine. For instance, how some cells but not others chose metastatic fates within primary carcinomas is not known. Recent studies support neither a straight clonal division of pro- and anti-metastatic fates nor the existence of common metastatic driver mutations distinct from those that drive tumor progression, although genetic selection of clones and genetic drift have been documented. Instead, metastasis may depend on intra-tumoral heterogeneity. Previous studies have addressed this issue through transcriptional analyses of single cells or combined omics of organoids from colon cancers, and the sampling of multiple sites from individual tumors. In this context, metastatic states could depend on epigenetic events and/or cell-to-cell signaling. Support for this idea includes the balance of anti- and pro-metastatic states via the modulation of protein secretion and the existence of positive interactions that affect metastatic growth, multiclonal invasion and clinical behavior in various cancers. However, since colon cancer heterogeneity is reestablished in single-cell clonal grafts, and dominant positive interactions would tend to homogenize the outcome, these may be secondary to the initial establishment of pro-metastatic states. Many of these studies rely on population assays of primary tumors and therefore it remained unclear if there could be operationally defined heterogeneity of metastatic potential (hereafter pro-metastatic heterogeneity) in primary tumors at the single cell level, and what kinds of cellular interactions may shape the choice of a pro-metastatic state. Single-cell RNA sequencing (scRNAseq) analyses have described the existence of different clusters within primary tumors although how these may relate to actual behaviors remains unknown. Functional tests with a heterogeneous population cannot ascribe a particular role to tumor sub-populations. Indeed, assigning functional properties to individual scRNAseq clusters is a major challenge in the field.

How cells with metastatic potential, or pro-metastatic states, arise within heterogeneous primary tumors remain unclear.

### Summary of the invention

A problem in the study of cell behaviour, both in the context of healthy tissue physiology and in the context of diseased tissue, is the inability to accurately define groups *(i.e.* "clusters") of cells within a heterogeneous cell population according to their function.

The inventors describe use of one index primary colon cancer to develop *spiked-scRNAseq* to link omics-defined single-cell clusters with clone behavior. Using spiked-scRNAseq we uncover cell populations with differential metastatic potential where pro-metastatic states are correlated with the expression of signaling and vesicle trafficking genes. Analyzing such heterogeneity we define an anti-metastatic, non-cell-autonomous interaction originating from non/low-metastatic cells, and identify membrane VSIG1 as a critical mediator of this interaction. VSIG1 acts to restrict the development of pro-metastatic states autonomously and non-cell-autonomously, in part by inhibiting YAP/TAZ-TEAD signaling. As VSIG1 re-expression is able to reduce metastatic behavior from multiple colon cancer cell types, regulation of VSIG1 or its effectors open new interventional opportunities. Generally, the inventors propose that crosstalk between cancer cells, including the action of VSIG1, dynamically define the degree of pro-metastatic intra-tumoral heterogeneity.

The method described herein advantageously does not require a priori knowledge of molecular identifiers of sub-populations of cells. For instance, the inventors' proof of principle with Vemurafenib (see Examples) opens the possibility to use spiked-scRNAseq to better understand drug effects in individual cell subpopulations aiming to select effective and personalized treatments.

Thus, the inventors provide a solution to the difficulty of accurately defining clusters of cells by function within a heterologous population by providing a method for identifying clusters of cells associated with a particular phenotype.

Accordingly, the invention provides a method for identifying a cluster of cells associated with a phenotype, wherein the method comprises:
a. spiking a heterogeneous population of cells with a tagged phenotypically characterised single cell; and
b. analysing single cell omics data from said spiked population of cells, wherein presence of the tagged single cell within a cluster of cells in the population identifies said cluster as associated with said phenotype.

The invention further provides a method for preventing cancer metastasis in an individual, wherein the method comprises administering to the individual an agent for inducing VSIG1.

The invention additionally provides a method for determining the efficacy of a medicament, wherein method comprises the identification of one or more clusters of cells in accordance with the method of identifying a cluster of cells associated with a phenotype of the invention, and wherein the method further comprises spiking and clustering a heterogeneous population of cells which has been subject to treatment with a medicament and assessing whether the proportion of a cluster of cells associated with a particular phenotype changes after treatment with said medicament.

The invention also provides a heterogeneous population of cells spiked with a phenotypically characterised and tagged single cell.

### Brief Description of the Figures

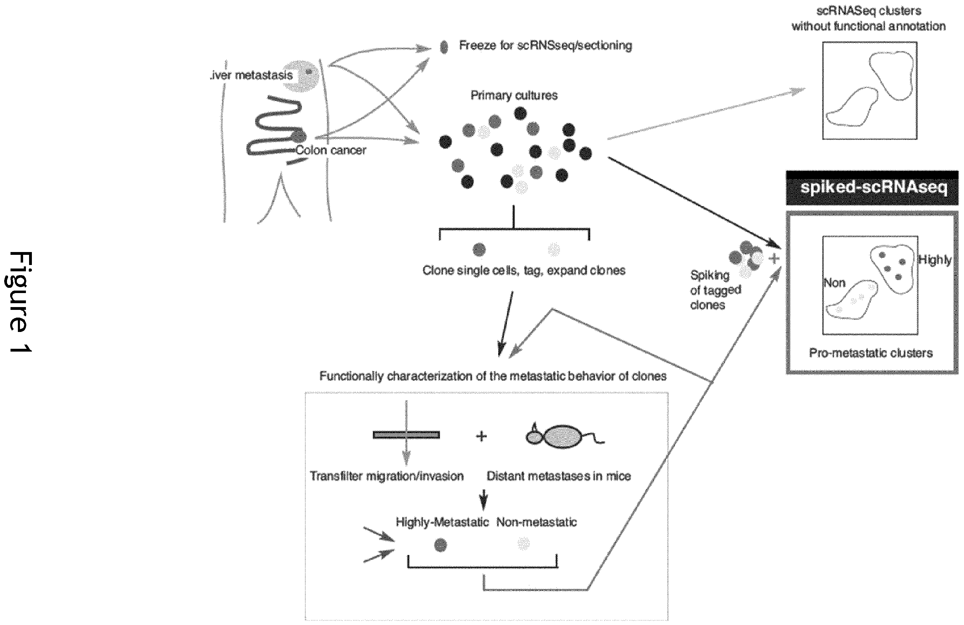

FIGURE 1 shows a schematic diagram of the workflow for spiked-scRNAseq. Without spikes, scRNAseq yields clusters without functional annotation. Spiked-scRNASeq, in contrast, links single-cell transcriptomic clusters to functional properties, in this case metastatic behavior. Black circles represent stromal/immune cells. Only two tumor cell populations with high (red) and no (yellow) metastatic potential are shown for simplicity.

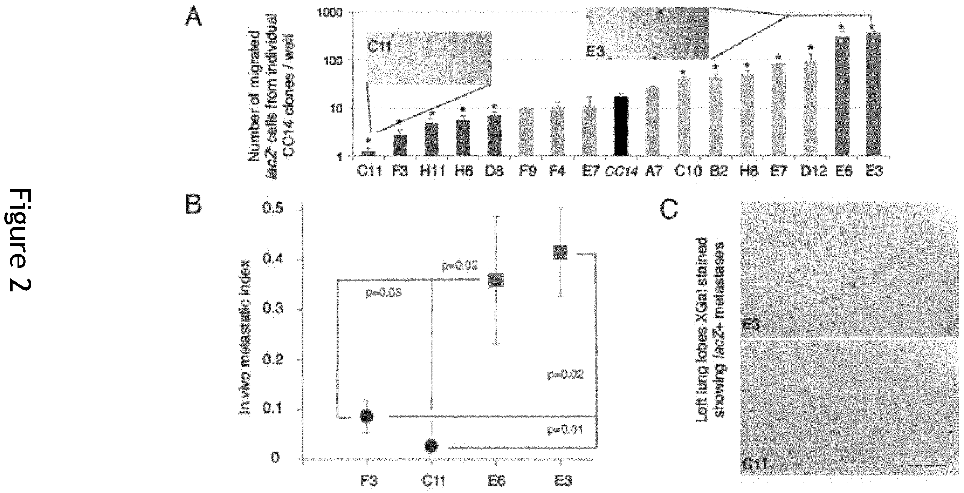

FIGURE 2 shows CC14 cloning and transwell efficiencies. A) Transfilter migration efficiencies of individual CC14 clones. Asterisks denote significant values vs. control parental CC14 cells (in black). High metastatic clones are noted in red/orange and low metastatic clones in blue. Insets show representative images of the XGAL-stained migratory cells in the filter. B) Quantification of distant metastases in vivo shown as the metastatic index (number of lacZ+ lung metastases over tumor volume). C) Representative images of XGAL stained lungs with E3 or C11 metastases. Scale bar = 0.2cm.

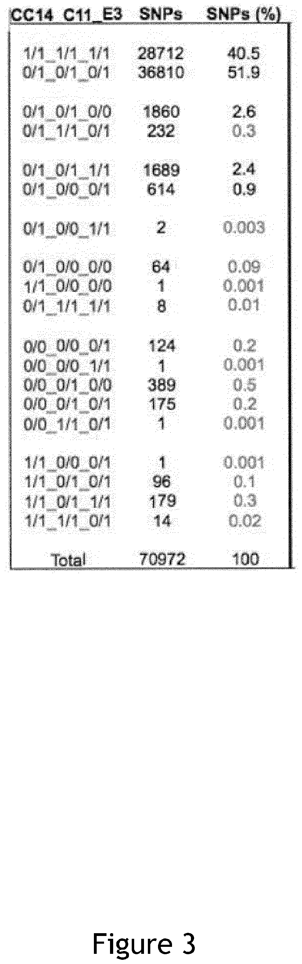

FIGURE 3 shows frequency of non-reference genome SNPs in CC14 primary culture and their presence in derived E3 and C11 clones. New SNPs in clones do not surpass 0.5% in any category. 0 denotes the reference genome sequence and 1 a divergent SNP.

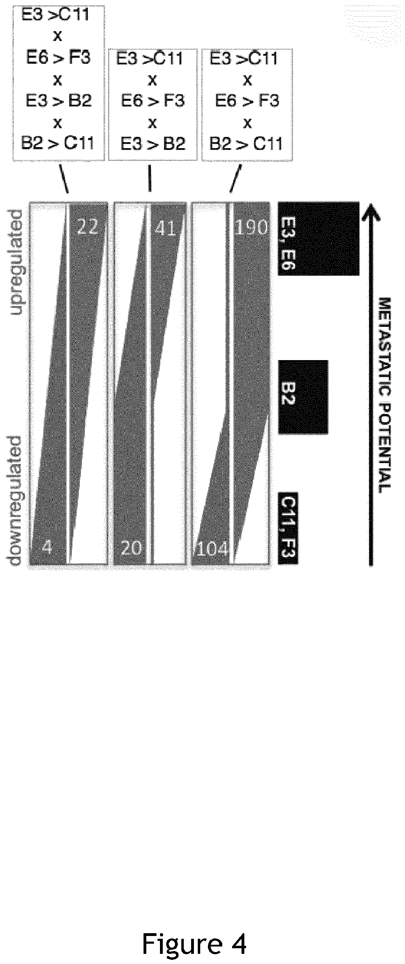

FIGURE 4 shows that transcriptional changes correlate metastatic efficiency. Comparison of 2 or more fold upregulated genes in each noted comparison reveals a correlation of metastatic efficiency with step-wise transcriptional changes. E3 and E6 are highly metastatic, B2 is medium-metastatic and C11 and F3 are low- or non-metastatic (see Fig. 2). Numbers in red and green backgrounds refer to the number of genes in each category.

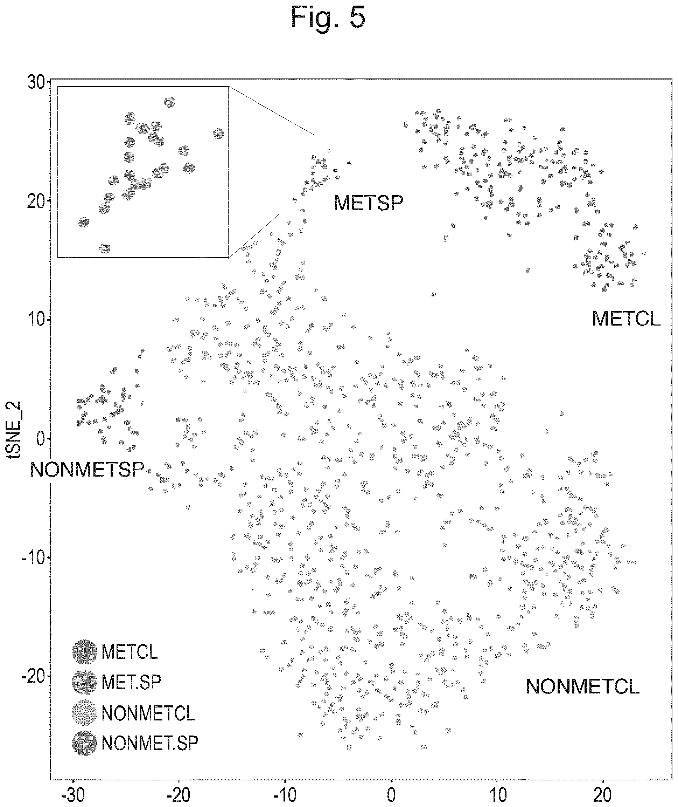

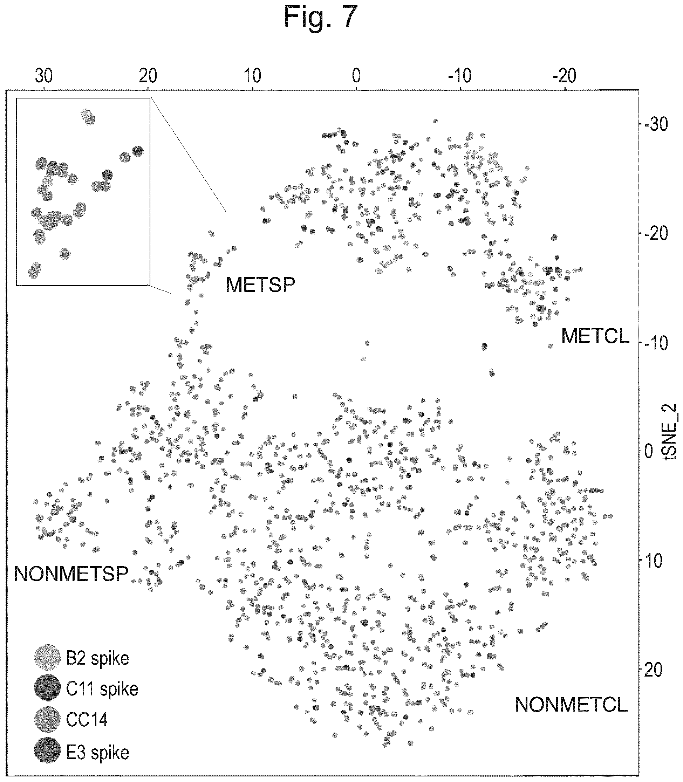

FIGURE 5 shows top) tSNE map of CC14 scRNAseq showing the four defined clusters (without PCA dimensions 1-3) using 1600 cells. Bottom) Heat map of top differentially expressed marker genes for each of the four defined clusters shown in the top panel. For nomenclature see below and Fig. 7.

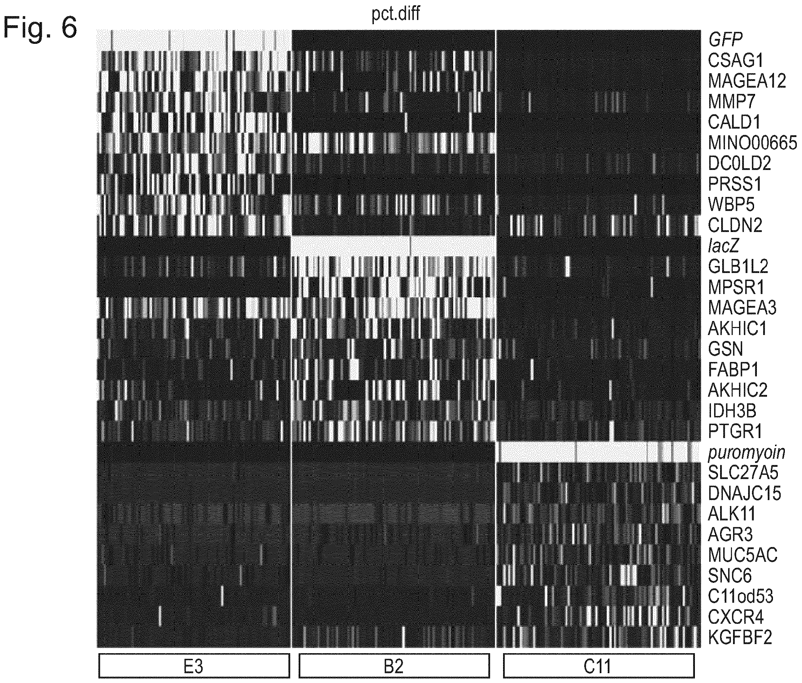

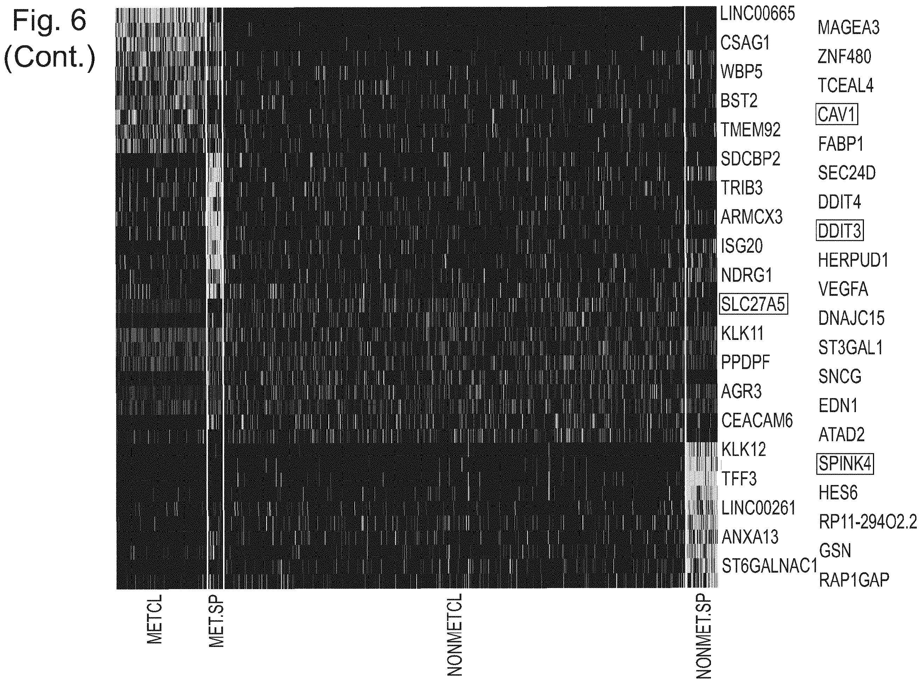

FIGURE 6 shows heat map of the nine best differentially expressed genes in spiked clones, scored as percentage of positive cells. Yellow shows highest expression and purple lowest. Note that genetic tags have been added for each clone type.
FIGURE 7 shows Spiked-scRNAseq; tSNE maps from spiked-scRNAseq of parental CC14 colon cancer heterogeneous primary cell population showing the homing of highly-metastatic E3, medium-metastatic B2 and non-metastatic C11 clone cell spikes, as noted by the color codes.

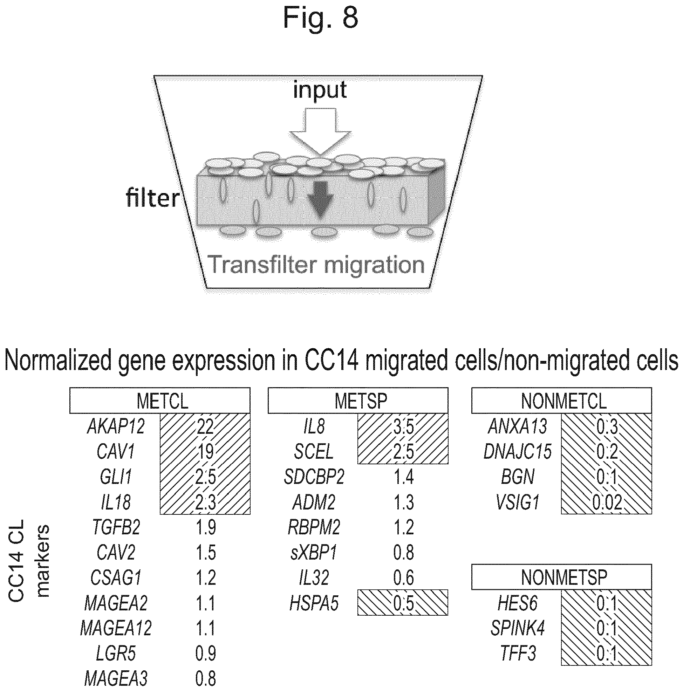

FIGURE 8 shows Left) Schematic diagram of the transfilter migration assay in which cells that actively cross the filter can be quantified. We routinely use XGAL to identify even single cells previously transduced with lacZ lentivectors. Cells that do and do not cross the filter can then be collected and rt-qPCR analyses performed to measure the expression of cluster-specific genes. Right) Heat map of such an assay is shown for markers of the four CC14 clusters. Several METCL and METSP markers (in red) are over-represented/filter, which also display a very strong repression/under-representation of non-metastatic values in migrating (bottom of filter) over non-migrating (top of filter) cell pools.

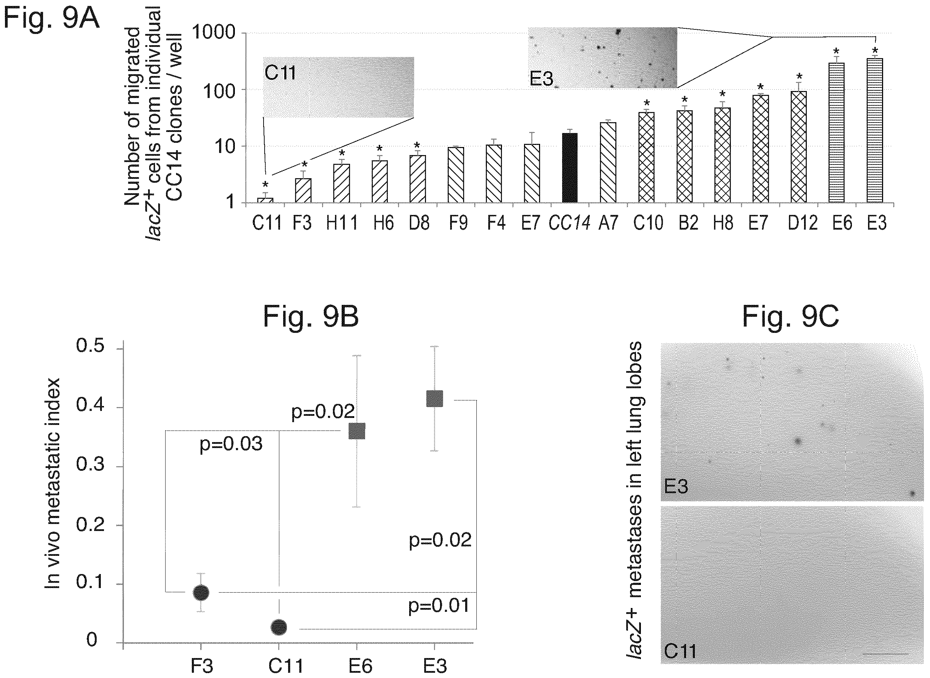

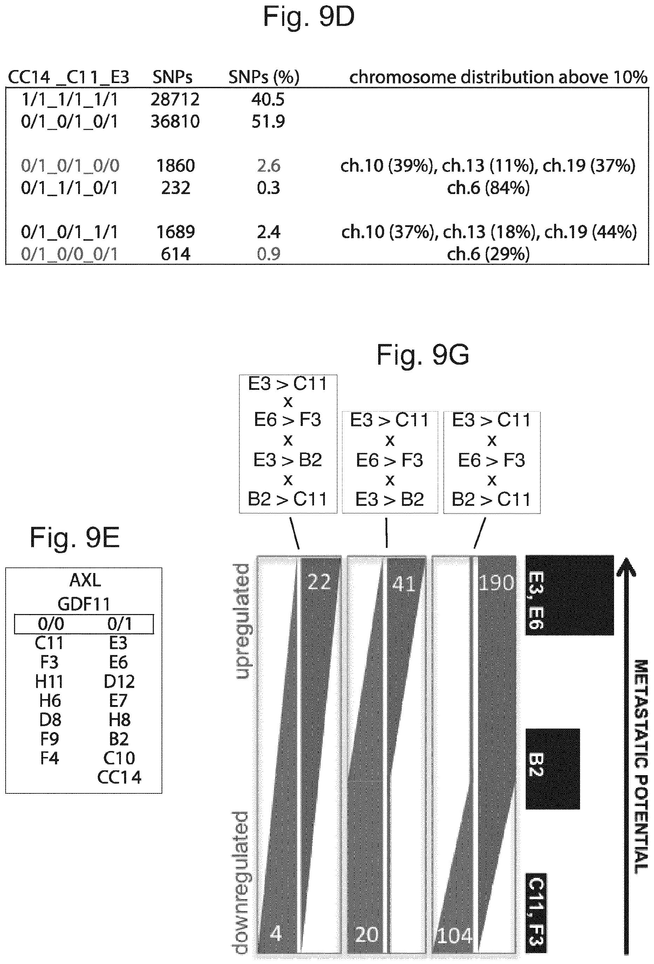

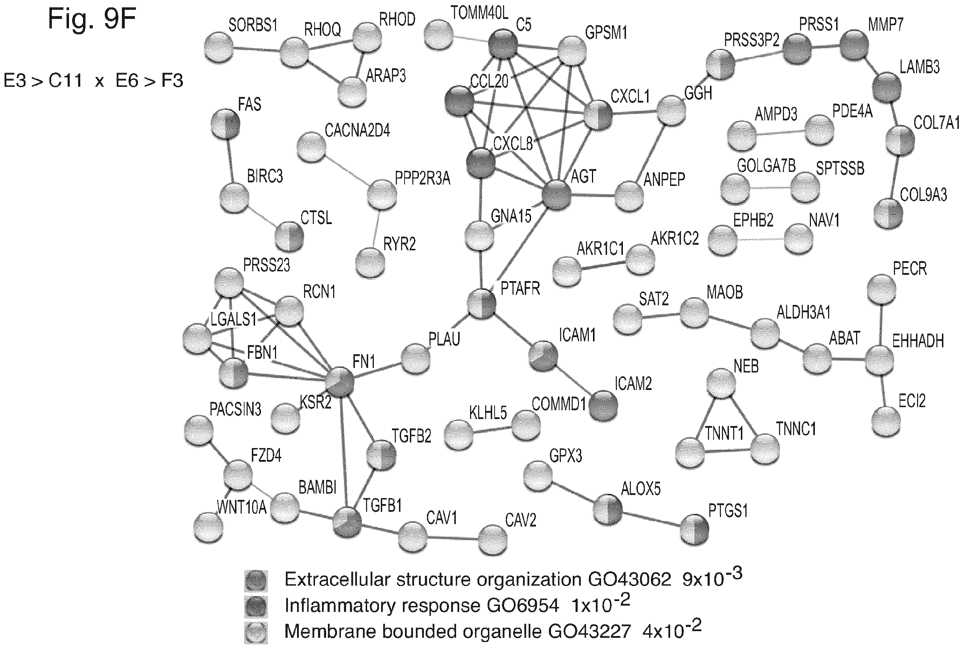

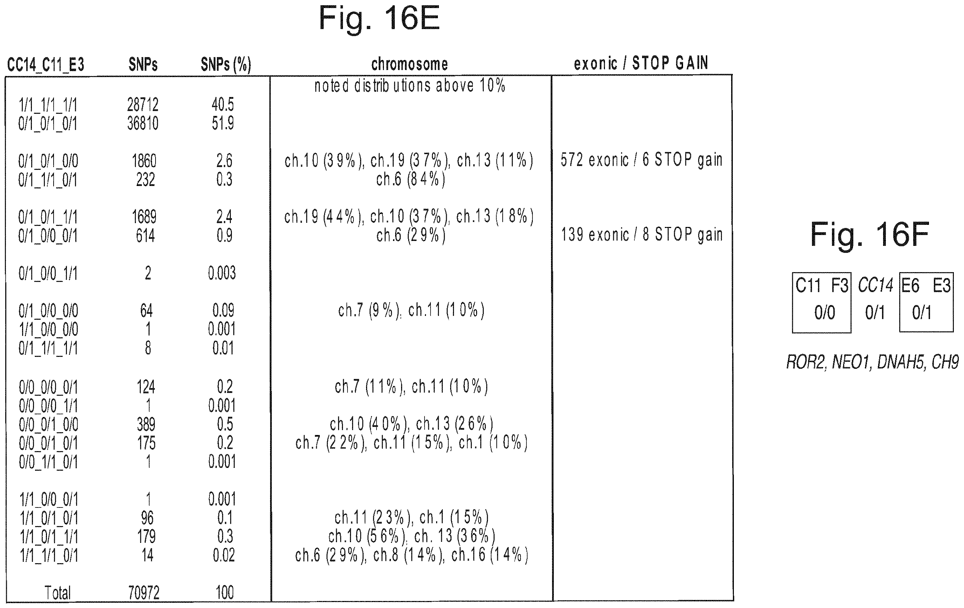

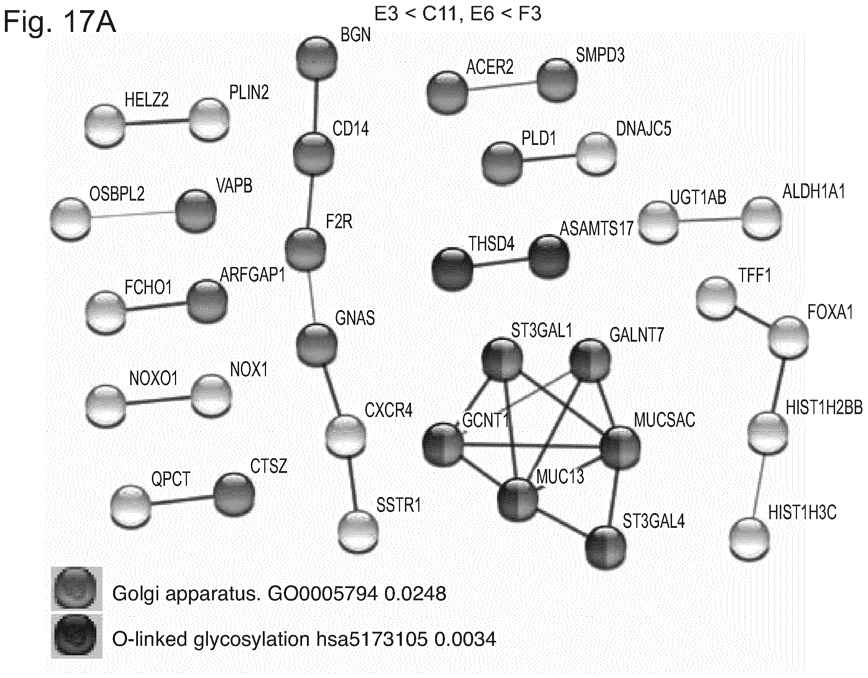

FIGURE 9 shows clonal metastatic heterogeneity, correlated SNPs and transcriptomic changes A) Quantitative analysis of transfilter assays of each of sixteen single-cell derived clones of the primary colon cancer CC14 population. (Asterisks: p<0.05 in relation to the CC14 sample in back). Insets show X-Gal-stained C11 and E3 filters. B) Quantification of in vivo distant lung metastases from high-metastatic E3lacZ and E6lacZ and low-metastatic F3lacZ and C11lacZ cell grafts. P values are for each pair tested simultaneously. The y-axis plots metastatic index (the ratio of number of metastases over tumor volume in mm3). n=5 mice per condition. C) Representative images of lungs after in toto X-Gal staining showing metastases from E3lacZ and C11lacZ. D) Summary table of the distribution of CC14-specific SNPs in E3 and C11. See Fig. 16E for alleles below 0.5%. E) Genotypes of AXL and GDF11 SNPs in clones by genomic PCR-sequencing. F) String and enrichment analyses from differentially expressed genes in high- vs. low-metastatic clone. See also Fig. 17A-C. In this and all String maps, colors highlight main processes. G) Diagram of the changes detected in the expression of genes from RNAseq analyses of five clones presented in two-by-two comparisons. The y-axis represents differential expression levels between high-metastatic (E3, E6, top) medium-metastatic (B2, middle) and low-metastatic (C11, F3, bottom) cells. The three categories along the x-axis represent different modes of regulation. Scale bar =150µm (A insets), 0.2cm (C). Error bars represent SEM. P values were determined using unpaired, two-tailed T-tests. Transfilter assays were performed in independent biological triplicates in which each one included technical duplicates.

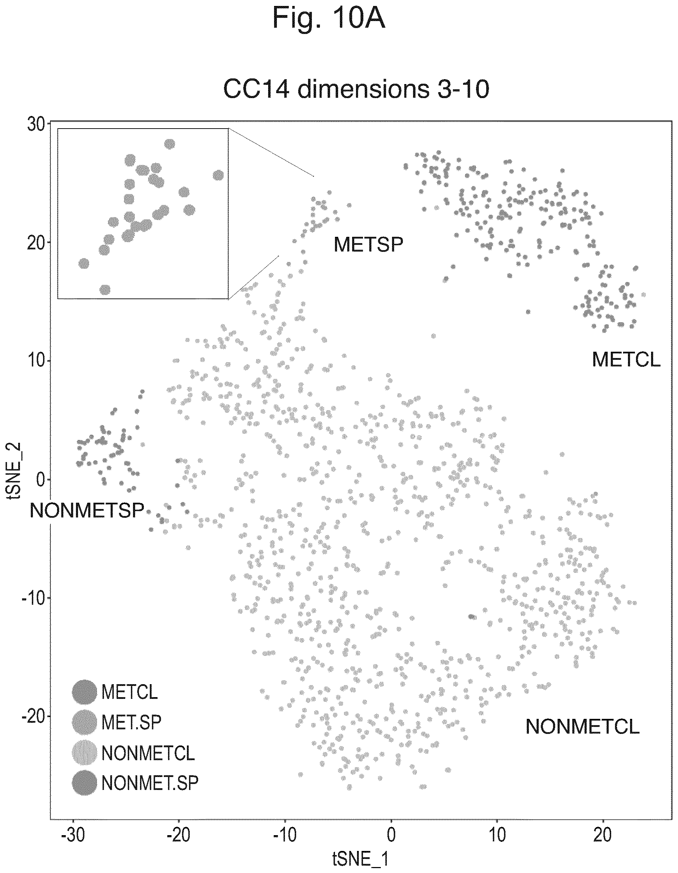

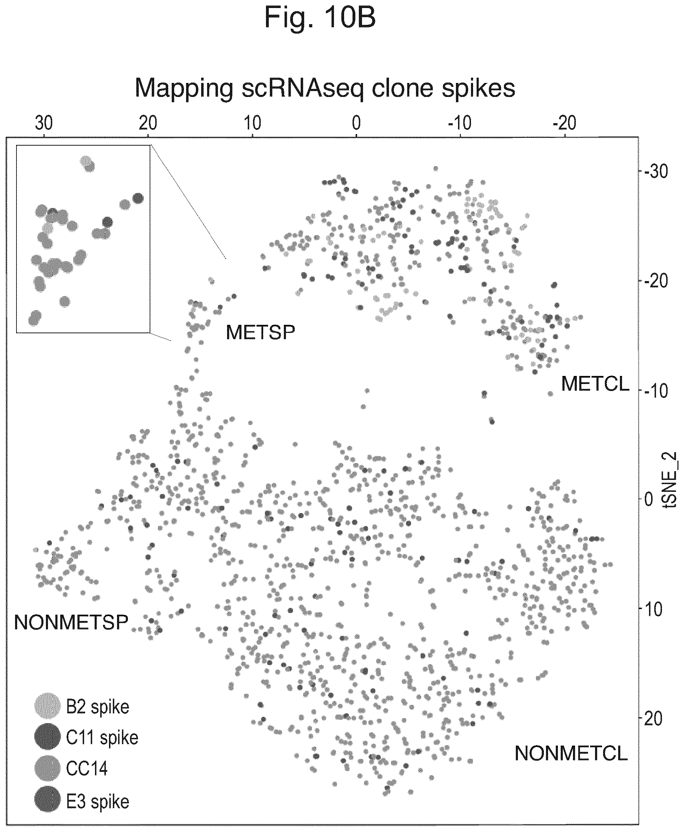

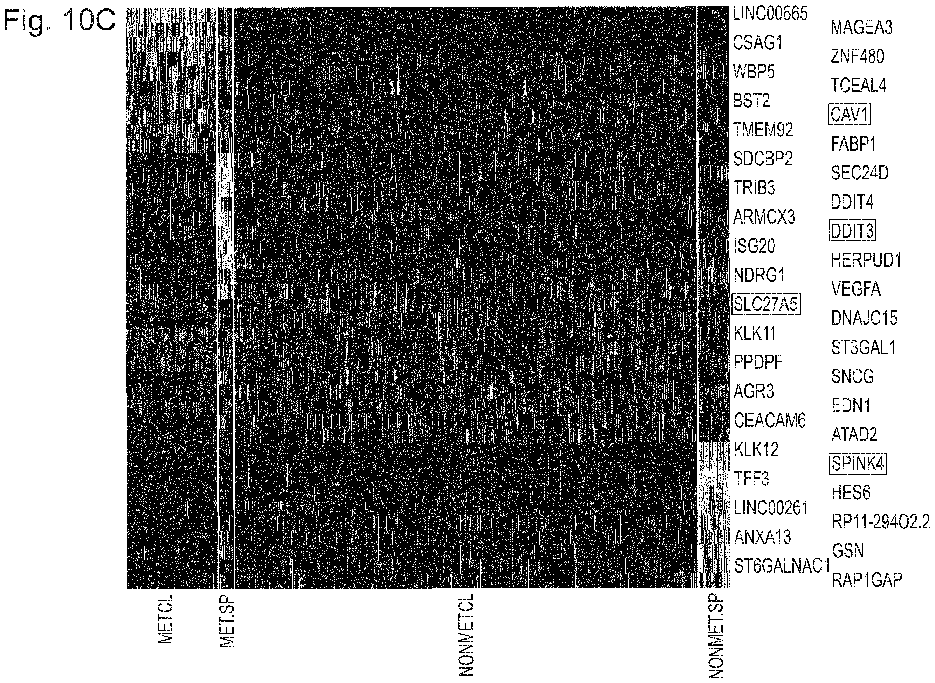

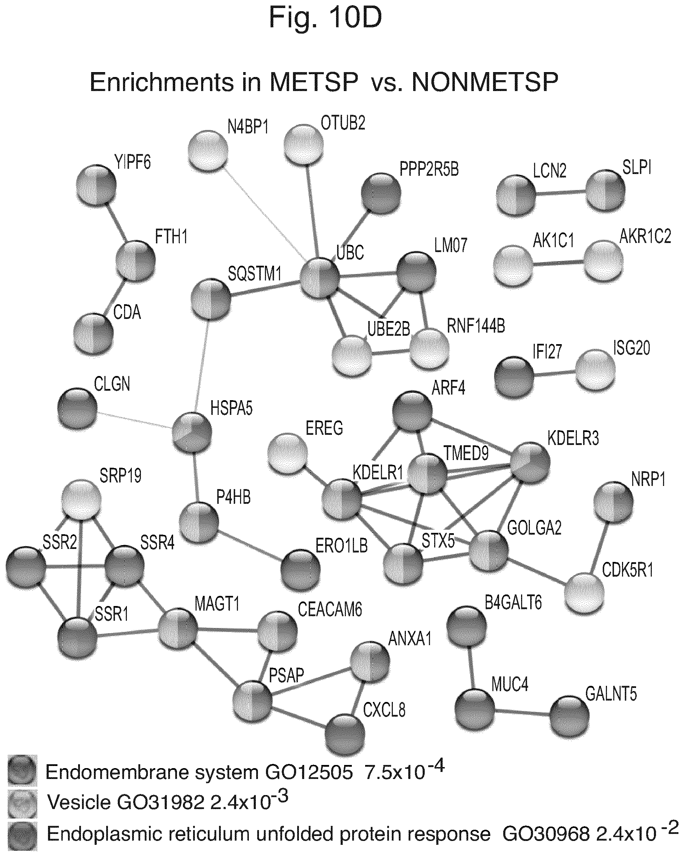

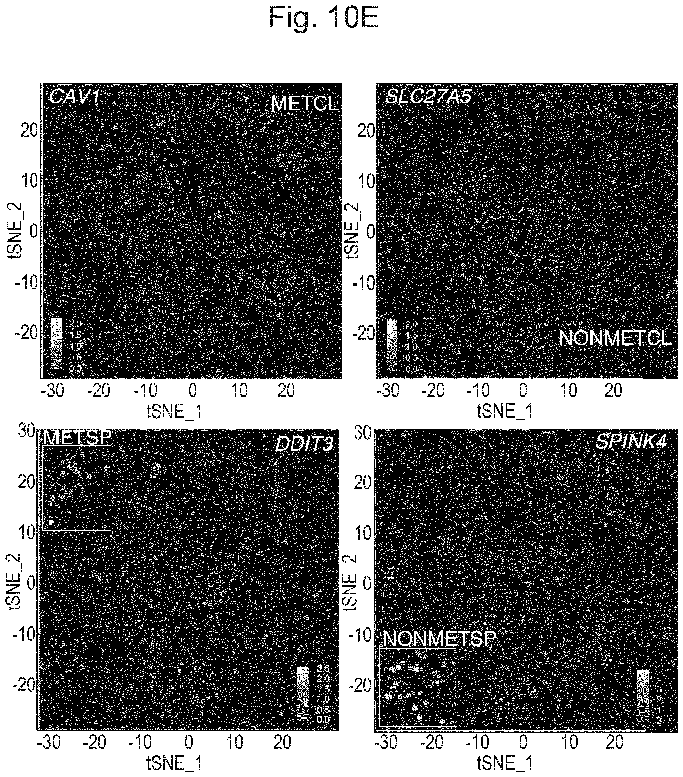

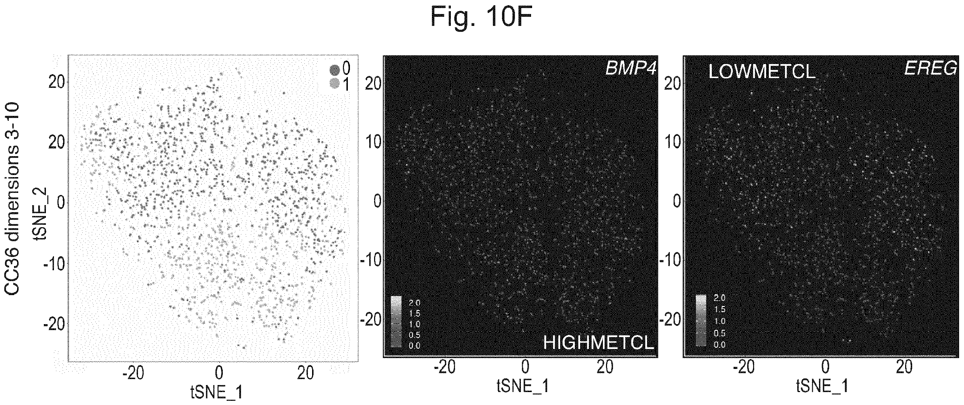

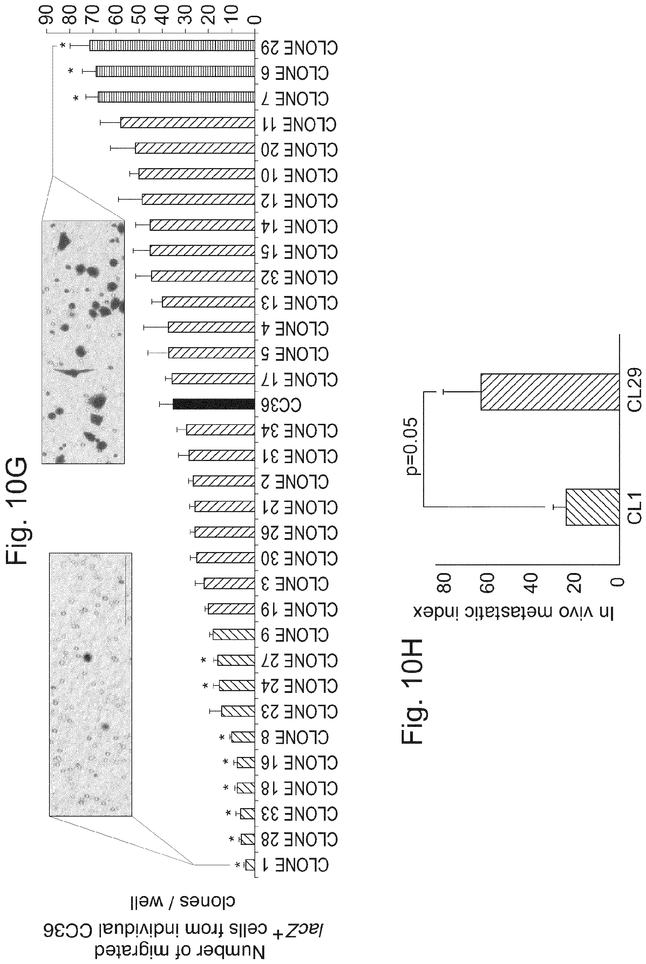

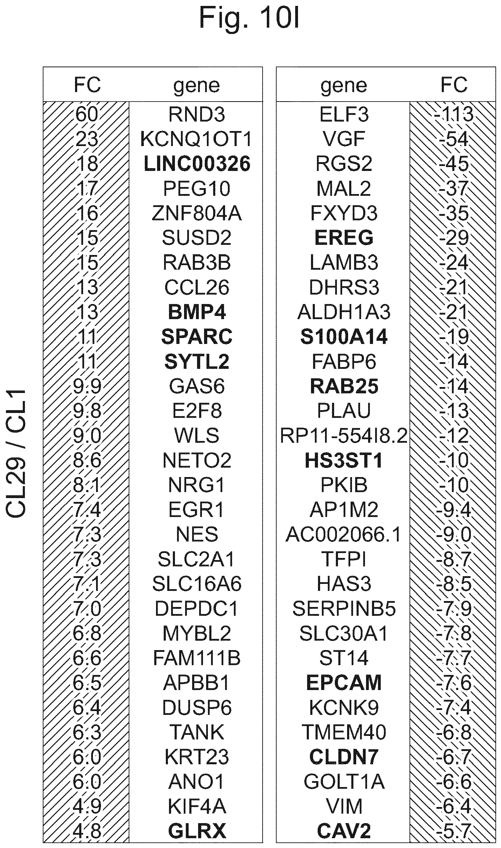

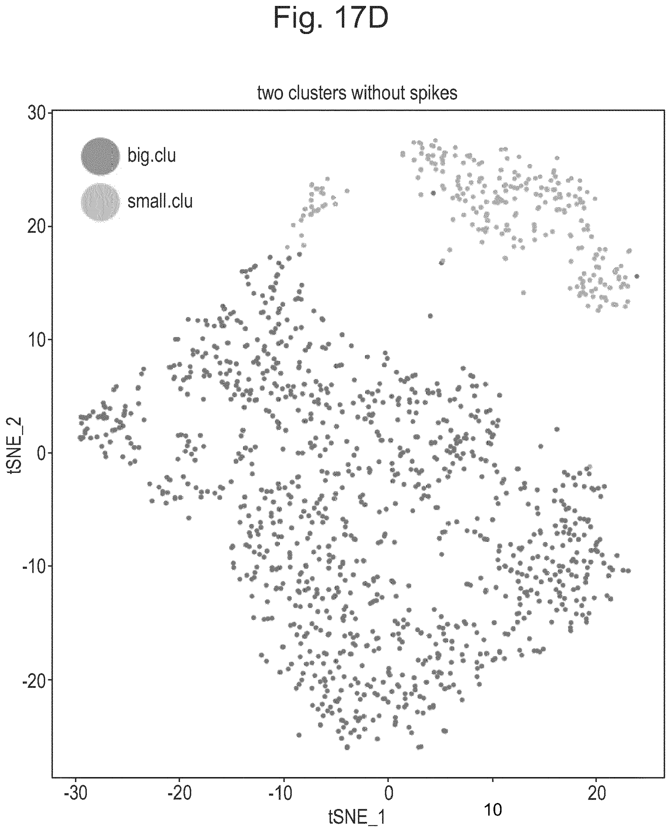

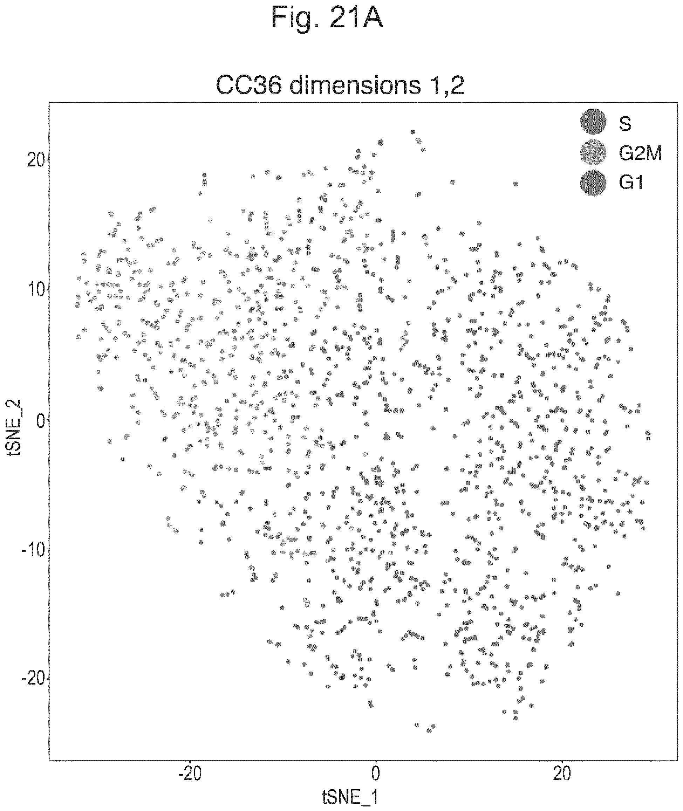

FIGURE 10 shows Spiked-scRNAseq identify pro-metastatic heterogeneity highlighting vesicular trafficking and intercellular signaling A) tSNE map of CC14 scRNAseq clusters. In this and all figures the x-axis of t-SNE maps represent t-SNE1 and the y-axis t-SNE2. See Fig. 17D-F. B) tSNE map of spiked-scRNAseq highlighting the distribution of spiked clone cells. The tSNE map varies slightly from (A) due to the inclusion of the spikes. C) Heat map of top differentially expressed genes in clusters (A) scored as percentage of expressing cells. Brightest yellow denotes highest expression and deep purple lowest. Frames mark genes mapped to highlight differences in (E). D) String and enrichment analyses for differentially expressed genes in the METSP vs. the NONMETSP. XBP-1 enriched genes (GSE46178 adj. p=7.7x10-64) include DDIT3 (encoding CHOP) and HSPA5 (encoding BIC). See also Fig. S3,S4. E) scRNAseq tSNE maps showing the differential localization of individuals cluster markers. See also Figs. S3. F) tSNE map of CC36 using PCA dimensions 3-10 that highlight two clusters and the localization of a marker of each one: BMP4 for the HIGHMETCL and EREG for the LOWMETCL. See also Figs. 21A-C. G) Quantitative analysis of transfilter migration of CC36 clones compared the parental heterogeneous population. (Asterisks = p<0.05 compared with CC36 in black). Insets show X-Gal 26 stained CL1 and CL29 filters. H) Quantification of the in vivo metastatic behavior, denoted by the metastatic index, of the CC36 clones CL1 and CL29. n=6 mice for CL1 and 5 for CL29. I) List of top differentially expressed genes in CL29 vs. CL1 from RNAseq data (>2-fold change; FDR<0.01). Genes highlighted in color are also differentially expressed between the two clusters of CC36 scRNAseq. Scale bar = 150µm (G insets) Error bars represent SEM. P values were determined using unpaired, two-tailed T-tests. Transfilter assays were performed in independent biological triplicates in which each one included technical duplicates.

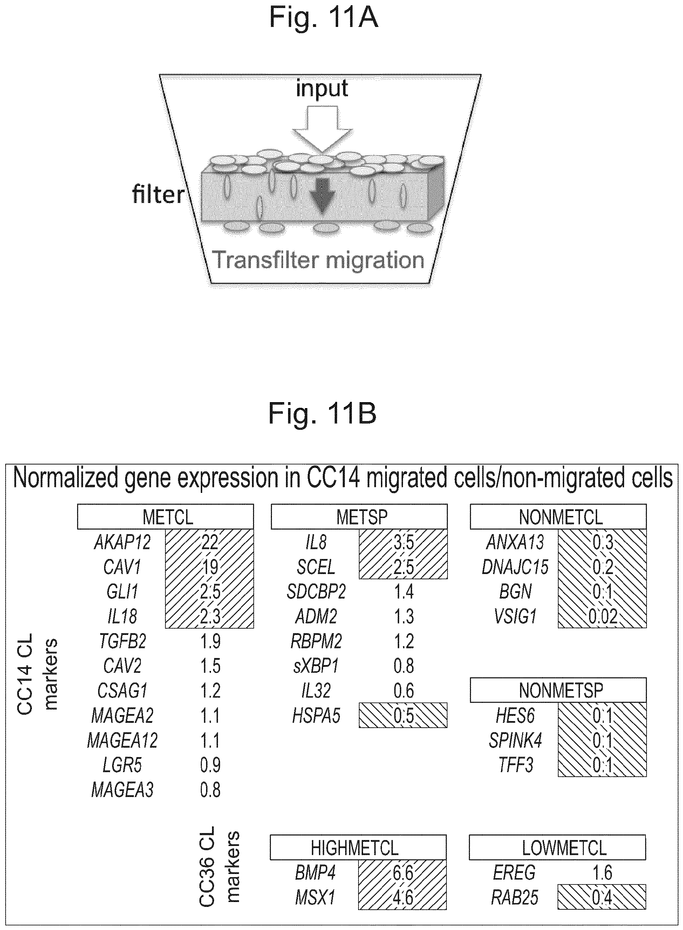

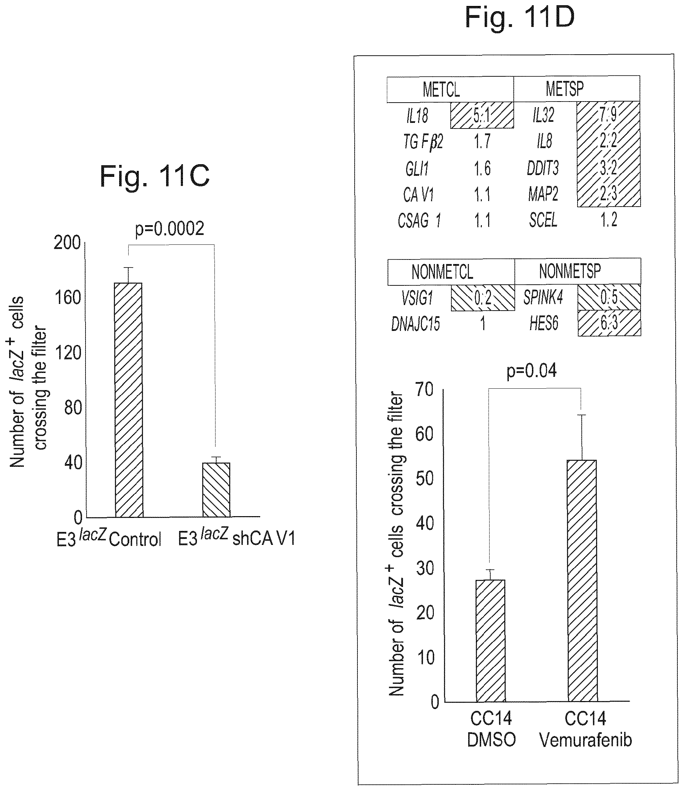

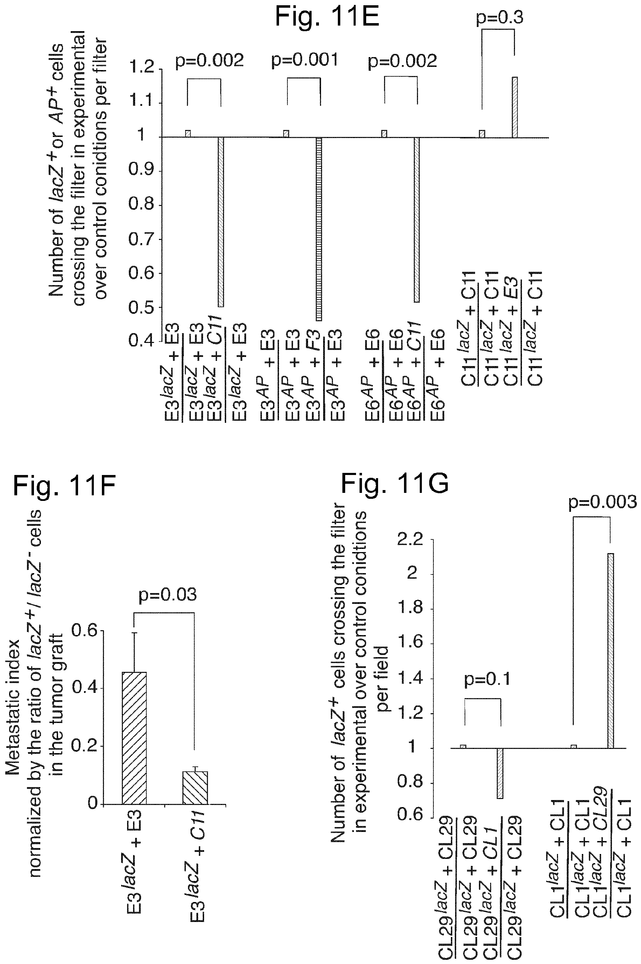

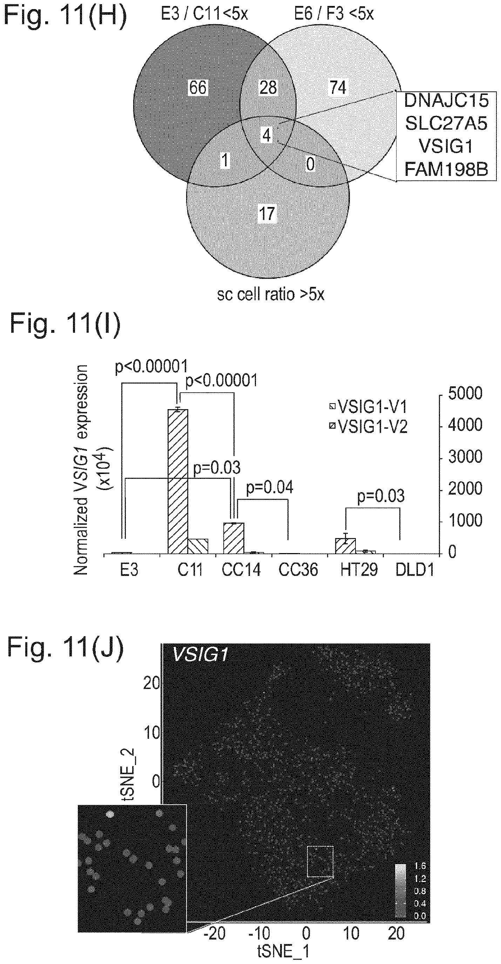

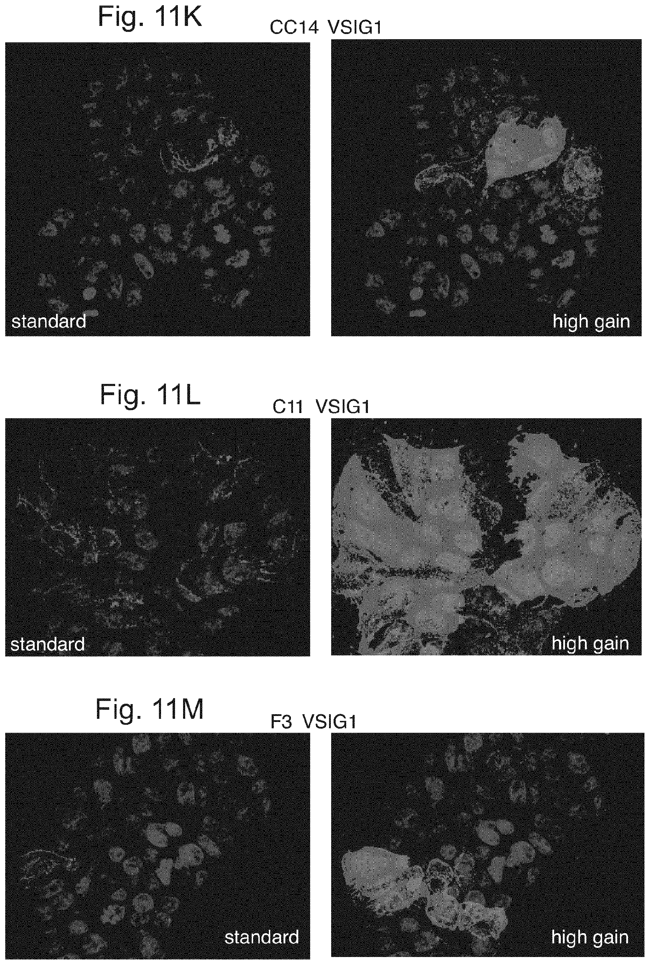

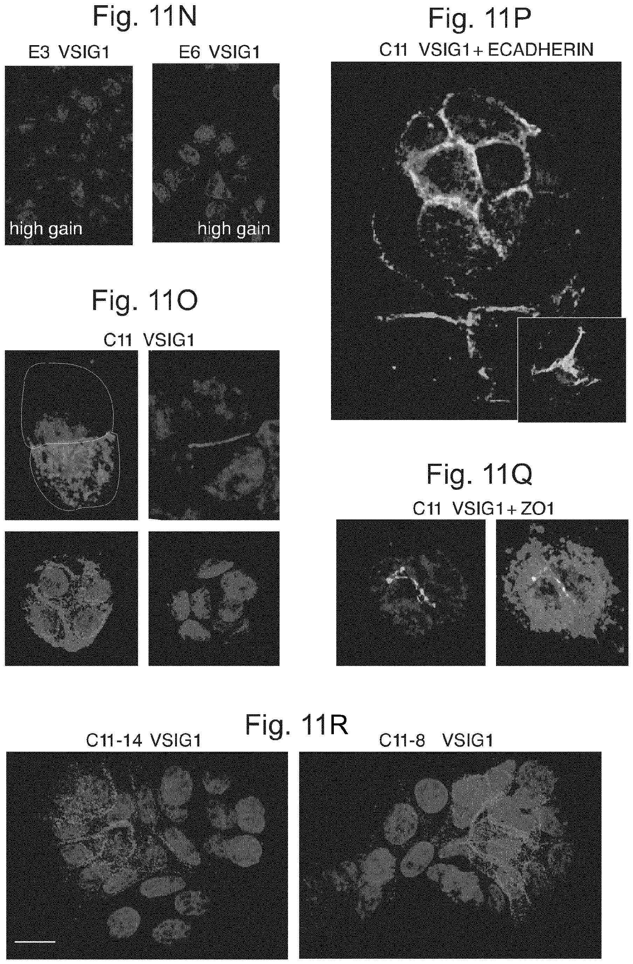

FIGURE 11 shows characterisation of metastatic cells, non-cell-autonomous interactions and identification of VSIG1. A) Transfilter assay scheme used to characterize metastatic behavior in vitro. B) Heat map of RT-qPCR results of the expression of different cluster markers of CC14 (top) or CC36 (bottom) in CC14 cells that migrated across the filter after normalization. 2-fold changes up (red (METCL, AKAP12: 22; CAV1: 19; GL1: 2.5; IL18: 2.3; METSP, IL8 (3.4); SCEL (2.5); HIGHMETCL, BMP4: 6.6; MSX1: 4.6)) or down (blue (METSP, HSPA5: 0.5; NONMETCL, ANXA13: 0.3; DNAJC15: 0.2; BGN, 0.1; VSIG1: 0.02; NONMETSP, HES6: 0.1, SPINK4: 0.1, TFF3: 0.1; LOWMETCL, RAB25: 0.4) are highlighted. C) Quantification of E3lacZ cells crossing the filter in transfilter assays harboring normal CAV1 (E3lacZControl) or compromised KD function through kd (E3lacZshCAV1). D) Gene expression and migratory changes driven by Vemurafenib treatment in CC14 cells. Top) Heat map of changes in gene expression over control as noted, colored as in (B), METCL, IL18: 5.1; METSP, IL32: 7.9, IL8:2.2, DDIT3: 3.2; MAP2: 2.3; NONMETCL, VSIG1: 0.2; NONMETSP, SPINK4: 0.5, HES6: 6.3). Bottom) Bar graph of the number of cells migrating in transfilter assays. E) Quantification of metastatic behavior in transfilter assays of E3lacZ, E6AP and C11lacZ co-cultured with different clones. Results were normalized by the number of labeled cells that have crossed the membrane in control conditions: traced clones co-cultured with the same untraced clone at 1:1, e.g. E3lacZ/E3. Cells modifying the behavior of traced clones are in italics. F) In vivo distant metastases by grafted E3lacZ cells are inhibited by co-grafted C11. n=4 mice per condition. The y-axis quantifies the metastatic index after normalization by the ratio of lacZ+ / lacZ- cells. G) Quantification of transfilter assays for CL1 and CL29 as in (E). H) Venn diagram of gene sets for each of three conditions used to identify VSIG1. I) Quantification of the expression of VSIG1 mRNA by RT-qPCR, using specific primers for each of two variants (VI and V2), in CC14, CC36, E3, C11, and in HT29 and DLD1 cell lines. J) Localization of VSIG1+ cells in the CC14 scRNAseq tSNE map. Insets show higher magnification of negative METCL (upper right) and positive NONMETCL (lower left) clusters. K-N) Confocal immunostaining with anti-VSIG1 mAb (red) in CC14 (K), C11 (L), F3 (M), E3 and E6 (N) non-permeabilized cells after Hoechst 33342 (blue) nuclear counterstaining. Images show maximal projections of z-stacks covering the entire depth of the cells by overlapping 0.6-0.8µm-thick z-slices. Identical confocal parameters have been used for VSIG1 in all pictures with standard gain, and the same for all with high gain. O) Varying distribution of VSIG1 protein in doublets and small C11 clusters. P,Q) Confocal double labeling of VSIG1 (red) and E-CADHERIN (green, P) or ZO1 (green, Q). All panels show single 0.6-0.8µm confocal slices. Right panel in Q shows a high gain image. R) Confocal maximal projection from collected z-stacks showing localization of VSIG1 protein in single-cell subclones of C11 Scale bar = 25µm (K-N), 7µm (O top panels, Q), 15µm (O bottom panels, P, R). Rt-qPCR data derived from two biological replicates, each analysis was carried out with three technical replicates. Error bars represent SEM. P values were determined using unpaired, two-tailed T-tests. Transfilter assays were performed in independent biological triplicates in which each one included technical duplicates.

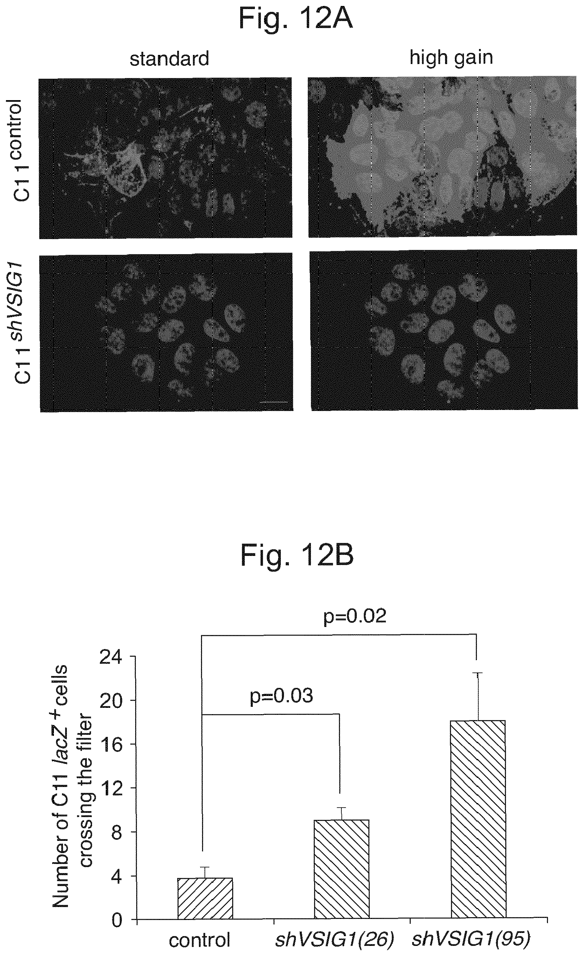

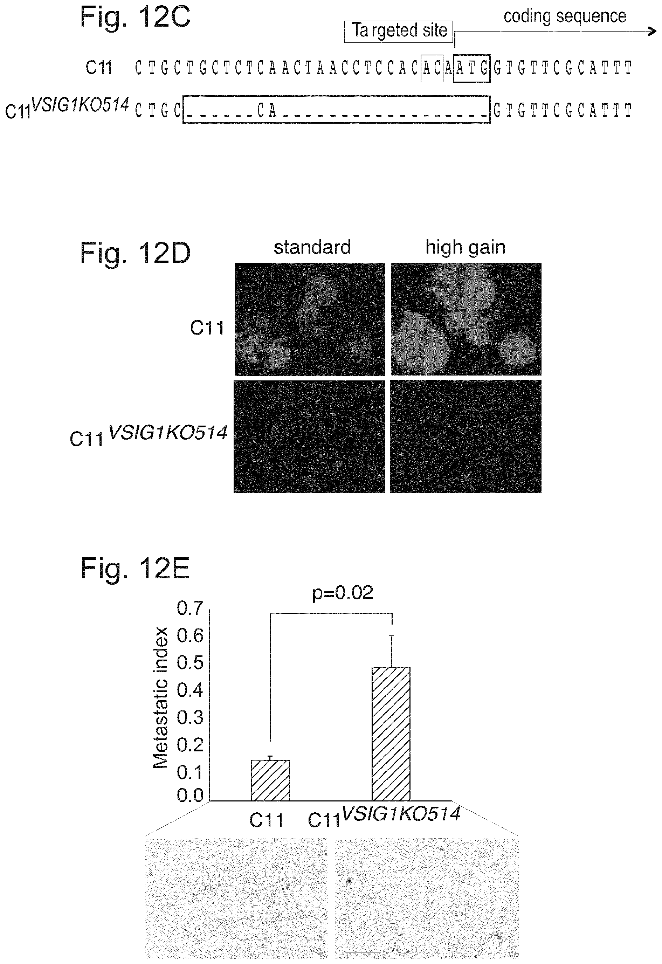

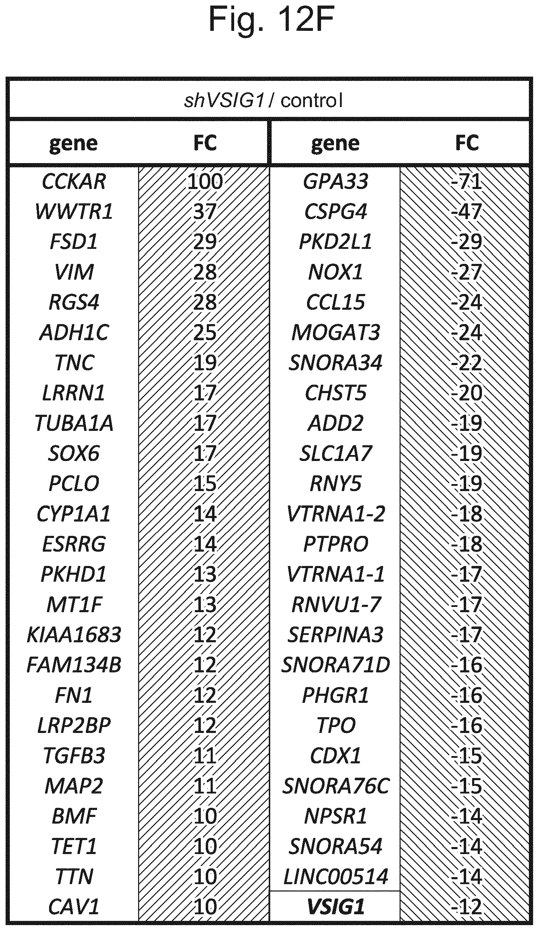

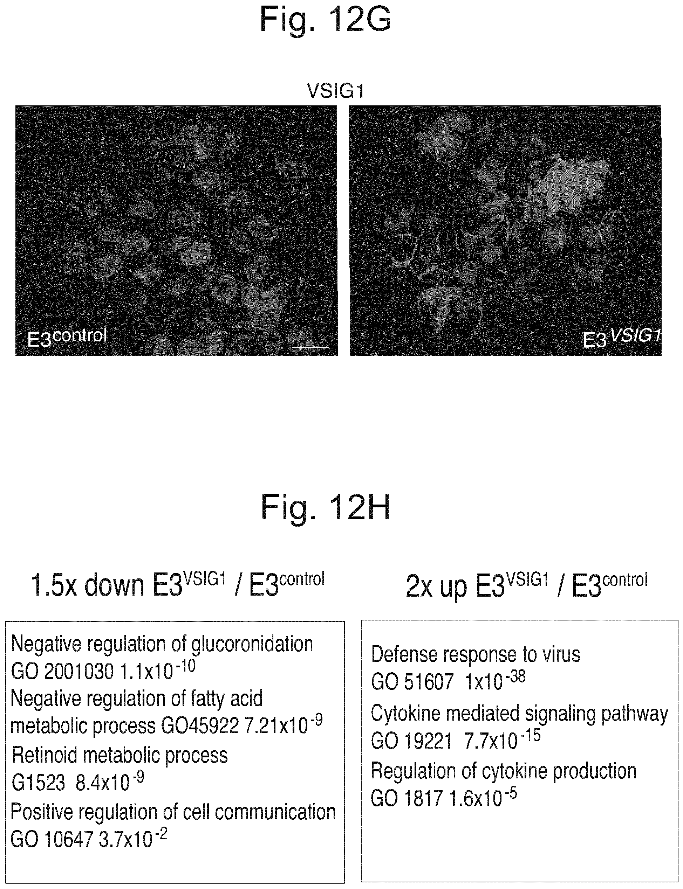

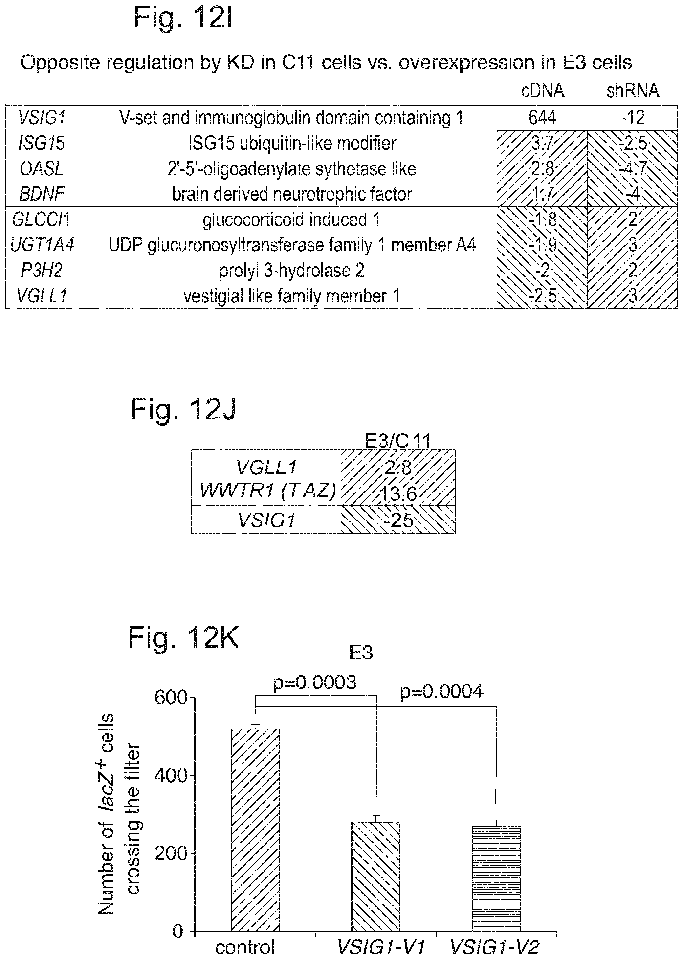

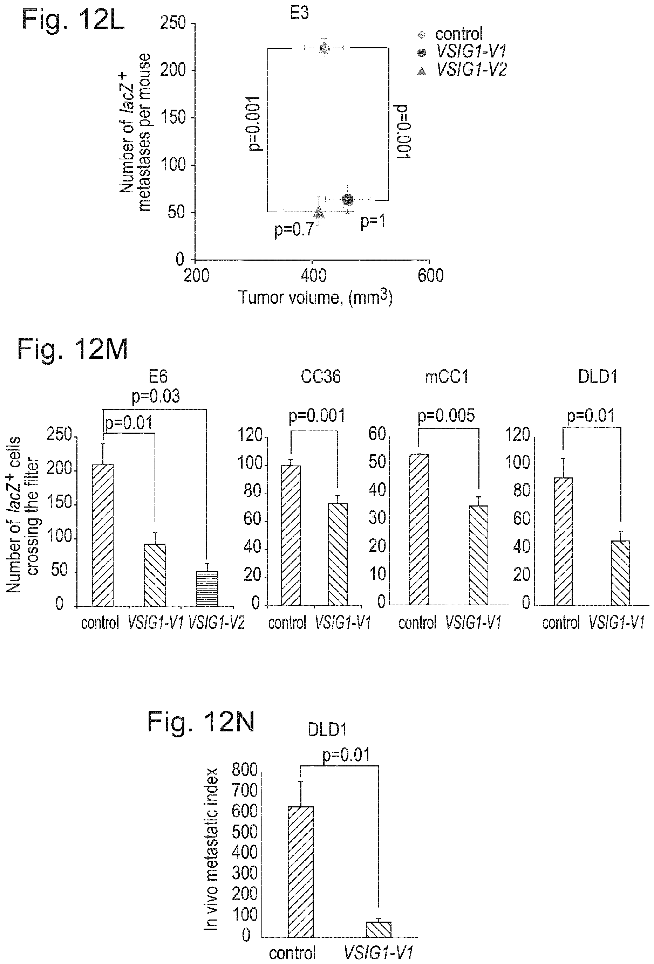

FIGURE 12 shows modulation of VSIG1 levels alters metastatic behavior and remodels the cell. A) Confocal maximal projection images at standard or high gain of C11 control cells and those with kd of VSIG1. B) Quantification analysis of transfilter assays with kd and control (PLKO.1 vector alone) C11 cells used as control. C) Diagram of the expressed mutant VSIG1 allele generated by CRISPR in C11 cells (C11VSIG1KO514). The initiator ATG (in yellow) is missing in the KO allele. D) Standard and high gain confocal images of VSIG1 antibody labeling of C11 (top row= and C11VSIG1KO514 (bottom row) showing the loss of VSIG1 protein expression in the mutant cells. E) Quantification of in vivo, lung distant metastases in mice bearing C11 or C11VSIG1KO514 xenografts. The y axis shows the metastatic index. n=8 mice for C11VSIG1KO514 and n=8 for control. F) Top upregulated (red) and downregulated (blue) genes, along with VSIG1, in RNAseq data from shVSIG1 C11 cells over control C11 cells. FDR<0.01 and 2-fold changes (up in red/cream, down in blue). G) Confocal maximal projection images of E3 control cells and those expressing exogenous VSIG1 (VI). H) Enrichment analyses of downregulated and upregulated genes as noted. I) Heat map table of genes showing inverse regulation by enhanced and repressed VSIG1 levels. VSIG1 is included as reference. J) Heat map showing enhance levels of VGLL1 and TAZ (WWTR1) in E3/C11 cells in opposition to VSIG1. K) Quantification of the number of E3lacZ+cells crossing the filter after exogenous expression of either of two isoforms of VSIG1 (VI, V2). L) Quantification of in vivo distant lung metastases from grafter E3lacZ+cells expressing each of the two VSIG1 isoforms (VI or V2) as compared with control E3lacZ+cells. n=5 mice for each condition. M) Quantification of the number of cells crossing the filter in transfilter assays after expression of VSIG1-V1 or VSIG1-V2 isoforms in E6 cells, or of VSIG-V1 in CC36 cells, mCC11 and DLD1 cells. N) Quantification of in vivo distant lung metastases generated by DLD1lacZVSIG-V1 cells. p values <0.05 are in red. Scale bars = 12µm for (A, G). Rt-qPCR data derived from two biological replicates, each analysis was carried out with three technical replicates. Error bars represent SEM. P values were determined using unpaired, two-tailed T-tests. Transfilter assays were performed in independent biological triplicates in which each one included technical duplicates.

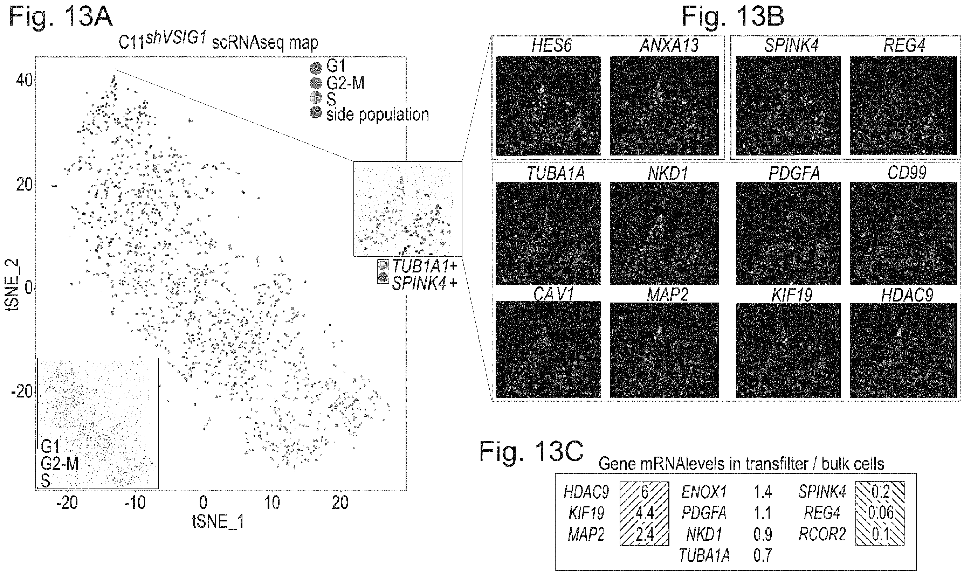

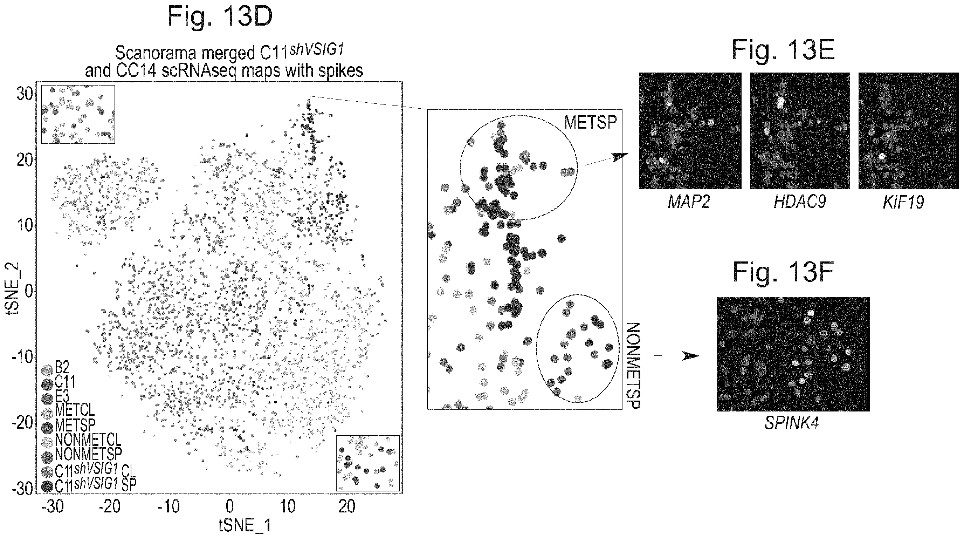

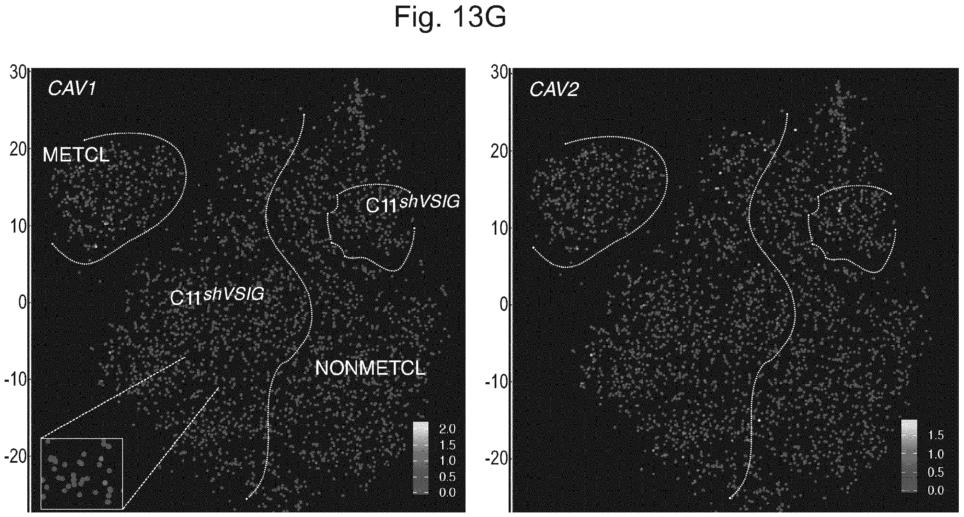

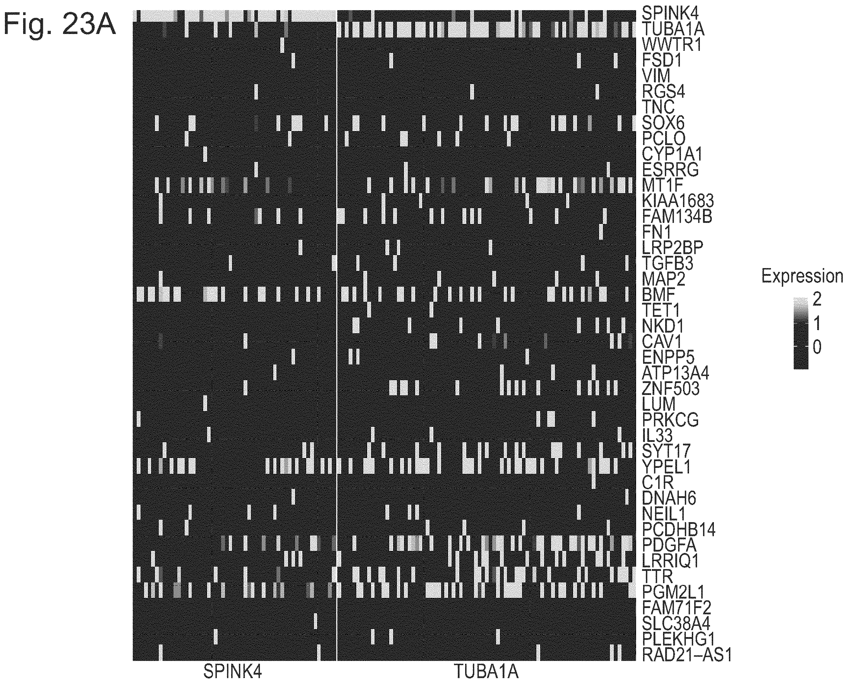

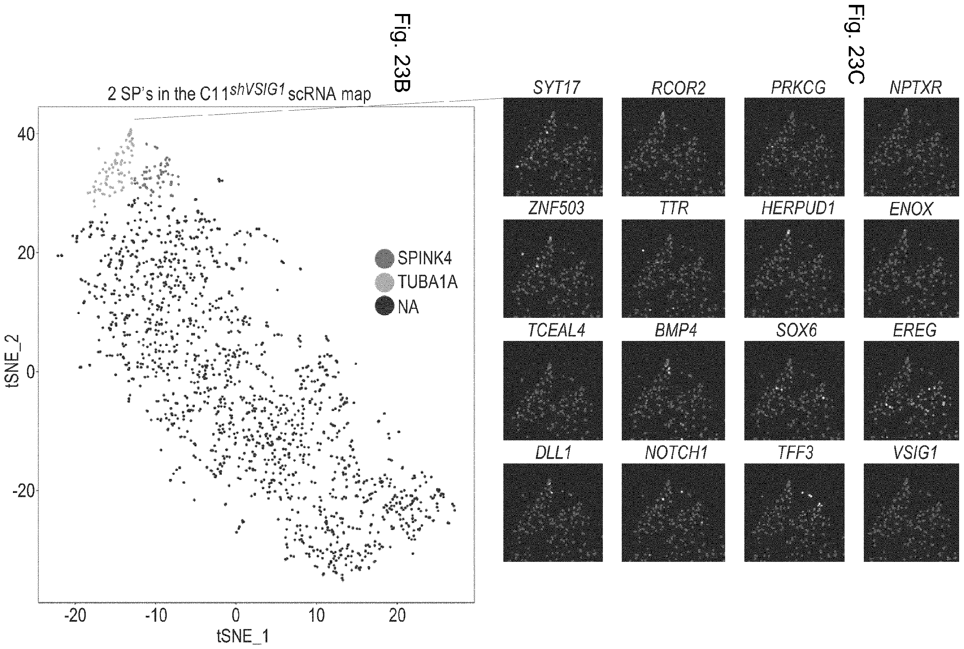

FIGURE 13 shows scRNAseq of C11shVSIG1 cells uncovers the appearance of a METSP-like population. A) scRNAseq tSNE map of C11shVSIG1 data showing clustering by cell cycle phases and the side population within G1 (lower left inset). The two horns in the SP (in purple in the large map) separate in two populations (right inset) through differential TUBA1A and SPINK4 expression B) Differential localization of NONMETSP and METCL/SP markers in the two horns of the C11shVSIG1SP. The markers shown were selected to be mostly (>98%) expressed in the C11shVSIG1SP. The colors of the frames refer to the colors of the horn populations in A and the right inset in A. See also Fig. 23. C) RT-qPCR analyses of genes in CC14 cells that have crossed the filter vs. the bulk as in Fig. 11A,B. D) SCANORAMA tSNE map of the merged C11shVSIG1 and CC14 with spikes data. The C11shVSIG1 bulk (light pink) cleanly separates from the CC14 NONMETCL (light green), where the C11 spike homes (purple). The C11shVSIG1SP (dark red) appears split into three: a minor part mingles with the C11shVSIG1 bulk, another one localized with NONMETSP cells (dark green) and a major one is instead close to METSP (dark blue) cells (inset). E, F) Mapping of the C11shVSIG1SP TUBA1A+ markers MAP2, HDAC9 and KIF19 that are enhanced in (C) reveal their co-localization with the METSP (E). In contrast, the C11shVSIG1SPSPINK4+marker SPINK4 labels the NONMETSP (F). G) Upregulated genes in C11shVSIG1 cells also included those with 'de novo' widespread expression in C11shVSIG1 that resemble the METCL/SP, like CAV1 and CAV2. RT-qPCR data derived from two biological replicates, each analysis was carried out with three technical replicates. Error bars represent SEM. P values were determined using unpaired, two-tailed T-tests.

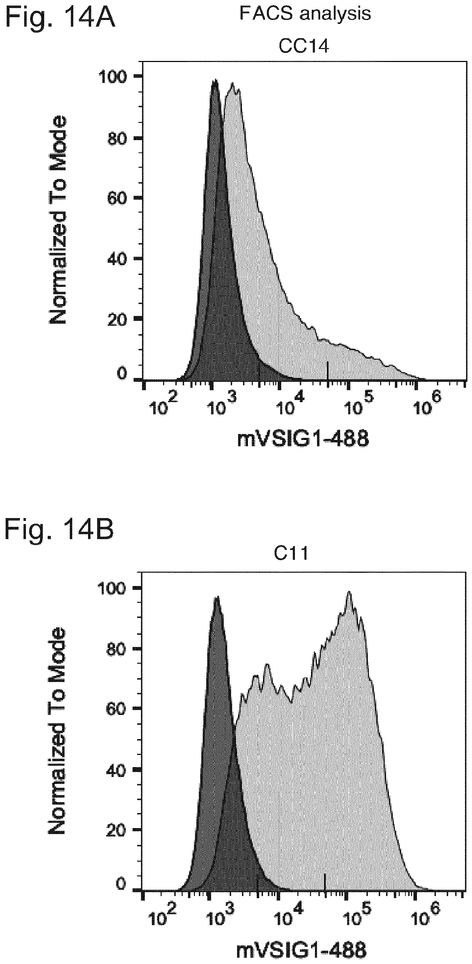

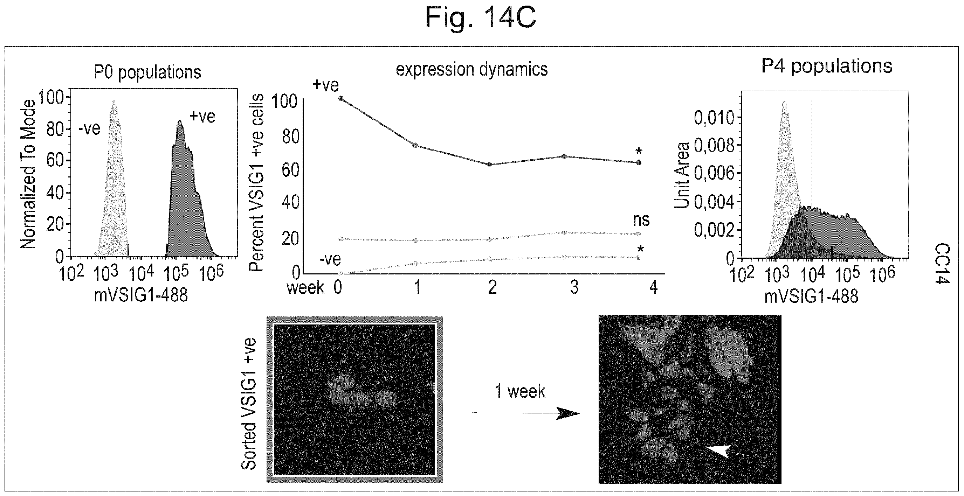

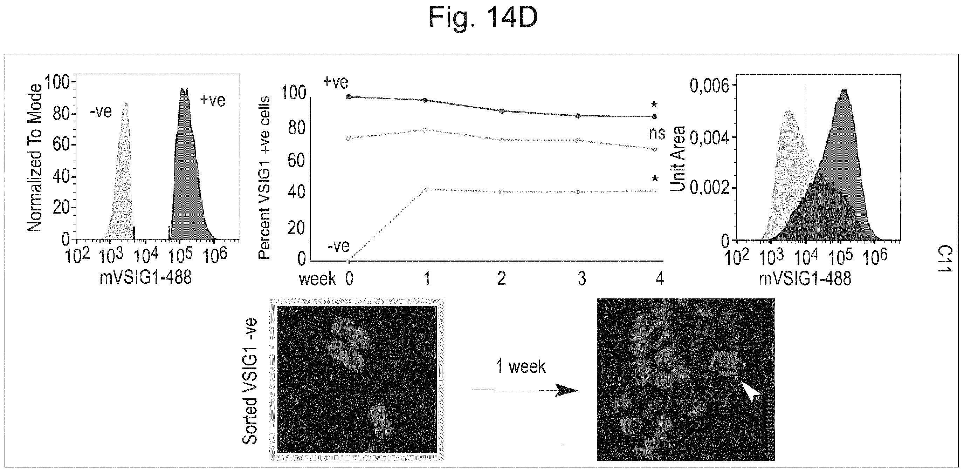

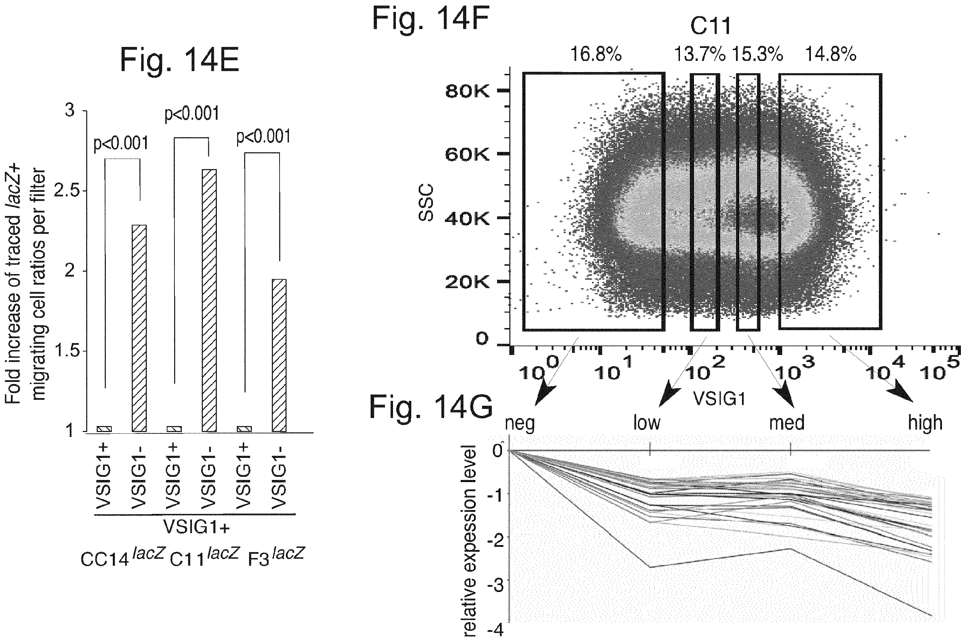

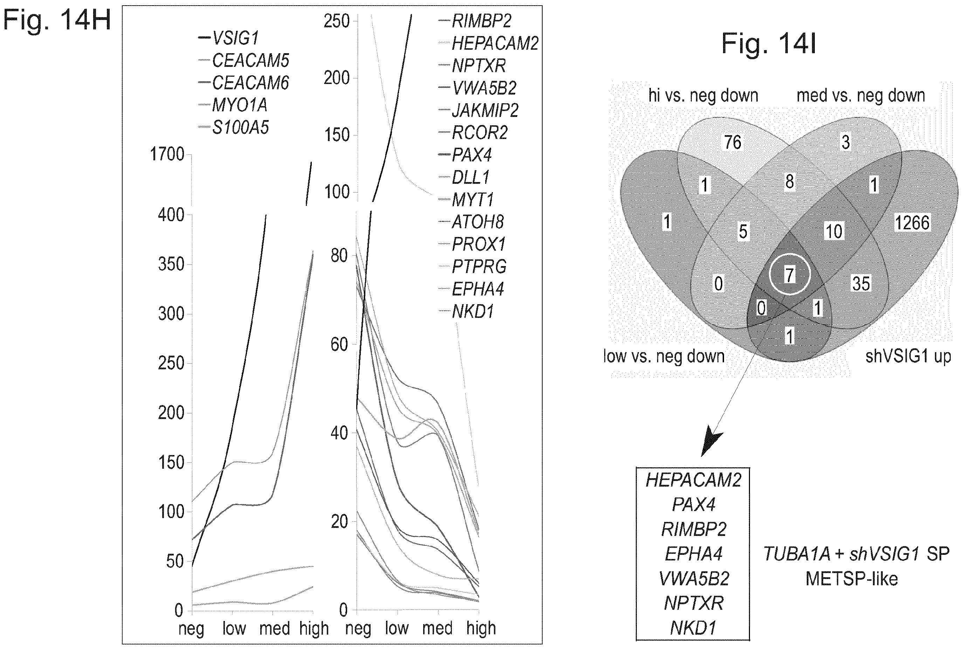

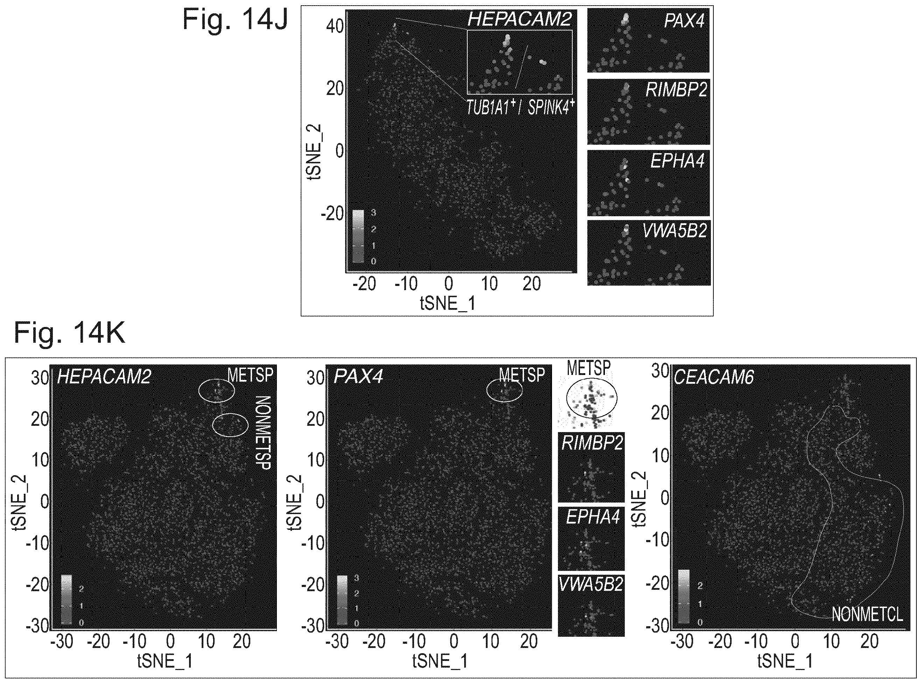

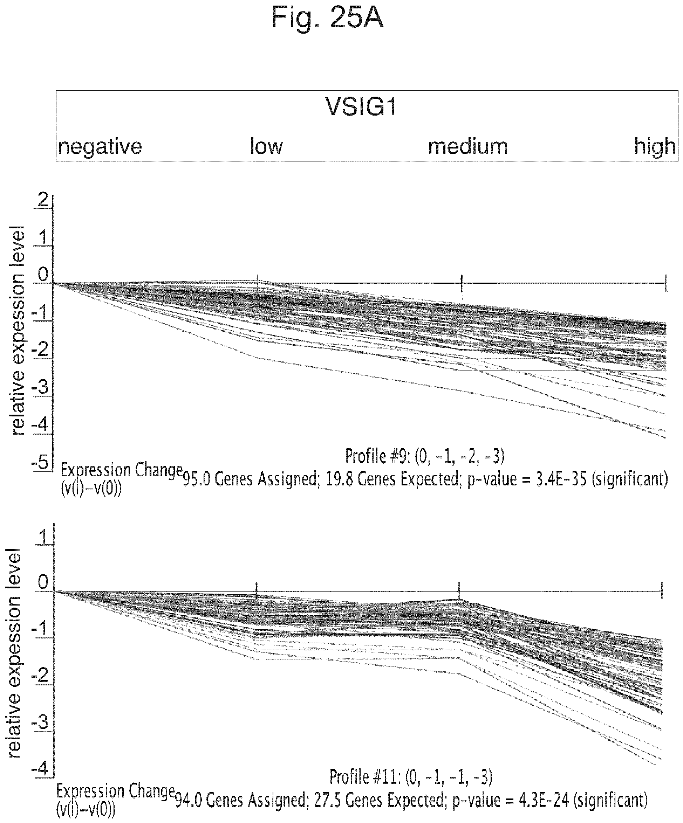

FIGURE 14 shows interconversion of VSIG1 positive and negative cells and inverse correlation of endogenous VSIG1 levels with METSP markers. A,B) FACS profiles (light gray) of CC14 (A) and C11 (B) cells including a technical negative control (no primary antibody, dark gray). The orange bar marks the threshold used to separate positive from negative cells. C,D) Temporally graded inter-conversion of positive and negative CC14 (C) and C11 (D) cells. Left graphs show the starting input of negative (yellow) and positive (purple) FACS-sorted cell populations for culture. Positive CC14 cells (left photo in C, purple frame) and negative C11 cells (left photo in D, yellow frame) gave rise to negative and positive clusters (right photos in C, D, arrows). Kinetic analyses (middle panels) showed a strong rate of inter-conversion within the 1st week (see Fig. S9), reaching near stable levels, which are cell-type-specific, by the 4th week. Asterisks or ns denote p<0.05 or p>0.05 vs. the starting point, respectively. E) FACS-sorted VSIG1 negative CC14lacZ+, C11lacZ+and F3lacZ+cells display enhanced transfilter migration as compared with their positive siblings. F,G) FACS gating of C11 positive (high, medium, low) and negative populations shown as boxes on the density plot was used to perform RNAseq in each one. Feeding the data to the STEM program allowed the identification of inversely correlated cohorts (G; see Fig. 25A). H) Identification of genes the expression of which is differential by at least 2-fold in the low vs. high pools (F,G) and regulated at least 2-fold by VSIG1 kd. The axis in the left panel is split to show the maximum levels of VSIG1 in the high pool. The right panels show the data at two scales. The y axes represent reads per million. I) Venn diagram of the intersection of negatively correlated genes to increasing VSIG1 levels >2 fold with p<0.01, as noted, and upregulated >2 fold in shVSIG1 C11 cells. J, K) tSNE maps showing the localization of cells expressing directly and inversely correlated genes that are also VSIG1-dependent in the C11shVSIG1 scRNAseq (J) and in the merged C11shVSIG1 and CC14 SCANORAMA (K). tSNE maps reveal the prominent expression of inversely correlated genes in the C11shVSIG1SPTUBA1A+ (J) and the METSP (K) populations. In contrast, directly correlated, VSIG1-dependent CEACAM5/6, and S100A5 showed expression in the NONMETCL (K and not shown). Few HEPACAM2+ cells were seen in the NONMETSP (J,K). The top inset in (K) denotes the METSP region from the SCANORAMA map in Fig. 13D as guide. A white dotted line separates the two subpopulations of the C11shVSIG1SP (J). Scale bar = 10µm (C,D). Error bars represent SEM. P values were determined using unpaired, two-tailed T-tests. Transfilter assays were performed in independent biological triplicates in which each one included technical duplicates.

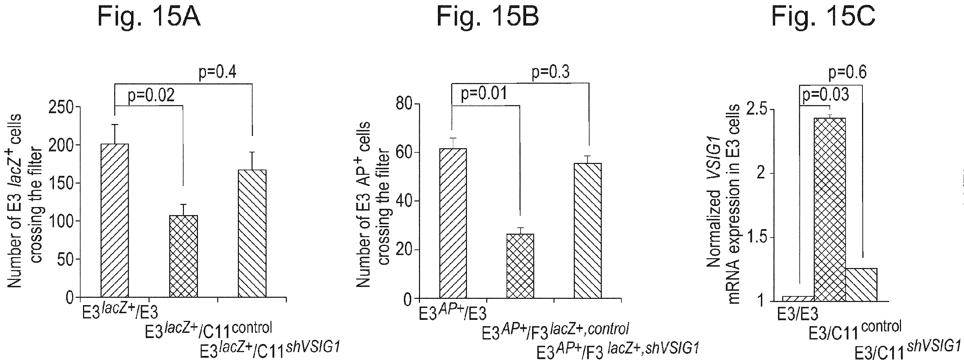

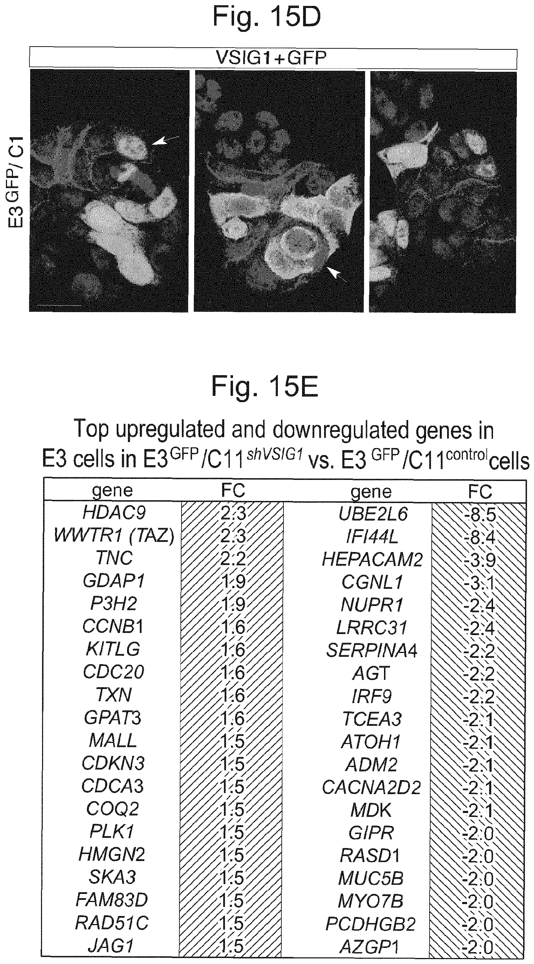

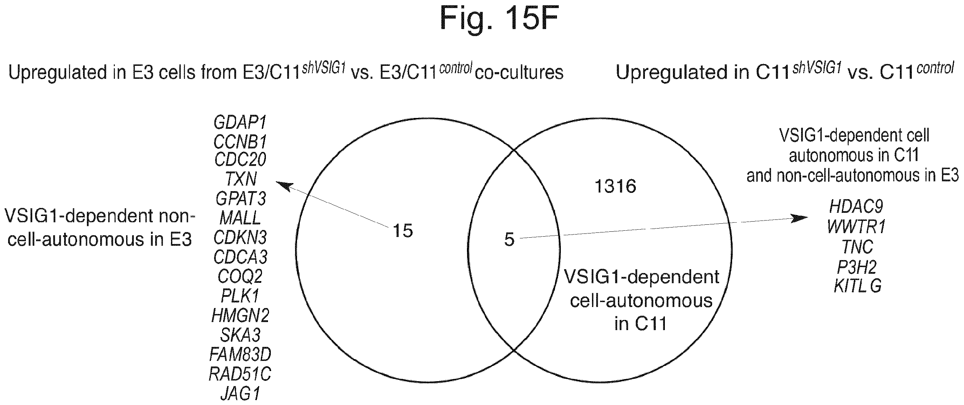

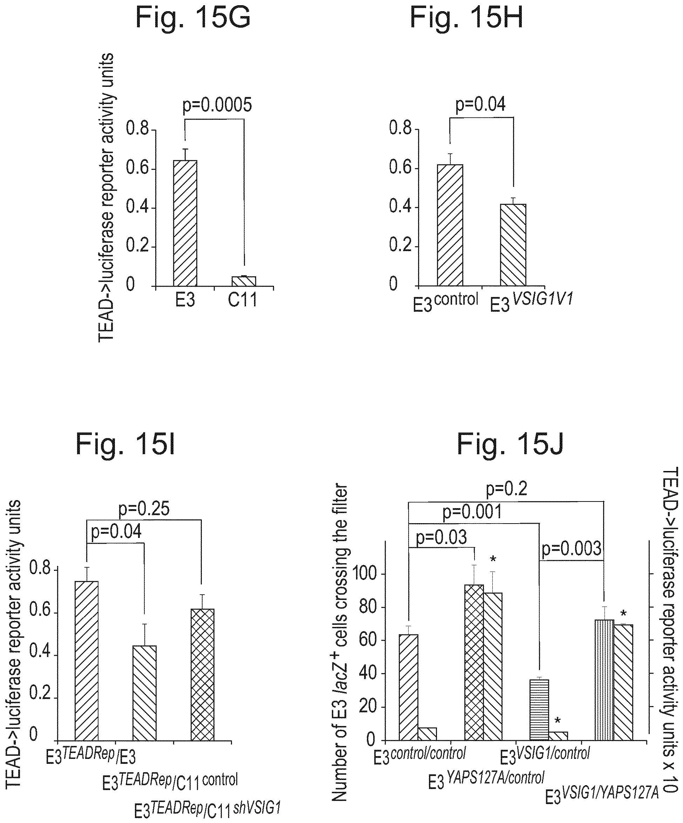

FIGURE 15 shows non cell-autonomous repression of metastatic behavior requires VSIG1 A,B) Quantification of transfilter assays with E3lacZ+ (A) or E3AP+ (B) in co-culture with C11 or C11shVSIG1 (A), and F3 or F3shVSIG1 (B). C) RT-qPCR quantification of the levels of VSIG1 mRNA expression in E3GFP+ cells co-cultured with sibling, C11 control or C11shVSIG1 cells. Y-axis values are ratios after normalization with those in E3/E3 samples. D) Confocal maximal projection image from z-stacks of the full extent of the cells showing VSIG1 expression (red) in E3GFP cells (arrows) in E3GFP /C11 co-cultures. The right panel shows a negative case. All nuclei were counterstained with Hoechst 33342. Scale bar = 15µm. E) Heat map table of RNAseq data from FACS sorted E3GFP cells from E3GFP /C11shVSIG1 vs. E3GFP /C11control co-cultures showing the top 20 up- or downregulated genes. F) Venn diagram showing the common genes upregulated 1.5-fold or more by cell-autonomous VSIG1 kd in C11 cells, and non-cell-autonomously in E3 cells through co-culture with C11shVSIG1. Only 5 are in common, which include the top three in (E); HDAC9, TAZ and TNC. See also Fig. S10D. G-I) Quantification of TEAD-luciferase reporter activity showing higher levels in E3 vs. C11 (G), its diminution by exogenous VSIG1 expressed in E3 cells (H) and the reversion of its repression in E3 cells by C11 when the latter have compromised levels of VSIG1 (I). Y-axes show relative activity levels after normalization with renilla controls. J) Bar graph showing TEAD->Luciferase reporter activity levels induced by activated YAP (YAP127A) in E3 cells under various conditions (hatched bars), and its rescue of the repression of transfilter migration by VSIG1 (VSIG-V1) expression (solid bars). Controls were empty vectors assuring the same amount of transfected DNA per sample. P values are given for the transfiter assay as indicated. Asterisks denote significant changes in TEAD->Luciferase activity over E3control/control cells. Neither transfilter migration nor TEAD->Luciferase activity were significantly different between 3YAPS127A/control and E3VSIG1/YAPS127A. RT-qPCR data derived from two biological replicates, each analysis was carried out with three technical replicates. Error bars represent SEM. P values were determined using unpaired, two-tailed T-tests. Transfilter and luciferase assays were performed in independent biological triplicates in which each one included technical duplicates

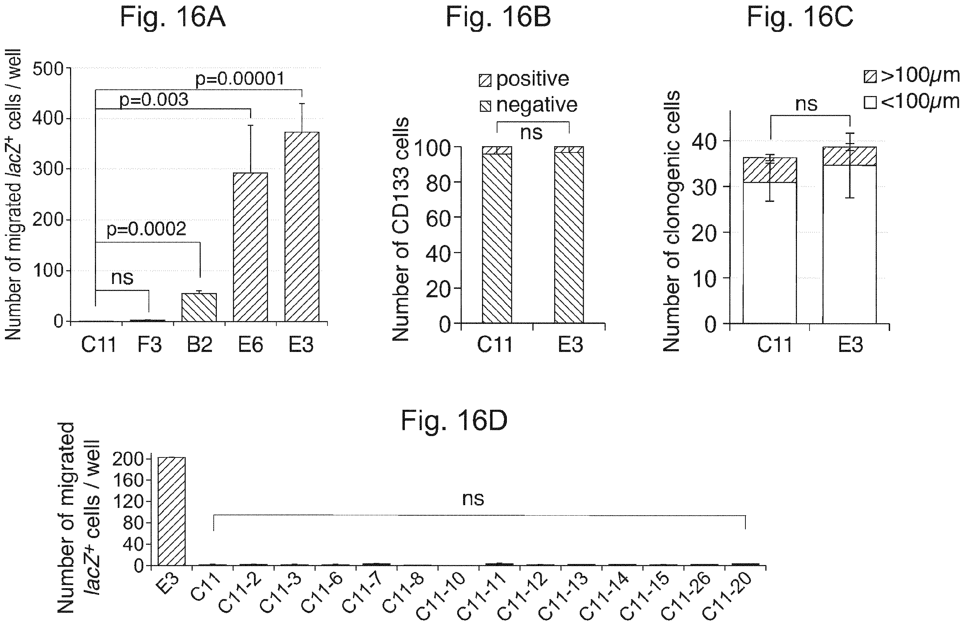

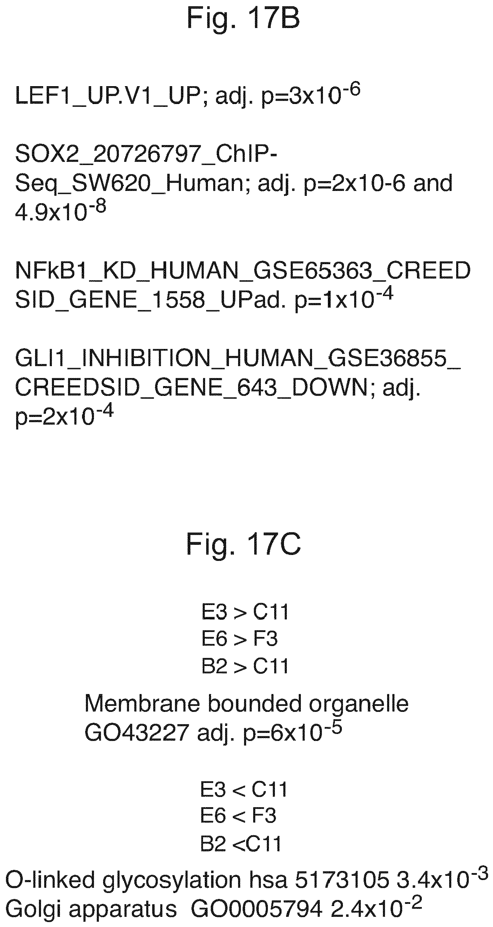

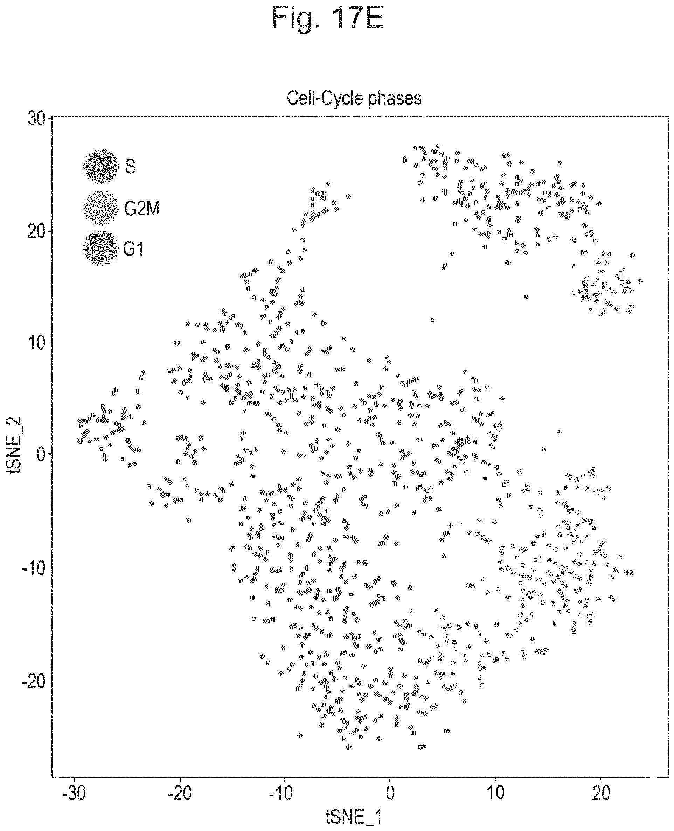

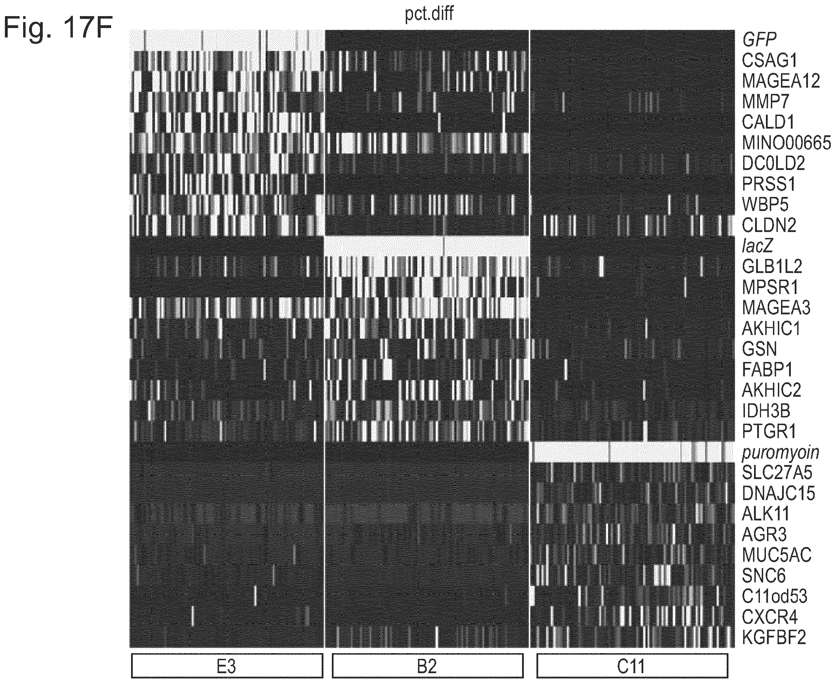

FIGURE 16 shows transfilter scores, CD133 content, clonogenic behavior of CC14 clones and summary of non-reference genome SNPs found in CC14, C11 or E3. Related to Figure 9. A) Quantification of the number of cells crossing the filter in transfilter assays of the listed clones, all after transduction with a lacZ expressing lentivector. p<0.05 in comparison to C11 are in red. Similar results were obtained in comparison with F3. Here and in all supplementary figures scale bars are s.e.m. This distribution was confirmed with 15 additional clones (not shown). B) Quantification of the number of MACS-isolated CD133+ and CD133- cells in C11 and E3. C) Quantification of the number of C11 and E3 cells giving rise to single-cell derived clones. D) Transfilter migration assays with E3 and C11 cells as controls (left colums) for those with thirteen C11 single-cell subclones. None showed a significative difference in regards to C11 (ns). The stability of the phenotypes was also demonstrated by similar transfilter efficiencies of C11, F3 and E3 and E6 cells after months in culture. E) The left columns show the allelic disposition of single nucleotide polymorphisms (SNPs) in the parental CC14 and derived C11 and E3 clones, the number of SNPs and the corresponding percentage. The middle column reports the highest concentration of SNPS in each category per chromosome (ch). The right column denotes the number of exonic SNPs and the number of STOP gain SNPs. The data derives from exome sequencing. F) Coherent and differential distribution of SNPs in the four indicated genes in low-metastatic (C11, F3) vs. high-metastatic (E3, E6) clones, and parental CC14 cells. Results are from genomic PCR followed by Sanger sequencing. FIGURE 17 shows enrichment prediction analyses for genes in high vs. low metastatic clones. Main and cell-cycle clusters from single-cell transcriptomic mapping of CC14, and top spiked clone markers. Related to Figures 9 and 10. A) STRING analysis of protein-protein interactions (PPI) for downregulated genes in common in high- (E3, E6) vs. low-metastatic (C11, F3) clones, with main biological processes indicated by color, name and adjusted p values. B,C) Enrichr analyses results between E3>C11 and E6>F3 for transcriptional regulators (B), and for biological processed upregulated in all comparisons between more-versus less-metastatic clone (C) comparisons, with identifier and adjusted p values. D,E) tSNE representations (D,E) showing two main clusters (D) or cell-cycle-related clusters (E). F) Heat map with high (yellow) and low (purple) values of top 10 differentially expressed genes by differential percentage of expressing cells. Note the genetic tracers for each spike: GFP for E3, lacZ for B2 and puromycin for C11. In a comparison of E3 vs. B2 spikes, exocytosis is the top biological process (GO:0045055 FDR=5.5x10-5).

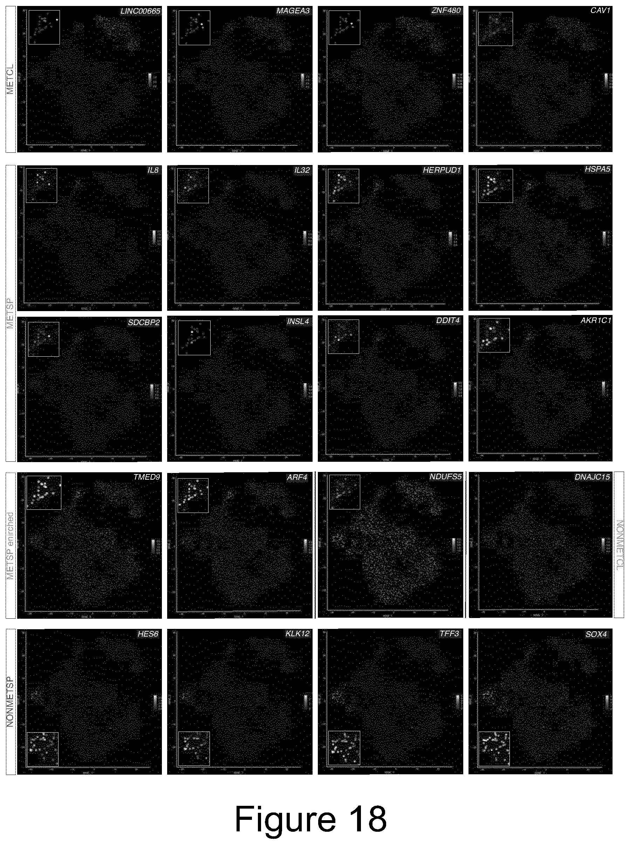

FIGURE 18 shows tSNE scRNAseq maps of CC14 highlighting the MET and NONMET main clusters (CL) and side populations (SP). Related to Figure 10. The maps show examples of genes marking the different clusters as marked. Markers widely expressed but impoverished in the METSP include NDUF55 (third row middle).

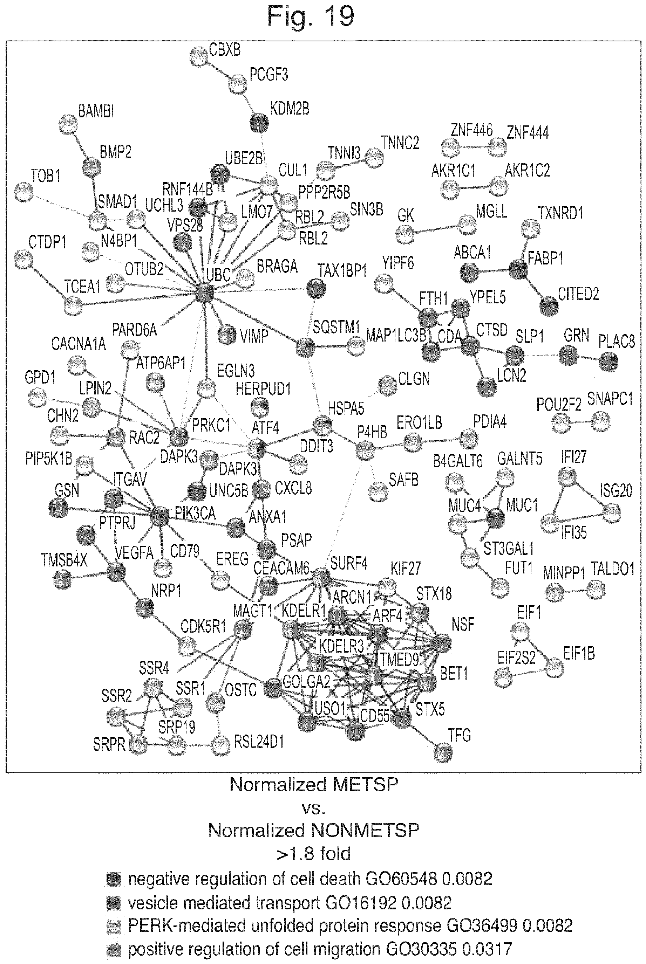

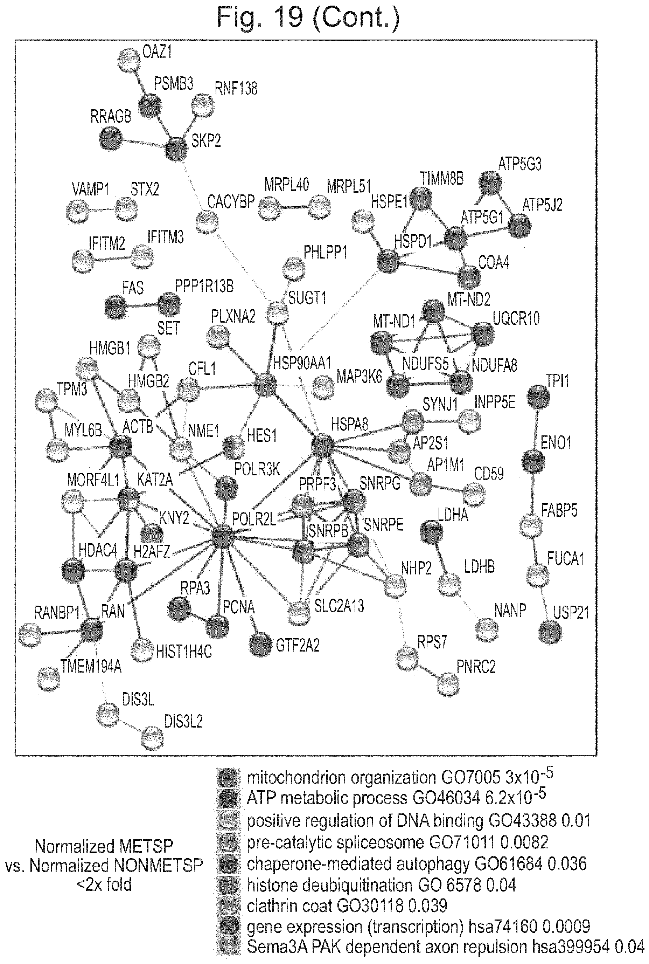

FIGURE 19 shows STRING protein-protein interaction analysis maps and gene sets enriched in genes found preferentially in the METSP (top) or NONMETSP (bottom). Related to Figure 10. 1.8-fold or more is used for the top comparison and less than 2-fold for the bottom one. Colors represent gene sets and individual components of these sets that map on the string map. GO or hsa identifier is given followed by the FDR value. Here and in all STRING maps only the interacting protein products are highlighted in the map but the enriched gene sets include isolated nodes not shown.

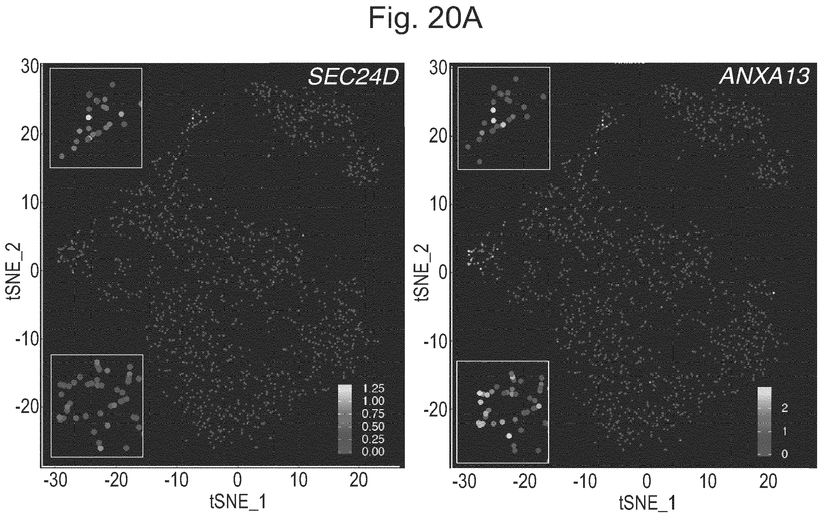

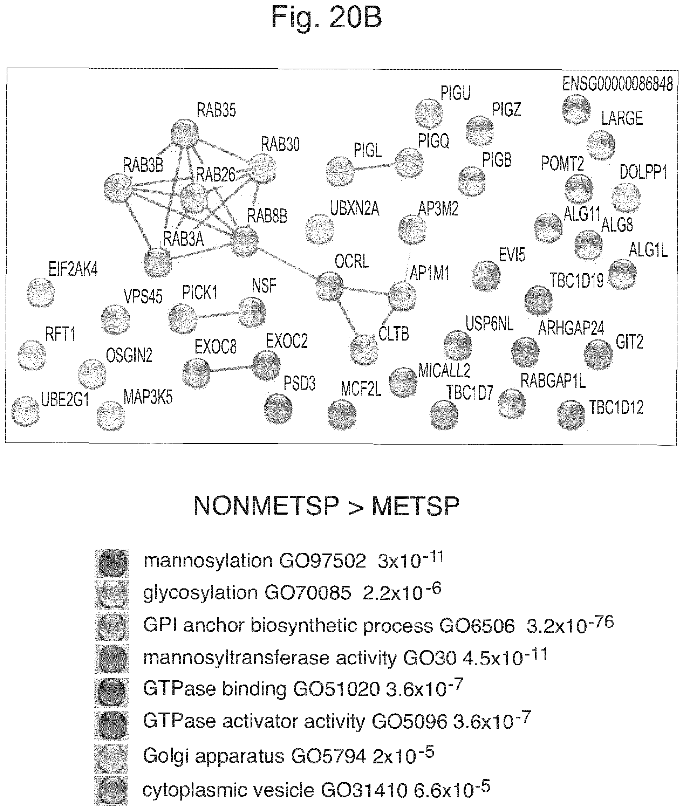

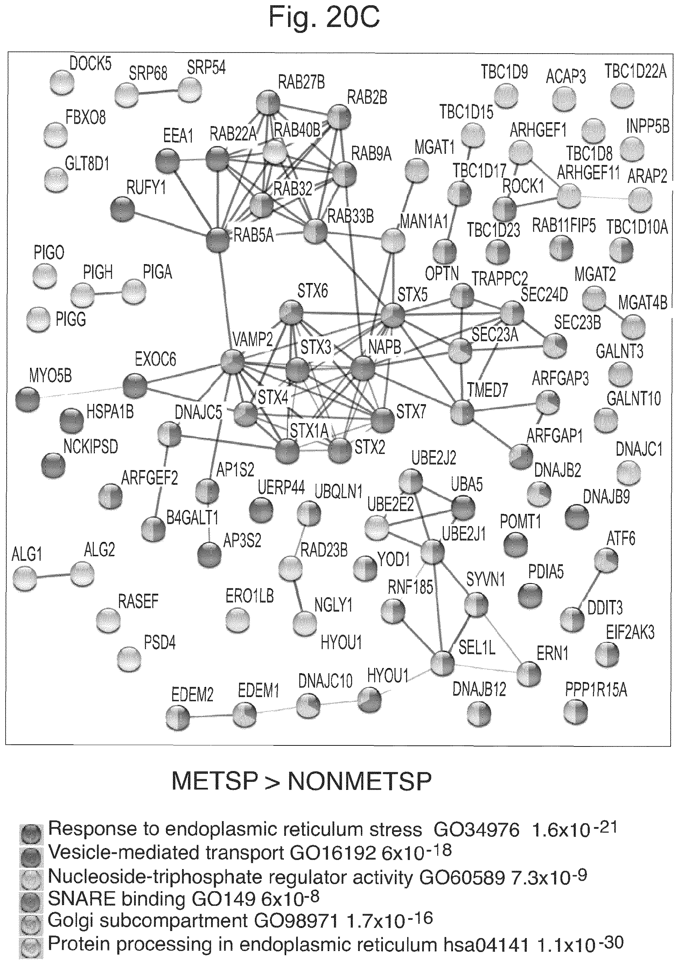

FIGURE 20 shows differential expression of secretory components in the METSP vs. the NONMETSP. Related to Figure 10. A) Localization of two genes involved in secretory aspects both the METSP and NONMETSP in CC14 scRNAseq tSNE map. B, C) 486 genes widely involved in secretory function (Feizi et al., 2017) detected in CC14 were tested for their expression in the METSP and NONMETSP since a number of genes were detected expressed in both as in (A). Genes from this get showing 2-fold or greater expression in the NONMETSP (characterized as Goblet-like) (B) or the METSP (C), after normalization by the values of the corresponding CL cluster, were analyzed with STRING and Enrichr. STRING PPI maps and enriched gene sets are shown for each case. All nodes are shown.

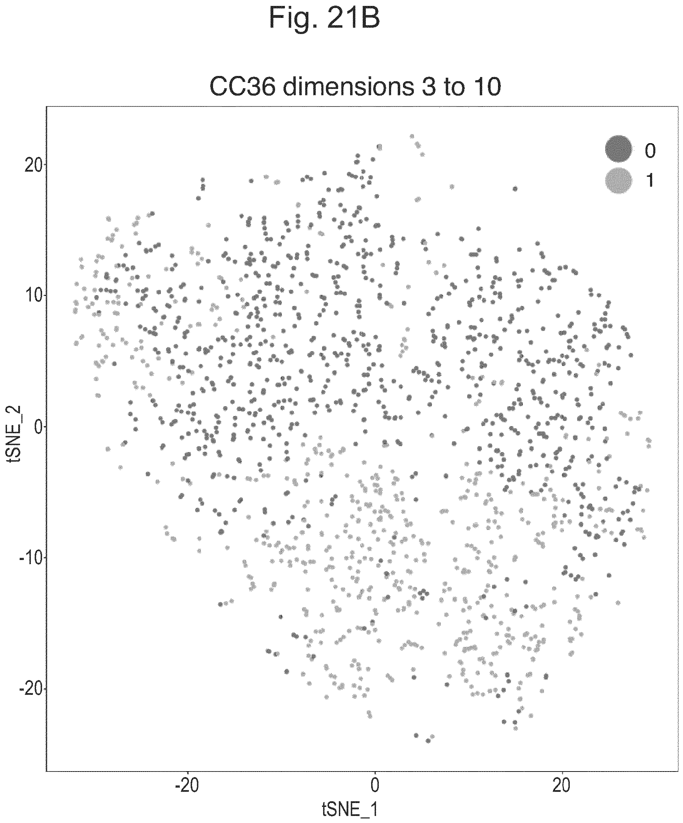

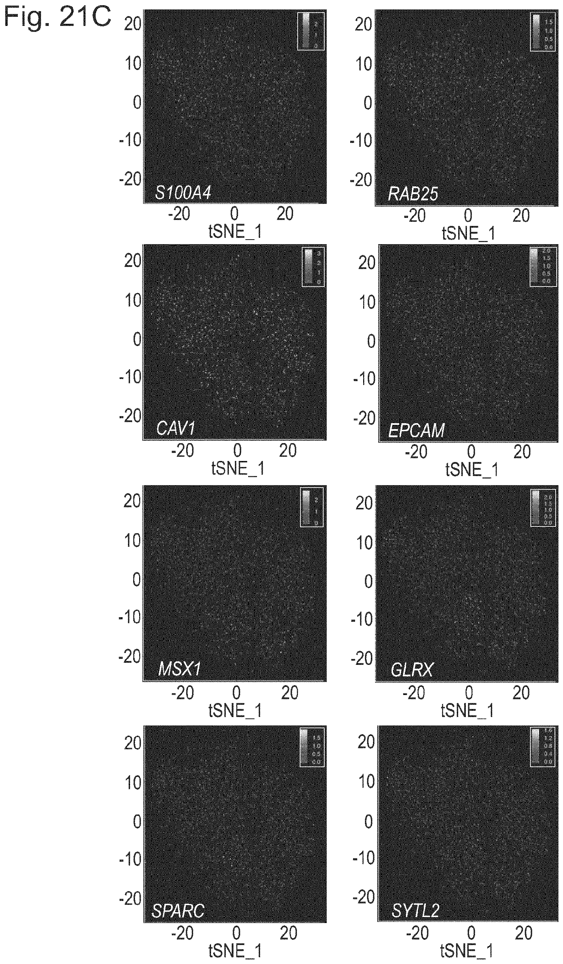

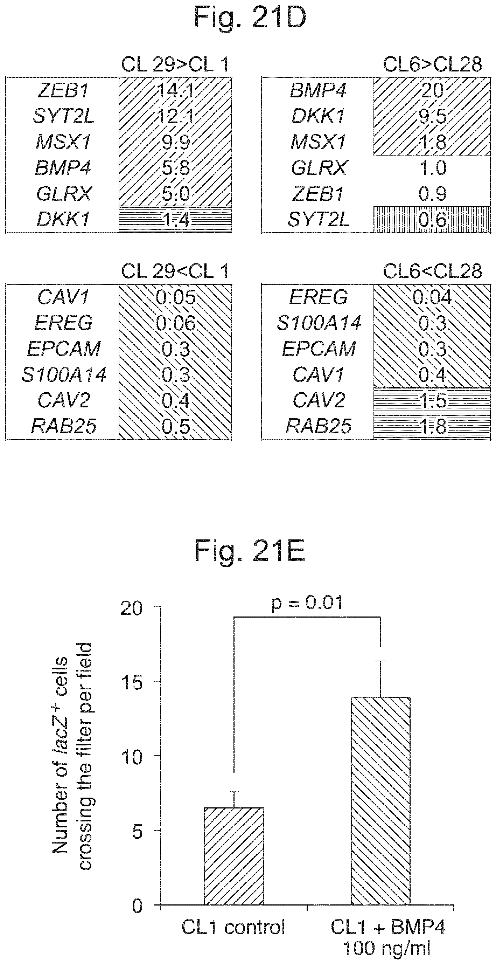

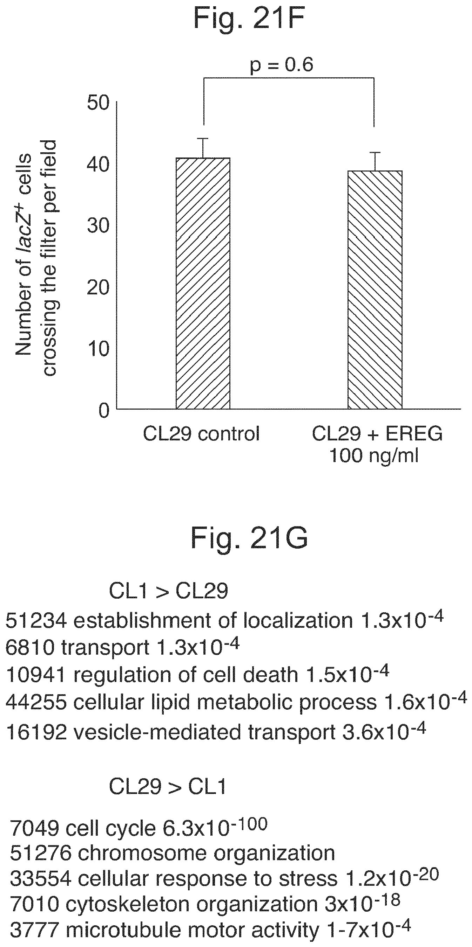

FIGURE 21 shows scRNAseq clusters, markers, enrichment analyses for CC36, PCRs of CC36 clones and effects of BMP4 and EREG ligands on CC36 clones. Related to Figure 10A,B) tSNE maps of scRNAseq data for CC36 showing cell cycle state cluster separation using the first two PCA dimensions (A) and the delineation of two clusters using PCA dimensions 3 to 10 (B). C) tSNE scRNAseq maps showing individual examples of markers for cluster 0 as in (B) (top 2 rows) and cluster 1 (bottom 2 rows). D) A set of differentially expressed genes in CL29 vs. CL1 detected by RNAseq were confirmed in these same cells by RT-qPCR (left columns) and tested in the same way in high-metastatic CL6 vs. low-metastatic CL28 (right columns). Note the good overall conservation with clone-specific differences, including EREG and BMP4. There was no inverted regulation above 2-fold for upregulated genes or below 2-fold for downregulated genes. E,F) Quantification of transfilter assays with CL1 (E) or CL29 (F) cells after treatment with BMP4 (E) or EPIREGULIN (EREG, F) ligands as noted, and as compared with untreated sibling cells. P values <0.05 are in red. G) Enriched gene sets are for PPI STRING analyses of genes 2-fold upregulated in CL1 vs. CL29 (upper set) or vice versa (lower set), with identifier, description and FDR value.

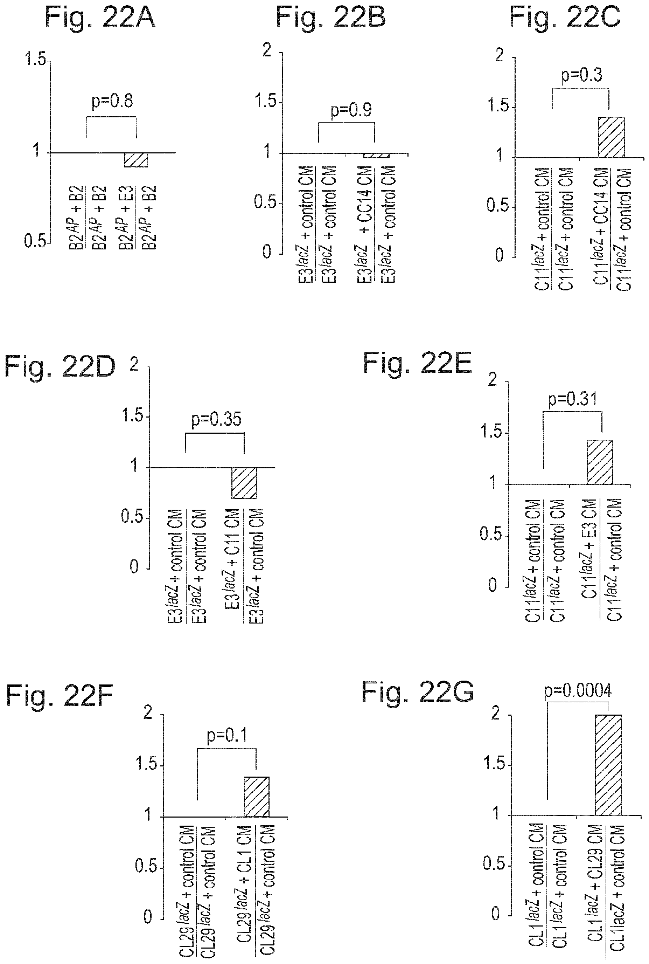

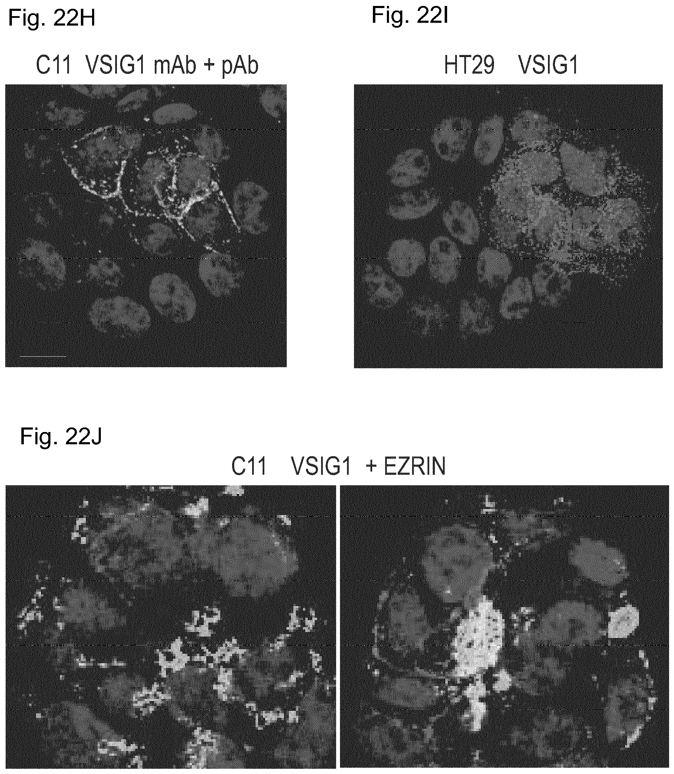

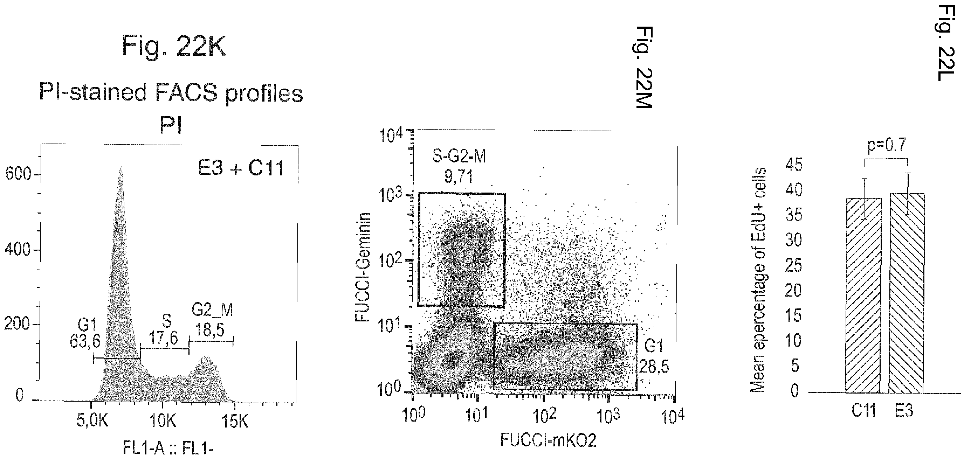

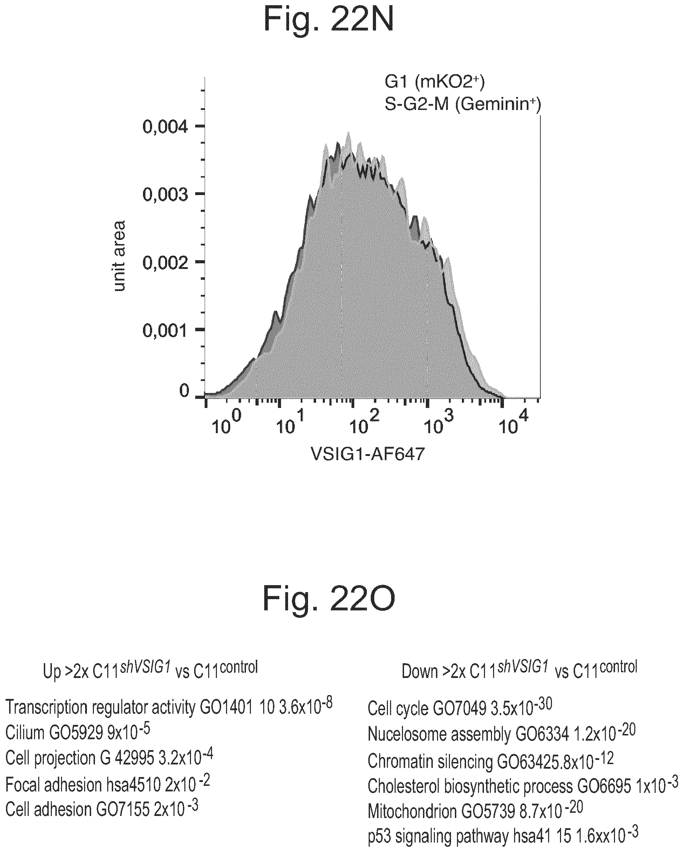

FIGURE 22 shows quantification of transfilter behavior in clone co-cultures or conditioned media, localization of VSIG1 protein and cell cycle analyses. Related to Figures 11 and 12. A-G) All panels show bar graphs depicting changes in the number of traced cells that crossed the filter after normalization by the result of control conditions (co-culture with same cells without tracer). A) Effects of co-cultured E3 on alkaline phosphatase (AP)-traced B2 cells. B-G) Effects of conditioned media from different cells, collected after 2 days and applied to traced cells in the transfilter assay. No significant effects are seen except with CC36 high-metastatic CL29 cells on lower-metastatic CL1 cells (G). p values <0.05 are in red. H) Double confocal single z-section image of immunolabeling C11 cells with both a monoclonal (mAb, red) and a polyclonal (pAb, green) antibodies against different epitopes of the VSIG1 protein. Since the polyclonal antibody labels transmembrane and cytoplasmic epitopes, cells were permeabilized. Extensive overlap is shown in orange/yellow. I) Localization of VSIG1 protein in HT29 cells using the anti-VSIG1 mAb. The maximal projection confocal image shows a rare positive cluster with heterogeneous membrane localization. J) Differential distribution of VSIG1 and EZRIN in C11 cells as noted. Minimal overlap was detected. K,L) E3 and C11 clones have similar cell cycle kinetics. The profiles of propidium iodide (PI) FACS-profiled E3 and C11 cells overlap, showing similar cell cycle distribution. Percentages for the main cell cycle phases (G1, S, G2-M) are given. Similarly, EdU-labeling (L) showed similar percentages of marked cells in both cases under standard culture conditions. M) FACS plot of C11 cells showing the distribution of cell cycle phases characterized by the FUCCI reporter labeling system in which Geminin+ cells represent those in S-G2-M and mKO2+ cells represent those in G1. The gates noted were used for the populations shown in (N). N) Overlapping distribution of VSIG1 positive cells labeled with AF647 (far red) in both the G1 (red) and S-G2-M phases (green) characterized by the FUCCI system (M). Nuclei were counterstained with Hoechst 33342 (H-J). O) STRING and Enrichr analyses for 2-fold or higher upregulated (Up) and downregulated (Down) genes from C11shVSIG1 vs. C11control RNAseq. Description, identifier and adjusted p value are noted. Scale bar = 15µm (H,I), 10µm (J).
FIGURE 23 two side populations are detected in C11shVSIG1 cells. Related to Figure 13. A) Heat map of genes tracking the differential expression of TUBA1A or SPINK4 in the two horns of the C11shVSIG1 side population. Brightest yellow denotes higher expression and black lowest. B) tSNE map of scRNAseq of C11shVSIG1 cells highlighting the side population split into two horns, a TUBA1A+ (turquoise) and a SPIN4+ (red) pool. C) tSNE maps for individual genes as noted showing the distribution of expressing cells in the C11shVSIG1 horns. Note differential (upper three rows) or shared expression albeit at different levels (bottom row). As control, the absence of VSIG1 from the TUBA1A+ and SPINK4+ populations is shown (bottom right).

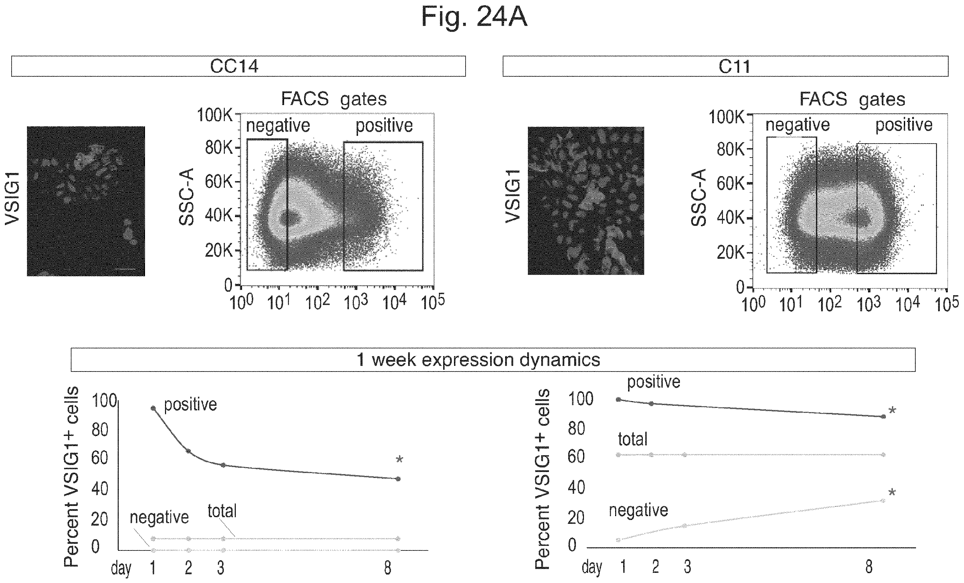

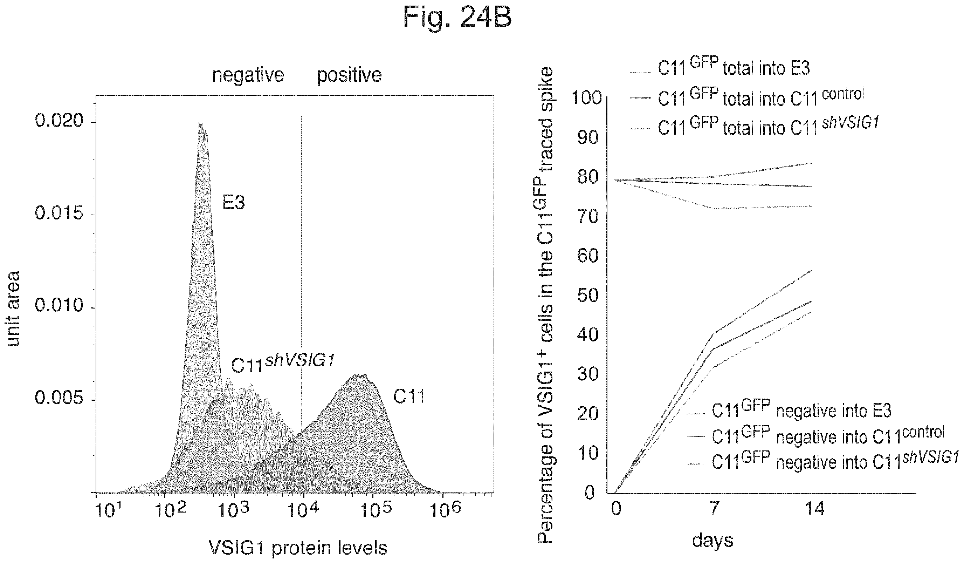

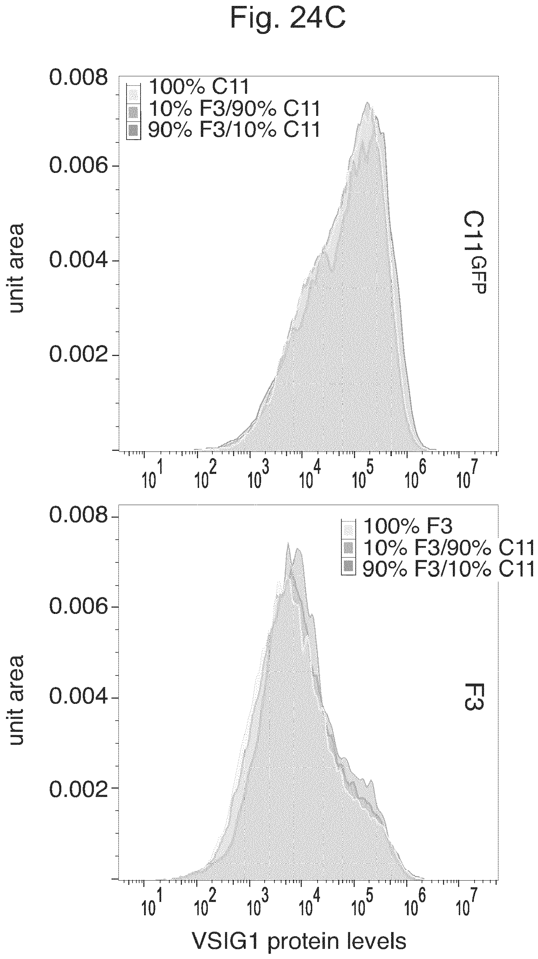

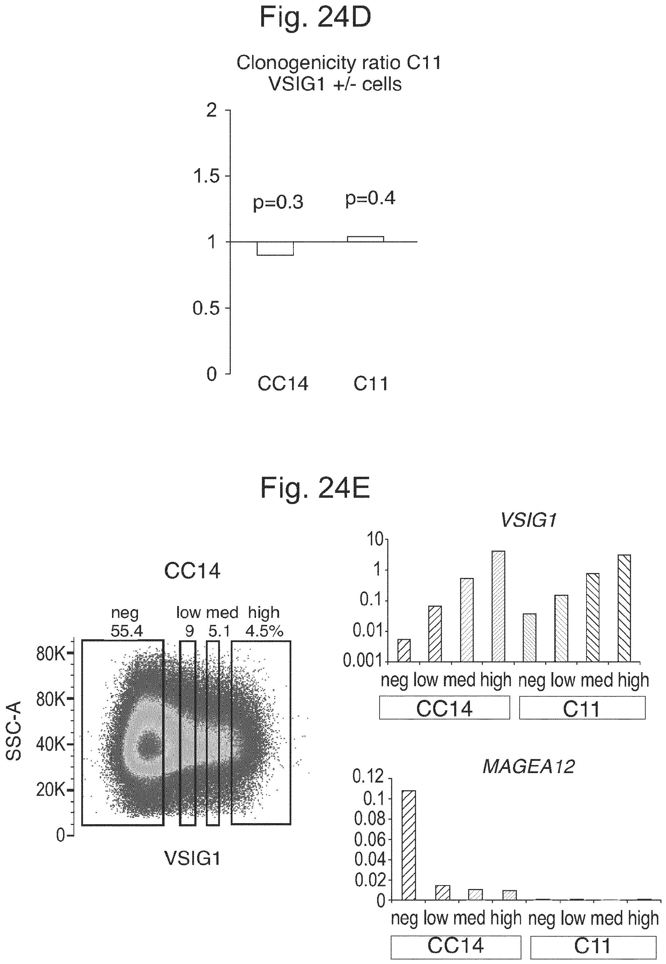

FIGURE 24 dynamics of changes in VSIG1 protein levels and characterization of cell populations with different VSIG1 levels. Related to Figure 14. A) CC14 (left) and C11 (right) cells showing the presence of VSIG1 positive cells in clusters (photos) and the FACS density plots of the distribution of VSIG1 expression. Bottom panels show the interconversion of positive and negative cells over 8 days of FACS-sorted CC14 cells (left) or C11-cells (right) revealed by immunofluorescence microscopy. B) Interconversion of GFP-traced VSIG1- C11 cells into VSIG1+ cells is not affected by cell context. Left panel) Superimposed FACS profiles of VSIG1 expression in E3, C11 and C11shVSIG cells. Note that E3 cells are VSIG1 negative and kd of VSIG1 in C11 cells leads to a major shift of the distribution towards the negative area. These three kinds of cells are used as dilutant for traced C11GFP cells in the experiments shown in the right panel. Right panel) Superimposed kinetics over 14d of the levels of VSIG1 protein analyzed by FACS in spiked C11GFP cells mixed with E3, C11 and C11shVSIG cells. The bottom three curves show the similar kinetics of gain of VSIG1 expression from VSIG1- C11GFP cells after mixing with E3, C11 or C11shVSIG1 untraced cells. As controls, the total C11GFP population was mixed with the same three cell types also showing no major change. C) Quantification by flow cytometry of the proportion of VSIG1+ cells in C11 and F3 cells as well as in mixtures of these clones in 90/10 and 10/90 ratios. Note the overlap of the profiles in each case. D) Quantification of the number of clonogenic cells present in FACS-sorted VSIG1 positive and negative cells. The bar graph shows clonogenic ratios of the FACS-sorted VSIG1+/- populations. E) Fine FACS gating of negative, low, middle, and high VSIG1 expressing cells in CC14 (left panel) and C11 (see Fig. 6F) followed by RNA extraction and RT-qPCR for selected markers showed the tracking of VSIG1 mRNA levels to VSIG1 protein levels and the expression of the METCL marker MAGEA12 only in CC14 VSIG1negative cells and not in C11 cells, as expected. Asterisks denote p<0.05 of the point of highest change versus the starting point. Scale bar = 20µm (A).

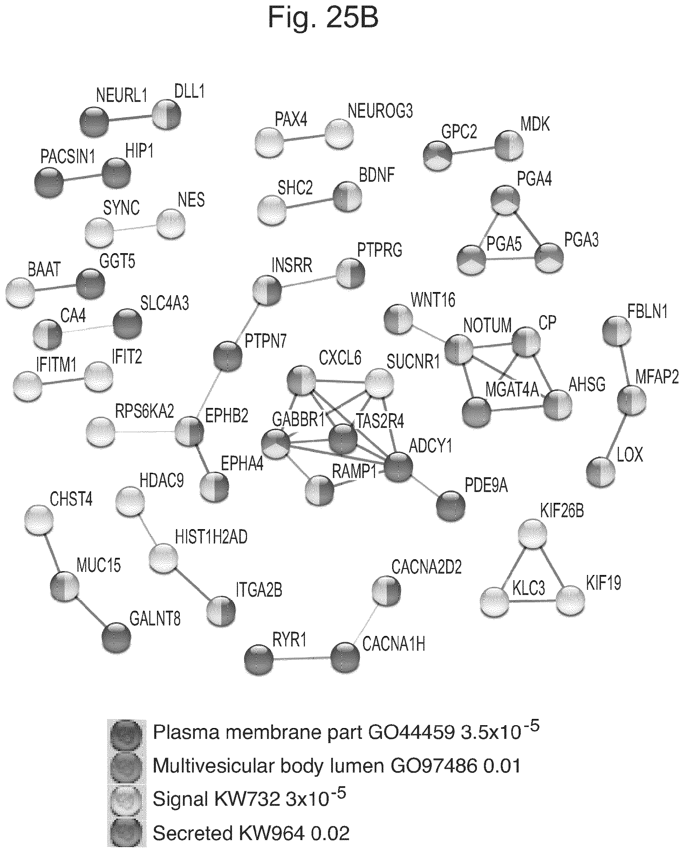

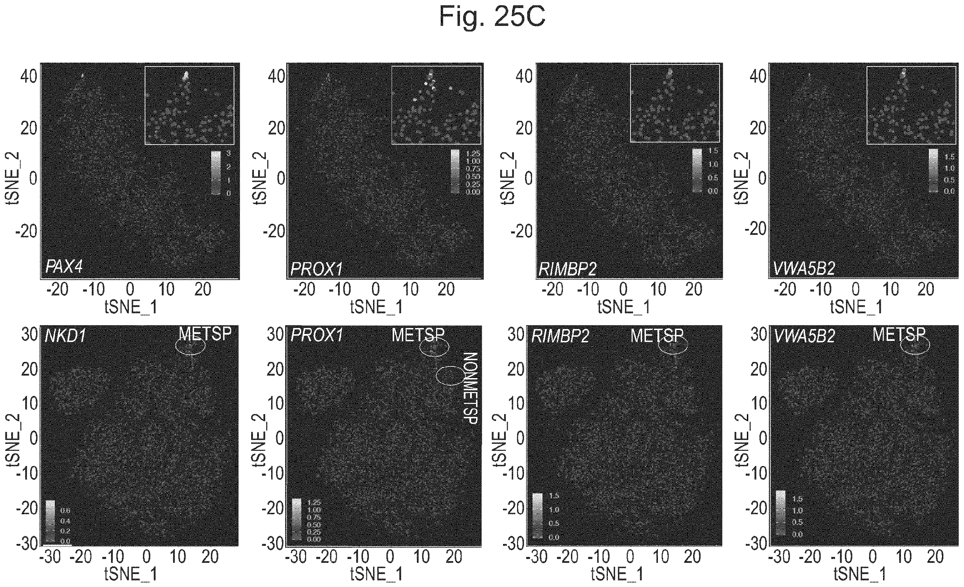

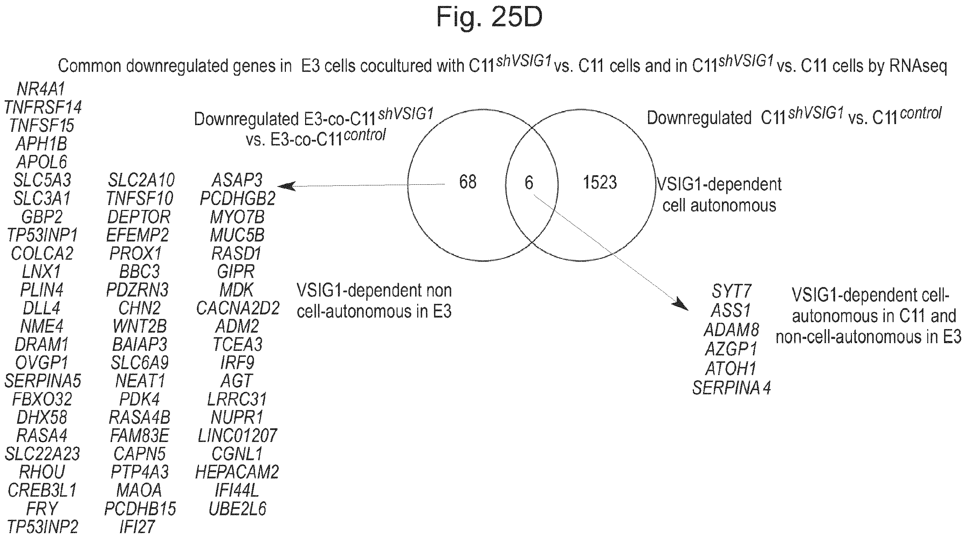

FIGURE 25 shows significant STEM profiles of genes from VSIG1 FACS-sorted cells, STRING analyses of these genes and individual tSNE scRNAseq maps showing the localization of inversely and directly VSIG1-correlated genes, and cell-autonomous and non-cell-autonomous regulation of gene expression by VSIG1. Related to Figures 14 and 15. A) One significant cluster with three similar patterns of gene expression was obtained by comparing negative, low, middle and high VSIG1 cell RNAseq data. One pattern is shown in Fig. 6G and the two others here. All included the same inversely correlated trend to VSIG1 levels although individual genes (each gene represented by an individual lines) show different correlations. For instance, some are already low in low-expressing VSIG1 cells while others have no change in these cells, all in relation to the levels in negative cells. B) Protein-protein interaction prediction STRING analyses of the 223 genes in the three significant STEM profiles of VSIG1-sorted C11 cells highlighted plasma membrane, multivesicular body lumen, signal and secreted components, including a small kinesin interactome. The PPI map's colors match the codes (top right), highlighted here to maximize the number of related nodes with distinct categories. Enrichr analyses of this same set revealed a YAP regulated gene cohort (see main text) as well as synaptogenesis (G07416, adj. p=0.02), calcium-dependent cell adhesion with four protocadherins (GO16339, adj. p=0.03) and the multivesicular body lumen (Jensen compartments, adj. p=0.03). C) tSNE maps with highlighted cells for the expression of individual genes show that inversely correlated genes to VSIG1 levels are mostly found in the TUBA1A+ horn of the of C11shVSIG1SP (top row) and in the METSP in combined CC14 and C11shVSIG1 SCANORAMA maps (bottom rows. As an independent test we mapped 158 genes identified as differentially expressed in C11 high VSIG1+ vs. VSIG1-cells having an opposite trend to increasing VSIG1 levels. Of these, 88% were expressed by C11shVSIG1SP cells, showing high congruency between the two approaches, with 31% being only expressed in this small population. Moreover, of this 31%, 60% were exclusively localized to the TUB1A1+ horn (not shown), which corresponds to the METSP. D) Venn diagram of the overlap of gene sets repressed in E3 cells in co-culture with C11shVSIG1 cells, over control, and those repressed in C11 cells with kd of VSIG1, over control. Six genes are both cell-autonomously and non-cell-autonomously regulated. See Fig. 7F for upregulated genes.

### Brief Description of the Sequences

SEQ ID NO: 115 is an open reading frame nucleotide sequence encoding the VSIG1 protein (Origene RC230056L2).
SEQ ID NO: 116 is an open reading frame nucleotide sequence encoding the VSIG1 protein (Origene RC207391L2).

### Detailed Description of the Invention

It is to be understood that different applications of the disclosed methods may be tailored to the specific needs in the art. It is also to be understood that the terminology used herein is for the purpose of describing particular embodiments of the invention only, and is not intended to be limiting.

In addition as used in this specification and the appended claims, the singular forms "a", "an", and "the" include plural referents unless the content clearly dictates otherwise. Thus, for example, reference to "a method" includes "methods", and the like.

The terms a "patient", "subject" and "individual" are used interchangeable and typically refer to a human.

All publications, patents and patent applications cited herein, whether supra or infra, are hereby incorporated by reference in their entirety.

### Methods for identifying a cluster of cells associated with a phenotype

A problem in the study of cell behaviour, both in the context of healthy tissue physiology and in the context of diseased tissue, is the inability to accurately define groups *(i.e.* "clusters") of cells within a heterogeneous cell population according to their function. The present invention solves this problem by providing a method for identifying clusters of cells associated with a particular phenotype.

The invention provides a method for identifying a cluster of cells associated with a phenotype, wherein the method comprises:
a. spiking a heterogeneous population of cells with a tagged phenotypically characterised single cell; and
b. analysing single cell omics data from said spiked population of cells, wherein presence of the tagged single cell within a cluster of cells in the population identifies said cluster as associated with said phenotype.

A "cluster of cells" refers to two or more cells within a larger population of cells which associate (*i.e.* cluster) with one another on the basis of similarity with respect to at least one variable. An association which leads to the formation of a cluster may also be described as covariance in the two or more cells of a particular variable. In the methods described herein, the variable is typically determined via the analysis of single cell omics data. In other aspects, the variable may be determined by analysis of molecular (e.g. genomic, epigenomic, transcriptomic, metabolomic and proteomic, particularly transcriptomic) or other data for a particular set of genes from the spiked population of cells.

Clustering methods are well known in the art and any suitable clustering method may be applied to the method of the invention. The method of clustering may depend on the type of omics data analysed in the method described herein.

In the methods described herein, the cluster of cells is defined within a heterogeneous population of cells. Thus, the population of cells contains a plurality of cells comprising more than one distinct cell type. There is no upper limit in the number of cells which may be defined to form a cluster, thus there is no upper limit in the number of cells which form the heterogeneous population of cells. To allow at least one cluster of cells to be formed within the heterogeneous population of cells, the heterogeneous population of cells must contain at least three cells. The population is heterogeneous on the basis of the population containing at least two cells of distinct cell types, wherein "distinct" is defined according to variability between cells with respect to at least one variable. In the methods described herein, the variable is determined on the basis of analysing single cell omics data. Any single cell omics method of analysis may be used in order to determine cell variability and consequently cluster cells within the heterogeneous cell population. In particular, variable characteristics may be determined by the single cell omics analysis of any one or more of the genomic, epigenomic, transcriptomic, metabolomic and proteomic content of the cells in the heterogeneous population.

The number of cells that form a cluster may depend on the total number of spiked cells subjected to an omics analysis, and further the number of cells subjected to an omics can vary particularly on the basis of the omics discipline use in the method. A person skilled in the art is able to determine the number of cells to be used in an omics analysis to generate clusters of interest, and to adapt parameters to a given analysis, based on their common general knowledge, and depending for example on the nature of the omics discipline and the phenotype of interest. Methods of determining cell numbers for omics analysis are well known in the art and described for example at https://satijalab.org/howmanycells. As described in the Examples, when a transcriptomics analysis is applied to the method of the invention, 1500-2000 cells in the omics analysis may be captured in order to reach 99% probability of seeing at least 50 cells per cluster assuming 2-4 cell types or states with a low-end frequency of 5%. A person skilled in the art is further able to determine an appropriate level of spiking with a phenotypically characterised cell for segregation of tagged cells within clusters of interest, and to adapt spiking parameters to a given analysis, as discussed below.

The heterogeneous population of cells is preferably a sample derived from a subject. The subject may have, for example, been derived from a human or animal. The cells in the sample are preferably capable of being cultured *in vitro.* Preferably, the cells in the sample are stable when cultured *in vitro,* as demonstrated by their ability to be passaged whilst maintaining their morphology and/or their phenotype. Maintenance of phenotype by a cell can be assessed by consistent phenotypic behaviour of the cell in one or more phenotypic assays following multiple cell divisions and passages after first being derived from a subject.

The heterogeneous population of cells may be obtained from a tumour biopsy. Any phenotypic trait of cancer may be assessed and associated with a cluster of cells, for example, metastasis and/or responsiveness to a medicament. Responsiveness to a medicament may provide the clinician with insight into therapeutics that may be particularly suitable for the subject *i.e.* personalised medicine. Preferably, the cells in the sample are capable of being implanted and grown in a model organism such as a mouse. This is especially preferable if the phenotype to be assessed is metastasis.

The purpose of the method of the invention is to provide a means for identifying a cluster of cells associated with a phenotype. The phenotype to be associated with the cluster of cells can be any phenotype. The phenotype may be associated with the functioning of normal cells within healthy tissue. The phenotype may be associated with the functioning of abnormal cells within diseased tissue. The phenotype may be associated with naturally disease-causing mutations or of induced (e.g. CRISPR/CAS9) mutations in experimental systems. The purpose of the method may be to allow the selection an efficacious, preferably optimally efficacious medicament for a particular patient by associating the efficacy of said medicament with sub-populations (i.e. clusters) of cells within a heterogeneous population of cells in diseased tissue. The purpose of the method may be to allow the determination of cell identity in the brain by associating phenotype with single cell state. Consequently, the association of phenotype with single cell state in the brain allows the development, testing and selection of the most efficacious drugs for sub populations (i.e. clusters) of cells in the brain. The purpose of the method may be to allow the determination of gene actions and drug actions to induce differentiation of stem cell-derived cells. The purpose of the method may be to allow the identification medicaments which target specific subpopulations of cells in order to induce specific phenotypes in said cells.

In any of the methods described herein, the method may involve phenotypically characterising a single cell or the method may involve the use of a single cell which has already been phenotypically characterised. In the methods described herein, a "phenotypically characterised single cell" should be understood as referring to one cell exhibiting a particular phenotype, or a population of such a cell. The single cell may be a single cell that has been isolated from the heterogeneous population of cells. The method of isolating the single cell can be through the use of any suitable method known in the art. For example, the single cell may be isolated and propagated by any suitable cloning technique (see, for example, those used in the Examples). In other instances, the single cell may be phenotypically characterised prior to being labelled or isolated, preferably wherein the single cell is a post-mitotic neuron and the phenotypic characterisation is via a patch-clamp technique.

The phenotypic characterisation of a single cell may involve, where necessary, comparison with a control cell, preferably wherein the control cell is assayed by the phenotypic characterisation assay. The control cell may be derived from the same heterogeneous population of cells as the single cell to be phenotypically characterised, preferably wherein the control cell has a contrasting phenotype to the single cell to be phenotypically characterised (e.g. non-metastatic vs. metastatic). The control cell may be derived from the same organism and/or tissue as the heterogeneous population of cells, for example from adjacent healthy tissue should the heterogeneous population of cells be derived from diseased tissue. The control cell may be a cell that is not derived from the heterogeneous population of cells, preferably wherein the control cell is derived from a cell line.

In some of the methods described herein, the single cell is not phenotypically characterised at the single cell level. Rather, the single cell may be cultured to form a larger homogeneous population of cells that is capable of being subjected to an assay for determining a phenotype which can then serve as an indicator for the phenotype of the single cell.

In some of the methods described herein, the single cell is phenotypically characterised at the single cell level. This is particularly suitable for phenotypically characterising a neuronal cell, or any cell for ion channel activity.

In any of the methods described herein, the phenotypically characterised single cell may be a "standard", wherein the phenotypically characterised single cell is derived from a population of cells other than the heterogeneous population of cells. For example, the "standard" single cell may be derived from a heterologous population of cells from a different source or from a cell line, for example a commercially available cell line, wherein the derived single cell has been subjected to phenotypic characterization, thereby allowing the attribution of said phenotype to the "standard" single cell.

The method of the invention is preferably for identifying a cluster of cells with a metastatic phenotype in a heterogeneous tumour cell population. Preferably, a single cell from the heterogeneous tumour cell population is phenotypically characterised to determine whether said cell has a metastatic phenotype. Metastatic phenotype may be determined, or inferred, by any suitable method known in the art. For example, a single cell exhibiting a high cell motility and/or invasiveness via *in vitro* assays such as a scratch assay or a transfilter migration assay could be inferred as having a metastatic phenotype. By way of a further example, a single cell which establishes lung metastases could be determined as having a metastatic phenotype.

In any of the methods described herein, the heterogeneous population of cells is spiked with a tagged phenotypically characterised single cell. The single cell may be tagged by any known method in the art, provided that the tag allows for identification of the omics data of the phenotypically characterised single cell among the single cell omics datasets of the cells within the heterogeneous population of cells. The tag utilised in the method therefore depends on the method of omics analysis used. For example, the single cell may be transduced with a nucleic acid sequence which comprises a heterologous gene that allows identification in genomic, proteomic and transcriptomic assays. The heterologous gene may, for example, encode one or more of *GFP, puromycin* and *lacZ.*

In methods of the invention wherein the single cell is phenotypically characterised prior to being labelled or isolated, the single cell may for example be labelled whilst subject to a patch clamp, for example by an optogenetic-based method. Such optogenetic labelling based methods are well known in the art.

Spiking the heterogeneous population of cells with a tagged phenotypically characterised single cell may involve mixing the heterogeneous population of cells with the tagged phenotypically characterised single cell to a proportion of the total number of the heterogeneous population of cells. The method of the invention is capable of successfully identifying one or more clusters of cells each associated with one or more phenotypically characterised single cells that are spiked into the heterogeneous population of cells, provided that each spiked single cell is detectable via a distinct label in an omics dataset. The detectability may depend on the single cell omics discipline applied and the depth of omics data that the technology of the omics discipline is capable of acquiring.

The proportion of phenotypically characterised single cells spiked into the heterogeneous population of cells can vary. The proportion of spiked cells may particularly vary depending on the omics discipline applied to the method and the depth of omics data that the technology of the omics discipline is capable of acquiring. The proportion of spiked cells should be sufficient to allow identification of the spiked cells in the omics data with sufficient resolution such that the spiked single cell omics data can be applied to a clustering analysis and potentially cluster with two or more cells in the heterogeneous population of cells. For example, the spiked cells may be identified at least one data point (e.g. a sequence read) in the omics data corresponding to the tag of the spiked cell, more preferably at least two data points, and most preferably at least three data points. Relative to the overall number of cells to be subject to a single cell omics analysis, the spiked cell may account for less than about 1% of the cells. Relative to the overall number of cells to be subject to a single cell omics analysis, the spiked cell may account for about 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, or 50% of the cells. Relative to the overall number of cells to be subject to a single cell omics analysis, the spiked cell may account for more than about 50% of the cells. Preferably, relative to the overall number of cells to be subject to a single cell omics analysis, the spiked cell may account for at least about 0.1% to 50% of the cells, or more preferably about 0.5% to 30% of the cells. Preferably, the spiked cell may account for no more than about 50%, 40% or 30% of the cells. More preferably, relative to the overall number of cells to be subject to a single cell omics analysis, the spiked cell may account for at least about 1%, 2%, 3%, 4% or 5% of the cells. Most preferably, relative to the overall number of cells to be subject to a single cell omics analysis, the spiked cell may account for about 5% of the cells.

The method of the invention involves analysing single cell omics data from a spiked population of cells, wherein presence of the tagged single cell within a cluster of cells in the population identifies said cluster as associated with a particular phenotype. Conversely, the method of the invention also enables the user to infer that any number of clusters not associated with the phenotypically characterised cell do not associate with that phenotype, and may, in some instances, have the opposing phenotype. Thus, the present invention also provides a method for identifying a cluster of cells having an opposing phenotype to that of a phenotypically characterised tagged single cell. Particularly, in this aspect, the method involves phenotypically characterising a single cell, tagging and spiking into a heterogeneous cell population, analysing the single cell omics data from the spiked population of cells, and identifying one or more clusters which do not associate with the phenotypically characterised cell and thereby inferring that the cells of the one or more clusters do not have the characterised phenotype. The present method is generally applicable to all omics disciplines which may be successfully applied to single cells currently or at a future date. Depending on the single cell omics discipline used, the analysis methods and subsequent approach to clustering will vary, however analysis methods and clustering analyses for all known single cell omics disciplines are well known in the art. For example, if single cell transcriptomics are to be performed on the spiked population of cells, single cells may be subjected to transcriptome analysis by preparing barcoded single cell sequencing libraries and subjecting to high throughput sequencing; demultiplexing the sequencing reads, for example by using the Seurat pipeline; mapping the reads to a transcriptome containing the whole transcriptome library of the relevant organism plus the sequences of any particular spike tags (e.g. *lacZ*), counting reads; filtering of single cell data represented by fewer than 3 unique barcode sequences; removing poor sequencing data; maintaining single cell data having 200-7000 genes and containing <10% mitochondrial genes; and normalising the data.

The analysis of single cell omics data from the spiked population of cells in the methods described herein involves the identification of a cluster of single cells. The presence of the tagged single cell exclusively within a particular cluster in the heterogeneous population of cells identifies said cluster as associated with the phenotype which characterises the single cell. As discussed above, a cluster of cells refers to two or more cells within a larger population of cells which associate with one another on the basis of similarity with respect to at least one variable. An association which leads to the formation of a cluster may also be described as covariance in the two or more cells of a particular variable. The variable is determined via the analysis of single cell data omics data. Clustering methods are well known in the art and any suitable clustering method may be applied to the method of the invention. The method of clustering may depend on the type of omics data analysed in the method described herein. For example, K-means clustering, Hierarchical clustering, or dimensionality reduction principal component analysis (PCA) may be used to cluster the single cell omics data. In methods of the invention whereby single cell transcriptomics are to be performed on the spiked population of cells, clustering may be performed on the basis of variable gene expression, preferably by PCA, and dimensionality is preferably reduced. Identification of the phenotypically characterised cell in a cluster of cells via the identification of its tag consequently associates phenotype with said cluster.

The method of the invention may further comprise spiking the heterogeneous population of cells with a first and second single cell each with distinct tags, wherein the first and second single cell have been subject to the same phenotypic characterisation assay to determine a particular phenotype, and wherein the first and second cell are characterised as having different phenotypes based on their behavior in the phenotypic characterization assay, and wherein the method involves identification of distinct clusters associated with each of the different phenotypes. This aspect of the invention could be applied to any phenotype. The most preferred phenotype for association with the method described herein is metastatic potential. Thus, the method of the invention may further comprise spiking the heterogeneous population of cells with a first and second single cell each with distinct tags, wherein the first and second single cell have been subject to the same phenotypic characterisation assay to determine metastatic potential, and wherein the first cell is characterised as metastatic and the second cell is characterised as non-metastatic, and wherein the method involves identification of a metastatic and non-metastatic cluster of cells. Preferably, the non-metastatic cluster identified by the second cell has altered VSIG1 expression relative to the metastatic cluster identified by the first cell . More preferably, the non-metastatic cluster identified by the second cell has increased VSIG1 expression relative to the metastatic cluster identified by the first cell.

The method of the invention may further comprise spiking the heterogeneous population of cells with a first and second single cell each with distinct tags, wherein the first and second single cell have been subject to a different phenotypic characterisation assay to determine distinct phenotypes, and wherein the first and second cell are characterised as having distinct phenotypes based on their behavior in the different phenotypic characterisation assays, and wherein the method involves identification of distinct clusters of cells associated with each of the distinct phenotypes.

Also provided by the present invention is a method comprising carrying out the method for identifying a cluster of cells associated with a phenotype of the invention, and further comprising merging single cell omics data from the spiked heterogeneous population of cells with single cell omics data from a tagged reference phenotypically characterised single cell, analyzing the merged data, and wherein the presence of the tagged reference single cell within a cluster of cells in the heterogeneous population identifies said cluster as associated with said phenotype. The methods of clustering and analyzing the single cell omics data; the applicable omics disciplines; the type of heterogeneous population of cells; and the applicable phenotypes as discussed with respect to other methods of the invention are likewise applicable to this aspect of the invention. A single cell may be tagged and phenotypically characterised as described herein and subjected to any suitable method of omics discipline described herein in order to generate a "reference" omics data set that is known to be associated with a particular phenotype. Provided that the omics discipline which the heterogeneous population of cells is subjected to is the same as that of any given reference dataset, the reference dataset may be merged with the omics data of the heterogeneous population of cells and used to identify a cluster of cells as being associated with the phenotype of the phenotypically characterised reference. An example of such a method is provided in Example 3.

In any of the methods described herein, there is no upper limit to the number of phenotypically characterised single cells which may be simultaneously spiked into the heterogeneous population of cells. Accordingly, the method may involve the simultaneous association of two, three, four, five or more phenotypes with clusters of cells in a heterogeneous cell population.

### Methods for preventing cancer metastasis

The invention provides a method for preventing cancer metastasis in an individual, wherein the method comprises administering to the individual an agent for inducing VSIG1. The invention also provides an agent for inducing VSIG1 for use in method for preventing cancer metastasis in an individual, wherein the method comprises administering the agent to the individual.

The inventors have shown the VSIG1 expression associates with non-metastatic clusters of cancer cells, and that re-expression of VSIG1 in cancer cells with a metastatic phenotype, or cells interacting with cells with a metastatic phenotype, leads to a prevention of cancer metastasis. Thus, VSIG1 induction in a population prevents metastasis.

Any suitable agent for inducing VSIG1 in a cancer may prevent metastasis of said cancer. The agent may be any agent which is capable of inducing the expression of endogenous VSIG1 in cells within the cancer. The agent may be any mimetic of VSIG1. The agent may be any agent which is capable of inducing the downstream effectors of VSIG1.

The agent may be a polynucleotide which encodes VSIG1, thereby providing increased copies of the VSIG1 gene in the cancer and thereby leading to induction by increased expression of VSIG1. In particularly, the polynucleotide may be defined by SEQ ID NO 115 or SEQ ID NO 116. The polynucleotide may be provided in isolated or substantially isolated form. By substantially isolated, it is meant that there may be substantial, but not total, isolation of the nucleic acid from any surrounding medium. The polynucleotide may be mixed with carriers or diluents which will not interfere with their intended use and still be regarded as substantially isolated. A polynucleotide which "encodes" VSIG1 is a nucleic acid molecule which is transcribed (in the case of DNA) and translated (in the case of mRNA) into VSIG1 in vivo when placed under the control of appropriate regulatory sequences, for example in an expression vector. The boundaries of the coding sequence are determined by a start codon at the 5' (amino) terminus and a translation stop codon at the 3' (carboxy) terminus. For the purposes of the invention, such nucleic acid sequences can include, but are not limited to, cDNA from viral, prokaryotic or eukaryotic mRNA, genomic sequences from viral or prokaryotic DNA or RNA, and even synthetic DNA sequences. A transcription termination sequence may be located 3' to the coding sequence. Polynucleotides can be synthesised according to methods well known in the art, as described by way of example in Sambrook et al (1989, Molecular Cloning - a laboratory manual; Cold Spring Harbor Press). The nucleic acid molecules of the present invention may be provided in the form of an expression cassette which includes control sequences operably linked to the inserted sequence, thus allowing for expression of the polypeptide of the invention in vivo. These expression cassettes, in turn, are typically provided within vectors (e.g., plasmids or recombinant viral vectors). Such an expression cassette may be administered directly to a host subject. Alternatively, a vector comprising a polynucleotide of the invention may be administered to a host subject. Preferably the polynucleotide is prepared and/or administered using a genetic vector. A suitable vector may be any vector which is capable of carrying a sufficient amount of genetic information, and allowing expression of a polypeptide of the invention.

The agent may be administered as comprised within a composition such also containing pharmaceutically acceptable carriers; a vesicle, or a viral delivery vehicle such as a lentivirus.

The administration of the agent is via any suitable means, although most preferably the agent is administered via an intratumoural route.

The cancer may be any cancer affecting a human. The cancer may particularly be a cancer known to be metastatic. The cancer may particularly be a cancer with low or no expression of VSIG1. The cancer is most preferably colon cancer.

The metastasis may be a primary or secondary metastasis.

### Methods for determining the efficacy of a medicament

The method of the invention allows for the assessment of the efficacy of medicaments for the treatment of a subject. In particularly, it is known in the art that diseased tissue, particularly cancer tissue, comprises a heterogeneous population of cells which may respond differently to a medicament. In the case of cancer, it is well known that sub-populations of cells *(i.e.* clusters) in a cancer lesion may be resistant to cancer therapies and/or may evoke re-establishment of a cancer lesion after an initial treatment regimen because of the inefficacy of a particularly medicament. It would therefore be advantageous, not only in cancer but also in other disease contexts, to ascertain the efficacy of medicaments to sub-populations of cells within a diseased tissue in a patient-specific manner. The present invention allows personalised treatment strategies to be established by ascertaining the efficacy of medicaments relative to each sub-population within a diseased tissue.

The invention thus provides a method for determining the efficacy of a medicament, wherein method comprises the identification of one or more clusters of cells in accordance with the method of the invention described herein, and wherein the method further comprises spiking and clustering a heterogeneous population of cells which has been subject to treatment with a medicament and assessment of whether the proportion of a cluster of cells associated with a particular phenotype changes after treatment with said medicament. For example, the phenotypic characterisation of the single cell in the method described herein may involve assessment of the efficacy of a particular medicament with respect to a phenotypic characteristic of said single cell e.g. proliferative capacity, within a heterogeneous cell population in a tumour. Furthermore, the same phenotypic characterisation could be performed on one or more further single cells to determine the efficacy of the medicament with respect to the proliferative capacity of one or more further clusters within the heterogeneous population. Consequently, it can be determined which clusters of cells within a the tumour may have a reduced or enhanced proliferative capacity following treatment with the medicament.

The methods of clustering and analyzing the single cell omics data; the applicable omics disciplines; the type of heterogeneous population of cells; and the applicable phenotypes as discussed with respect to other methods of the invention are likewise applicable to this aspect of the invention. Most preferably, the method is for determining the efficacy of a cancer medicament, and most preferably the method is for determining the efficacy of a cancer medicament in the prevention of cancer metastasis.

### A spiked heterogeneous population of cells

The invention also provides a heterogeneous population of cells spiked with a phenotypically characterised and tagged single cell. The spiked heterogeneous population of cells may be used to identify a cluster of cells within the population that associate with a particular phenotype.

The methods of spiking of a heterogeneous population of cells; the methods of phenotypic characterisation; and the methods of tagging are likewise applicable to this aspect of the invention.

Preferably, any of the spiking methods and proportions described in the context of the method of the invention may characterise the heterogeneous cell population. The proportion of spiked cells should be sufficient to allow identification of the spiked cells in the omics data with sufficient resolution such that the spiked single cell omics data can be applied to a clustering analysis and potentially cluster with two or more cells in the heterogeneous population of cells. Preferably, relative to the overall number of cells to be subject to a single cell omics analysis, the spiked cell may account for at least about 0.1% to 50% of the cells, or about 0.5% to 30% of the cells, or about 5% to 10% of the cells.

The tagged single cell is preferably autologous to the heterogeneous population of cells, and more preferably the heterogeneous population of cells is derived from tumour biopsy.

Preferably, the phenotypically characterised and tagged single cell is tagged using any tag that allows identification of the tagged cells using an omics assay, particularly wherein the tag is any of those described in the context of the method of the invention described herein. The tag may be a heterologous transgene, preferably wherein the single cell may be transduced with a nucleic acid sequence which comprises a heterologous gene that particularly allows identification in genomic, proteomic and transcriptomic assays. The heterologous gene may, for example, encode one or more of *GFP, puromycin* and *lacZ.*

Preferably, the phenotype may be any phenotype, particularly those described in the context of the method described herein. Most preferably the phenotype is metastatic potential.

The present invention is further illustrated by the following examples that, however, are not to be construed as limiting the scope of protection. The features disclosed in the foregoing description and in the following examples may, both separately and in any combination thereof, be material for realizing the invention in diverse forms thereof.

### Example 1

### Materials and methods

### Primary cell cultures and cell lines

CC14 (from a left colon TNM4 adenocarcinoma from a 73-y.o. female patient) and CC36 (from a left colon *KRAS*^{G13D}, *TP53*^{P72R}, *PIK3CA*^{H1047R} TNM3 adenocarcinoma from a 57-y.o. female patient) heterogeneous primary colon cancer cultures were derived from the large bowels of 74- and a 57-year old female patients, respectively (Varnat et al., 2009). Attached early (2-3) passage cultures were maintained in DMEM/F12 supplemented with 5%fetal bovine serum and penicillin-streptomycin at 37C and 5%CO2. DLD1 cells were obtained from ATCC and cultured in RPMI supplemented with 10% fetal bovine serum and 1x penicillin-streptomycin at 37C and 5%CO2.

### Single-cell cloning

Single cell clones were prepared from the patient-derived heterogeneous colon cancer cultures. CC14 cloning was performed manually, plated in 96-well plate and single cell founding status and division dynamics were monitored visually. After 2 days (d) in culture 65% of the seeded wells had doublets or triplets, after 7d 44% of wells had small colonies and after 18d 23% had large viable colonies ready to be expand. Individual clones displayed similar morphologies with varying compaction of the formed islands. CC36 primary culture was single-cell flow sorted using a FACS BDAria. Single cells were automatically plated in 96-well plates and single cell status verified visually. After 24h 30% of the wells had doublets/triplets, after 4d 20% had small aggregates, after 7d 15% had medium-sized colonies, and after 14d 11% had large colonies. Clones were collected and expanded at day 15. Clones were cultured in DMEM/F12, supplemented with 5% FBS and 1% penicillin-streptomycin.

### In vivo animal studies

All in vivo experiments were performed following approved protocols and animal care standards from the Swiss Office Cantonal de Vétérinaire de Genève. 5x10⁵ cells were resuspended in 100µl of HBSS and injected subcutaneously into the flank of 8-week old NUDE (NMRI-foxn1 nu/nu) female mice (Janvier Laboratories). Tumor growth was monitored every other day with a caliper. Mice were taken before tumors reached the legal size limit (100 mm3 volumes) and before any overt discomfort. Tumors and lungs were collected for further analysis. Lungs were fixed in fresh 4% PFA for 16h and stained with X-Gal for 8h. X-Gal positive cells and colonies were counted on both sides of each lobe under microscope. For co-culture xenografts, tumors were collected and dissociated as described in the art. The tumor cell suspension was treated with Mouse Cell Depletion kit (Miltenyi Biotec, cat no. 130-104-694), for the enrichment of the human cell fraction. Such suspensions were seeded and allowed to attached to the plate for 16h. Cells were then fixed and stained with X-Gal for counting. The ratio of *lacZ*⁺/white cell was calculated and the metastatic index (the number of *lacZ*⁺ lung metastases over tumor volume in mm³) was normalized by this ratio.

### Plasmids, lentiviruses

Cells were transduced with *lacZ*-expressing lentivector (Varnat et al., 2009) and/or Alkaline Phosphatase-expressing retrovector (AP) (CMV-PLAP) (Addgene #15164). Two different shRNA lentivectors targeting *VSIG1* transcripts were used: (5'-3') shVSIG1(26) (TRCN0000432026:CCGGACGTGACTGTTGGATCTAATGCTCGAGCATTAGATCCAA CAGTCACGTTTTTTTG); shVSIG1(95) (TRCN0000158295:CCGGCCAACGATCCAGGTAATGCATCTCGAGATGCATTACCT GGATCGTTGGTTTTTTG). shCAV1 (TRCN0000008002: CCGGGACGTGGTCAAGATTGACTTTCTCGAGAAAGTCAATCTTGACCACGTCTTT TT). pLKO.1-puromycin was used as control vector.

Two vectors were used for the ectopic expression of VSIG1: *VSIG1*variant1-*GFP* (Origene RC230056L2) and *VSIG1*variant2-*GFP* (Origene RC207391L2). The 8XGTIIC-luciferase synthetic TEAD luciferase reporter was used to measure endogenous TEAD-cofactor activity (Addgene 34615). Constitutively active YAP construct used harbored the S127A mutation (Addgene 27370).
For the FUCCI reporter cell cycle system the inventors used the combined expression lentivector pBOB-EF1-FastFUCCI-Puro (Addgene#86849).

### CRISPR-CAS9 induced VSIG1 KO

CRISPR guide RNAs (gRNAs) were designed using the CRISPOR (http://crispor.tefor.net/) program, and the closest gRNA from the initiator ATG having the highest score was chosen: CTCAACTAACCTCCACACAA. The final gRNA was obtained by Fusion/annealing the specific crRNA (Thermofisher A35512) plus the common tracrRNA (Thermofisher A35506) using the manufacturer's instructions for PCR reaction and program.
CAS9+gRNA ribo-nucleo-protein complexes were delivered to cells using the Neon Transfection System (Thermofisher NEON 10ul Transfection Kit MPK1025) into 10⁵ cells. The RNP complex was formed at a 1/1 molecular ratio between the CAS9 protein (truecut CAS9 protein, Thermofisher A36498) and the annealed complex cr/tracrRNA in buffer R. The electric parameters were: condition #16, 1400V, 20mS 2 repetitions. The efficiency of cleavage was determined with the Gene art cleavage detection Kit (Thermofisher, A24372), and validated by PCR to generate a 500bp fragment centered around the cutting site. Electrophoretic gel band intensities were quantified with ImageJ. Primers were 5'->3': VSIG KO V-P F1: ATCAAGGAAACACGCCCCAT (218 bp upstream of the VSIG1 ATG) and VSIG KO V-P R1: TGAGAAAATTTTCATTCAGAAGCTCCA (305 bp downstream of the VSIG1 ATG).

Single-cell derived clones were grown after FACSeeding individual cells in 96-well plates and their mutation status determined by Sanger sequencing the targeted region using the primers described above. As VSIG1 is located in the X chromosome, the mutational status of the expressed allele was determined by RT-PCR followed by Sanger sequencing. Confirmation of the KO status was performed by immunohistochemistry using specific anti-VSIG1 antibodies.

### RNA isolation, RT-qPCR, DNA extraction, PCR and Sanger sequencing

RNA extraction followed the procedures in Varnat et al. (2009). cDNA synthesis was performed using Superscript II kit (Invitrogen) and the real-time quantitative PCR (RT-qPCR) using SYBR Green PCR Master Mix (BioRad). The Primers are listed in the Key Resources Table below. DNA extraction was performed with DNeasy Blood & Tissue Kit (Qiagen Cat No.69504) following manufacturer's instructions. Amplification by PCR products from selected genomic regions was performed with Go Taq G2 DNA polymerase (Promega Cat No. M7841) using primers listed in Key Resources Table. Sanger sequencing of purified PCR products was carried out by Fasteris SA and the sequences analyzed with the ApE program.

### Cell co-cultures, transfilter assays, treatments, clonogenic assays and FACS-cell cycle analyses

Clones previously infected with lacZ or GFP lentiviruses, or AP retroviruses, were mixed at 1:1 ratio with different or same untraced clone cells as described in the text. Cell mixtures were seeded and co-cultured for 3d before testing. For transfilter assays, 1x10⁵ cells in 0.5%FBS medium were seeded in the upper chamber of the transfilter insert and 10% FBS medium was added to the bottom of the well. Cell migration was assessed 16 hours (CC36 and derived clones) or 72 hours (CC14 and derived clones) after seeding. Traced cells having crossed the filter were then counted after 4% PFA fixation of the filter, washing the top with a cotton swab and staining with X-Gal or AP (NBT, BCIP) reagents. Transfilter assays were also performed on clones pre-treated 2 days with conditioned media prepared as described in the art derived from different clones or from parental cultures. CL1 and CL29 were treated respectively with BMP4 (B853-40N Signal Chem) and EREG (SRP3033-Sigma Aldrich) ligand (100ng/ml) for 48h. For clonogenic spheroid-forming assays, one single cell was seeded per well manually in non-treated 96-well plates (CytoOne n°CC7672-7596) and confirmed visually. DMEM/F12 medium supplemented with B27, penicillin-streptomycin and EGF 10ng/ml was used under standard conditions. The percentage of spheres per plate was calculated after 14d. CD133 sorting was performed as described in the art. For cell cycle analyses using flow cytometry, single-cell suspensions were fixed with 70% ethanol at -20°C for at least 2h, rinsed twice with PBS and incubated with 0. 1mg/ml of RNAse A at 37°C for 30'. Propidium iodide (Sigma P4170-10mg) was added at a final concentration of 20 µg/ml before analysis on a flow cytometer (BD Accuri C6). Vemurafenib (Sellenchem) was used at 7µM for 48h in 0.1% DMSO. Control for Vemurafenib treatment was 0.1% DMSO alone.

### VSIG1 immunostaining, FACS analysis and sorting

For immunostaining, 1x10⁵ cells were seeded on the top of coverslips in 24-well pates and after 2d they were fixed for 10' using fresh 4% PFA. Two PBS washes were performed and cells were permeabilized using PBS containing 0.2% TritonX-100 for 30' at RT when required. After two PBS washes, cells were incubated in blocking solution (PBS containing 10%FBS and 1%BSA) for 1h at RT. Primary antibodies were diluted in blocking solution and incubated overnight at 4°C. Secondary antibody (Jackson Immuno-Research, Life Technologies) were incubated 45' at RT. Four final washes were performed for 10' each with PBS containing 0.1%Tween20 and cells counterstained with Hoechst dye. Coverslips were mounted with Immu-Mount (Thermo 9990402) on glass slides. For non-permeabilized cells, washes were performed with PBSwithout Tween20. Antibodies and concentrations used are listed in Key Resources Table .

For FACS, cells were dissociated using Trypsin and resuspended in PBS with 1%BSA at a density of 1.10⁷cells/ml. Mouse primary antibody mVSIG1 (R&D MAB4818) was added at 5µg/ml final concentration for 30' at RT with agitation. After two washes with PBS cells were again resuspended at 1.10⁷ cells/ml in PBS with 1% BSA. Cells were then incubated with secondary antibodies at 2µg/ml for 30' with agitation. Two final PBS washes were performed and the cells FACS analyzed or sorted. Multiple flow-cytometer were used for sorting (Beckman Coulter MoFlo Astrios; Beckman Coulter MoFlo Astrios; Biorad S3). For a higher reproducibility, all replicates of an experiment were FACS-sorted on the same machine. For the isolation of 4 populations expressing different level of VSIG1, the gating strategy was standardized among replicates using Rainbow beads calibration 8peaks (Biolegend #422903).

### Transfections and Luciferase reporter assays

Cells were transfected with Lipofectamime 2000 (5ul/ug of DNA, Invitrogen) in Opti-MEM medium (Gibco). For luciferase assays 5x10⁴ cells were plated in each well of 24-well plates and transfected with 500ng of 8XGTIIC binding site Luciferase reporter ,100ng of Renilla control plasmid DNA and 250ng of S127AYAP with Lipofectamine 2000 (Invitrogen). After 24h activity was assessed following the manufacturer's protocol as described in the art. For co-cultures, transfected cells were harvest and mixed the day after transfection with un-transfected clones and co-cultured for 3d, at this time cells were lysed to perform the luciferase assay. Relative luciferase activity is calculated as the ratio of luciferase and renilla activity.

### Exome sequencing and population transcriptomes

All sequencing was performed at the University of Geneva/iGE3 genomic platform using standard techniques (https://ige3.genomics.unige). The Agilent SureSelect Human All Exon V4 capture was used for exome capture. Paired end sequencing was done on Illumina platform producing 100bp reads. Variants were called following GATK best practices (GATK v3.6). Briefly, after ascertaining good quality reads using FastQC, the reads were mapped to the UCSC hg19 reference genome using BWA mem (v0.7.15-r1140). PCR duplicates were then marked using Picard Tools (v2.5.0) MarkDuplicates, and Indel realignment done followed by Picard Tools FixMateInformation. Base quality scores were properly recalibrated using GATK BaseRecalibrator and BQSR. GATK haplotypecaller was then used to produce genotype GVCFs for individual samples. Finally, joint genotyping on gVCF files was done using GATK GenotypeGVCFs producing the variant VCF-file. The variants were then separated as SNPs and INDELs and GATK hard filters were applied with the parameters: --filterExpression "QD < 2.0 ∥ FS > 60.0 ∥ MQ < 40.0 ∥ MQRankSum < - 12.5 ∥ ReadPosRankSum < -8.0 for SNPs and --filterExpression "QD < 2.0 ∥ FS > 200.0 ∥ ReadPosRankSum < -20.0" for INDELs. The latter were not further analyzed. Variants were annotated using ANNOVAR. SNPs in *AXL* (ch19:41754704), *GDF11* (ch12:56142589), ROR2 (ch9:94486590), *NEO1* (ch15:73409008), *DNAH5* (ch5:13751222) and *CH9* (ch16:53190863) were used to distinguish clones. The CC36 transcriptome is available in the art.

For population transcriptomes (RNAseq) ribosomal-RNA depleted libraries from 3 independent experiments were sequenced on the Illumina Hiseq 4000 platform as single-end 50bp reads. The reads were mapped on human genome hg38 using STAR (v2.5.2b) and differential expression was analyzed using DESeq2 (v1.20.0). Genes represented by less than 3 reads per million were not considered further. FDR is noted in the text. Enrichment analyses were performed with STRING only considering experiments and database settings at 0.4 confidence, or with Enrichr. All population-sequencing assays were performed at the University of Geneva/iGE3 genomic platform using standard techniques (https://ige3.genomics.unige.ch/). STEM analyses were performed with standard parameters.

### Single cell RNA sequencing

The aim was to capture 1500-2000 cells per sample to reach 99% probability of seeing at least 50 cells per cluster assuming 2-4 cell types or states with a low-end frequency of 5% (https://satijalab.org/howmanycells). Cells were treated following the manufacturer's protocols using 10xGenomic ChromiumTM Single Cell 3' Library and Gel Bead Kit v2 ( 120267) and ChromiumTM Single Cell A Chip Kit (1000009). Given the maximal efficiency of cell capture of 65%, and the subsequent known attrition during quality controls, mapping, elimination of doublets, etc, 3500-4000 CC14 cells were loaded with Gem-beads into the Chromium Cell A Chip after mixing in the spikes, when appropriate, to obtain a final distribution of 85%CC14, 5%C11, 5%B2 and 5%E3. Clones were grown under the same conditions as parental cells and mixed in just before processing. Single cell libraries were sequenced (HiSeq 4000 platform, Ilumina) as 100-bp paired-end reads in multiplex.

Analyses were performed in house using the Cell Ranger pipeline (version 2.1.1) to demultiplex the single cell barcodes followed by standard analyses using the Seurat pipeline: Reads were mapped to a transcriptome containing hg19 plus the sequences of the spike tags (*GFP, puromycin* and *lacZ*) with 10xCell Ranger and unique molecular identifiers (UMI) counted. Count data were imported into the Seurat single cell analysis software. To minimize the false positives and correctly identify the spiked cells, it was required that these cells were supported by at least 3 UMI counts of the spike tags. Following the Seurat standard pipeline (https://satijalab.org/seurat/v2.4/pbmc3k_tutorial.html), quality control (QC) was performed to remove outlier cells: only those cells having 200-7000 genes and containing <10% of mitochondrial genes were kept. The inventors obtained in total 1602 QC-approved cells (including 91 C11, 88 B2 and 86 E3 cells). The data was then normalized using LogNormalize method and inherent variation caused by mitochondrial gene expression and the number of UMIs per cell was regressed out. PCA was performed on variable genes and dimensionality was reduced. tSNE was used for visualization of results. The Scanorama algorithm was used to merge scRNAseq data sets obtained independently (Hie et al., 2019) since analyzing data directly imported into Seurat from two different sets was not efficient. The robustness of the main features discussed for the Scanorama map in the text was confirmed altering parameters, which yielded varying maps that nevertheless conserved the features discussed.

### Quantification and statistical analysis

In all cases, different biological repeats were performed with low passage cells independently transduced. All p values were obtained using unpaired, two-tailed, T-tests, p<0.05 was considered statistically significant. All RT-qPCR analyses included technical triplicates. Except if stated differently on a particular section, all charts are representing the mean and the dispersion represent the SEM. For in vivo experiments, "n" represent the number of mice per conditions. Statistical details for exome sequencing, RNAseq and Single Cell sequencing can be found in each specific method paragraph.

### Example 2

A problem in the study of cell behaviour, both in the context of healthy tissue physiology and in the context of diseased tissue, is the inability to accurately define groups (*i.e.* "clusters") of cells within a heterogeneous cell population according to their function. The present invention solves this problem by providing a method which surprisingly identifying clusters of cells associated with a particular phenotype. The present inventors also surprisingly demonstrate that VSIG1 induction can be efficacious in the prevention of cancer metastasis.

When utilizing the method of the invention to associate a phenotype to clusters of cells within a heterogeneous tumor, it is preferable for tumor cell populations to be able to grow in culture, be tagged, take in mice and that single-cell clones are stable in their tested phenotypes and transcriptomes. The inventors have previously shown that his can be the case, with 25% overall success rate using colon cancers from the OR. The inventors have previously shown that (e.g. from Varnat et al., 2009), once the cultures grow (and frozen stocks of early passages established) they are stable and maintain their morphology and rate of 'metastases' in vitro over multiple passages, measured as migratory transfilter efficiencies. Similarly, in vivo these cultures display a stable phenotype as measured by their metastatic indexes (the number of distant lung metastases from subcutaneous grafts, over tumor size). Transfilter efficiencies in vitro faithfully track in vivo results, and in the absence of true orthotopic grafts (e.g. without cecal micro-vessel rupture) distant metastases in the lungs from subcutaneous grafts are a clean measure of distant metastatic behavior in vivo.

All samples subjected to single-cell cloning yielded viable clones using standard dilution techniques in 5% FBS media. Clones, were then tested for transfilter behavior. Previous transduction with lacZ-expressing lentivectors allowed the trace even single cells through XGAL staining and thus quantification of migratory and invasive (when using a matrix that cells need to traverse) behaviors.

Selected clones with different migratory abilities can then be used for further testing. The inventors used those from the low-passage, primary colon cancer cell population CC14 (Varnat et al., 2009) with maximal and minimal migratory efficiencies for in vivo grafts. The top migratory clones were highly metastatic whereas the least migratory clones showed very limited metastatic behavior (Fig. 2). These clones retained their stability and phenotypes upon multiple passes and freeze-thawing cycles. Subclones similarly retained the phenotypes of parental clones.

Analyses of the exomes and transcriptomes of most and least metastatic clones form CC14 - E3 and C11, respectively (Fig. 2) - showed that i- both contained all classical mutations for this tumor subtype (e.g. BRAF and SMAD4), ii- the genetic variation in the parental population could be accounted for by the two clones above a background level of 0.5% of all SNPs (Fig. 3). Thus, the difference between the different high- and non-metastatic populations in this high-mutator cancer cell population is unlikely to be genetic (Bernal, Silvano et al., submitted).

Transcriptionally, however, these two clones were very different, highlighting a large cohort of differentially expressed genes: for instance, 411 genes were consistently differentially expressed in E3 vs. C11 and E6 vs. F3 clones (see Fig. 2A) by 2-fold or more. Analyses of the transcriptomes of these clones against that of an intermediate metastatic clone, named B2 (see Fig. 2A), showed that metastatic efficiency is indeed not genetic-driven but is, instead, correlated with step-wise transcriptional changes (Fig. 4).

To map behavior to single cell states the inventors first performed scRNAseq analyses of the CC14 heterogeneous primary culture, which identified two major populations using the CellRanger and Seurat pipeline, beyond any cell cycle differences. Refinement of the clustering depth allowed the identification of two additional clusters or subpopulations, one for each main cluster, with distinct transcriptional signatures (Fig. 5).

The next step was to map cells with characterized metastatic function to single cell clusters; that is, to functionally label the single cell clusters with behaviorally-tested clones. To achieve this, clones were spiked to about 5% each into the parental heterogeneous population to scRNAseq sequence. Each clone was genetically tagged and stably expressed either GFP, lacZ or Puromycin resistance gene in this case. These transgenes were used to individually identify spiked cells.

Mapping tagged clones, which display distinct signatures (Fig. 6), within the heterogeneous population allowed the positioning of the tagged cells in the map using Seurat and a bioinformatics pipeline. Each clone mapped exclusively to a single cluster, with spiked cells scattering within that specific cluster. This indicates that each clone represents the mapped cluster. In this manner the inventors identified distinct metastatic and non-metastatic clusters and established a link between transcriptome and metastatic phenotype (Fig. 7).

Note that the metastatic cluster includes both highly- E3 and medium-metastatic B2 cells and that separating them may require greater depth.

Additional characterization, including bioinformatic enrichment analyses, as well as testing the expression of specific cluster markers in migrating cells from transfilter assays (Fig. 8), has allowed the inventors to describe the large cluster as the non-metastatic cluster (NONMETCL) and its subpopulation (NONMETSP) as goblet-like secretory cells; and the second largest cluster as the pro-metastatic cluster (METCL), with its subpopulation (METSP) composed with pro-metastatic cells likely engaged in migratory behavior (Fig. 7).

### Example 3

### Results

### Pro-metastatic heterogeneity of primary tumor single-cell clones

Single cell clones were made from the heterogeneous primary CC14 colon cancer cell population (Varnat et al., 2009) and challenged for transfilter migratory behavior in vitro (Fig.9A). Low/non- (C11) and high-migratory (E3) clones showed a stable >10-fold difference (Figs.9A, 16A), although they displayed similar morphology, and percentages of CD133⁺ and of spheroid-forming cells (Figs.16B,C). Subcutaneous xenografts of *lacZ*⁺ E3 and C11 yielded similar tumor volumes. However, E3*^{lacZ}* produced 10-fold more lung metastases than C11*^{lacZ}* (Figs.9B,C). Given these congruent results (see also Repesh et al., 1989; Mishra et al., 2019) the inventors hereafter describe such phenotypes as metastatic behavior. Analyses of additional high- (E6*^{lacZ}*), medium- (B2*^{lacZ}*) and low/non- (F3*^{lacZ}*) metastatic clones reproduced these results (Figs.1A,B, 16A), and single-cell subcloning of C11 confirmed the stability of the phenotype (Fig. 16D).

Exome sequencing of E3 and C11 revealed shared early and late driver mutations (e.g. *APC* and *SMAD4*) suggesting their late split (Gerstung et al., 2020). Only 3.5% of CC14-specific SNPs different from the reference genome were differentially segregated in a heterozygous manner in E3 vs. C11 (Fig.9D,16E). As no alleles were detected in CC14 (with frequencies >0.5%) absent from E3 or C11, and these clones did not exhibit significant levels of SNPs not found in the parental mixture, they represent the parental population's allelic variation. Whereas two coding and four STOP SNPs distributed in E3 vs. C11 separated high-/medium- (E3,E6,B2) vs. low-metastatic (C11,F3) clones (Figs.9E,16F, not shown), they cannot account for the intermediate degree of metastatic behavior (e.g. B2 vs. E3,E6), suggesting a separation of genetic determinants and metastatic behavior.

Enrichment and predicted-protein interaction software analyses of clone transcriptomic data highlighted changes in extracellular remodeling, inflammatory responses, membrane bound organelles and signaling components including cytokines in E3/E6 high-vs. C11/F3 low/non-metastatic clones. Upregulated genes included those involved in signaling, cell adhesion, vesicular trafficking (e.g. *CAV1*) and transcription (with some families were clustered: e.g. *MAGEAs* in chX and *ZNFs* in ch19), whereas downregulated genes highlighted the Golgi (Fig.9F,16G,H). Moreover, the transcriptome of B2 cells allowed the classification of gene expression changes in relation to graded metastatic behavior (Fig.9G).

### Spiked-scRNAseq links phenotypes to single-cell clusters

ScRNAseq analyses of CC14 yielded two main clusters (CL) with cell cycle phases (Figs. 16I) and two small sides populations (SP) in G1 (Fig.10A). To assign metastatic phenotypes to single-cell clusters, the inventors included approximately 100 cells of each of the E3*^{GFP}*, C11*^{puromycin}* and B2*^{lacZ}* clones as tracing spikes into the bulk CC14 population. After sequencing, spiked cells identified by their stable genetic lentiviral tags (*GFP, puromycin* or *lacZ*) were mapped to different clusters (Fig. 10B). High- E3*^{GFP}* and medium-metastatic B2*^{lacZ}* cells homed to the smaller cluster, hereafter metastatic cluster (METCL), and low/non-metastatic C11*^{puromycin}* cells homed to the larger cluster, hereafter NONMETCL. The relative sizes of these clusters approximated the distribution of C11 and E3-specific SNPs.

The side populations display differential expression of secretory and trafficking components

The SP of the NONMETCL, hereafter NONMETSP, was characterized by the high level and frequency of cells expressing *SPINK4, TFF3* and other genes (Figs.10C-E,17) that identified it as comprising Goblet-like secretory cells (Dalerba et al., 2011). The METSP, in contrast, showed a high enrichment for XBP1-regulated genes over the NONMETSP (Figs. 10D,17,18A), suggesting ER stress responses. It also contained a predicted network of endoplasmic reticulum (ER)-Golgi protein trafficking including *TMED9* (Figs. 10,17), which encodes a protein secretion cargo selector implicated in pro-metastatic states (Mishra et al., 2019). Given that a number of secretory markers were detected in both SPs (Fig.18C), the expression of a 486-strong secretory cohort (Feizi et al. 2017) was analyzed, revealing marked differences between the SPs (Fig. 18D,E) and suggesting the secretory but non-Goblet-like nature of METSP cells.

### Pro-metastatic heterogeneity in another primary colon cancer cell population

scRNAseq mapping of CC36, a *KRAS*^{G13D} colon cancer primary culture (Varnat et al., 2009), revealed two clusters using PCA dimensions 3-10 including the prominent inverse expression of *EREG* and *BMP4* (Figs. 10F,19A-C. Single-cell clones from the parental population were made and tested in transfilter assays, showing two main groups with low vs. high metastatic behaviors (Fig.10G). Highly-metastatic clone 29 (CL29*^{laCZ}*) and low-metastatic clone 1 (CL1*^{lacZ}*) reproduced their different behavior in vivo xenografts scoring for distant metastases (Fig. 10H). To match behavior to transcriptomic profiles, instead of spiking here the inventors directly compared bulk clone transcriptomes and scRNAseq data using a cut off of detected expression in >10% of cells analyzed. Genes mapped to the *EREG* cluster were upregulated in the transcriptome of low-metastatic CL1 vs. highly-metastatic CL29, and conversely, genes mapped to the *BMP4* cluster were upregulated in CL29 vs. CL1 (Figs.10F,I,19C. Critically there was no cross-matching of genes (Fig. 10I). The partial overlap between the sets likely derives from the low depth of the scRNAseq data. The differential expression of genes marking the *EREG*^{high} (LOWMETCL) vs. *BMP4*^{high} (HIGHMETCL) clusters genes was reproduced in a second pair of low-metastatic (CL28) and high-metastatic (CL6) CC36 clones (Fig.19D). Testing the participation of these markers in metastatic behavior revealed that BMP4 but not EREG promoted cell migration (Fig. 19E,F), consistent with previous data.

Analyses of predicted protein interactions using CL29 vs. CL1 transcriptomes highlighted upregulated transport/localization/secretion, vesicle-mediated transport, lipid metabolism, vs. downregulated cell cycle parameters, response to stress and microtubule/cytoskeleton activity (Fig. 19G). It is paradoxical that CC14 high-metastatic cells display high levels of specific vesicle trafficking markers whereas these are notable in the lower metastatic cluster of CC36. Whereas CAV1, for instance, can have context-dependent functions, it is possible that instead of specific genes, it is the processes they participate, such as secretion, vesicle trafficking and signaling, that mark pro-metastatic cells.

### Gene expression analyses of migratory cells identifies them as METCL/SP cells

To test the match between highly-metastatic gene signatures with migratory behavior the inventors used CC14 cells that had actively migrated in transfilter assays (Fig.11A,B). Most MET genes tested showed higher or similar expression ratios in the migratory over the control non-migrated population. In contrast, all CC14 NONMET cluster markers were strongly underrepresented (Fig.11B). Moreover, the CC36 HIGHMET markers *BMP4* and *MSX1* were highly expressed in migrating CC14 cells (Fig. 11B). Of the most highly expressed genes in migrating cells *CAV1* was also strongly differentially expressed in highly-vs. low-metastatic cells (Fig.9F). The inventors thus tested the role of this gene as a representative of the vesicular trafficking gene cohort: *CAV1* knock-down (kd) (using a validated shRNA) in E3 cells resulted in a reduction in transfilter migration by nearly 80% (Fig. 11C), paralleling its involvement in other cases .

### Spiked-scRNAseq reveals differential drug action on distinct clusters

The inventors next asked spiked-scRNAseq could be used to ascertain differential drug effects on cell subpopulations. As a proof of principle the inventors chose the BRAF inhibitor Vemurafenib as all CC14 cells carry the BRAF^{V600E} mutation. Strikingly, the majority of METSP markers tested were highly upregulated after 48h treatment (Fig.11D), raising the possibility that whereas the inventors found that BRAF inhibition halves cell numbers (not shown), it could actually promote metastatic behavior in surviving cells, perhaps in line with its very poor efficacy in the clinics for this indication. Consistently, transfilter assays revealed that Vemurafenib-treated CC14 cells were 2-fold more migratory than untreated controls (Fig.11D).

### Non-cell-autonomous interactions modulate pro-metastatic heterogeneity

As the transfilter efficiencies of the heterogenous parental primary populations lie in the middle range of those of the derived clones (Figs.9A, 10G) the inventors tested the possibility that the frequency of pro-metastatic states in a heterogenous cell mixture depends on non-cell-autonomous influences. High-metastatic CC14 E3*^{lacZ}* cells mixed at a 1:1 ratio with unmodified low-metastatic C11 (C11^{white}) cells, yielded 50% less migratory *lacZ*⁺ cells in transfilter assays than the control E3*^{lacZ}*/E3^{white} mix (Fig.11E). Similarly, E3*^{AP}* mixed with F3*^{lacZ}* cells yielded a 50% reduction in migrated AP⁺ cells (Fig.11E), which was recapitulated by the effect of C11^{white} or F3*^{lacZ}* on E6*^{AP}* cells (Fig. 1 IE). Inversely, high-metastatic E3^{white} did not significantly affect the migratory levels of C11*^{lacZ}* or B2*^{AP}* cells (Figs.11E,20A). Conditioned media were ineffective (Fig.20B-E). In vivo, distant metastases from tumors formed from a 1:1 mixture of E3*^{lacZ}* and C11^{white} were 3-times less abundant (after normalization by the ratio of *lacZ*⁺ cells in the tumor) than those derived from tumors from a similar E3*^{lacZ}*/E3^{white} mixture (Fig.11F).

Analyses with CC36 CL1 and CL29 cells did not reveal a significant negative interaction. However, the highly metastatic CL29 cells prompted CL1 cells to become more metastatic, which was recapitulated by its conditioned media (Figs. 11G,20F,G), paralleling previously data.

Thus, beyond previously described positive influences, the present results reveal critical negative cell-cell interactions, which may be mediated by membrane components.

### Identification of VSIG1 expression in low/non-metastatic cells

To identify candidates mediating the negative non-cell-autonomous effect the inventors compared genes differentially expressed (>5-fold) in C11 vs. E3 and in F3 vs. E6 clones, upregulated in METCL vs. NONMETCL, and absent in CC36 where such negative interaction is not detected (Fig. 11G). Four genes satisfied the criteria but only one encoded a transmembrane protein: v-set and immunoglobulin domain-containing protein 1 (VSIG1, Fig.11H,I). Mapping *VSIG1* onto the CC14 scRNAseq plot showed a <10% scattering of positive cells restricted to the NONMETCL (Fig. 11J). The choice of *VSIG1* was reinforced by previous findings with clinical relevance showing that i- VSIG1 is heterogeneously expressed in normal human colon epithelial cells, ii- that it is generally not expressed (with only rare structural alterations) in a majority of advanced tumors although it can be present in few colon cancers, and iii- that there is a positive trend between higher *VSIG1* expression levels and better colon adenocarcinoma outcomes.

### Heterogeneous expression of VSIG1 protein

Immunolabeling of VSIG1 was routinely performed in non-permeabilized cells with a mAb recognizing an extracellular epitope (Fig.11K-N,S5H). Only a percentage of cells in CC14 (5-10%), C11 (40-60%) and F3 (10-15%) clones, but none in E3 or E6, were strongly VSIG1 positive (Fig. 11K-N). Increasing confocal gain under conditions that remain specific (Fig.12A) uncovered 4-fold more weakly-expressing cells in CC14, 2-fold in F3 and 50% more in C11 cells (Fig.11K-M). Very low VSIG1 protein levels were also detected in rare (<0.01%) colon cancer HT29 cells, which display very low*VSIG1* mRNA levels (Figs. 11I,20I). Positive and negative cells were not arranged in any consistent pattern although positive cells tended to be together (e.g. Figs.11K-M). Single cells and doublets could be positive or negative (Fig. 11O). VSIG1 protein was generally distributed in the plasma membrane (Fig. 11O) resembling but not matching apically enriched E-CADHERIN (Fig.11P), and with minor overlap with the tight junction marker ZO1 (Fig. 11Q) or with EZRIN (Fig.20J). Propidium iodide, EdU and FUCCI FACS analyses showed independence of VSIG1 levels from cell cycle phases (Fig.20K-N). C11 subclones recapitulated VSIG1 heterogenous expression (Fig.11R).

### VSIG1 represses metastatic potential and modulates vesicular trafficking and signaling components

ShRNA-mediated kd of *VSIG1* (*shVSIG1⁹⁵* with a kd of 90%; C11*^{shVSIG1}*) yielded only traces of VSIG1 protein under high-gain confocal conditions (Fig. 12A). VSIG1 kd increased the number of migrated *lacZ*⁺ cells in transfilter assays by 5-fold (Fig. 12B). A second shRNA (*shVSIG1²⁶* with a kd of 90%) similarly afforded a 2-fold increase (Fig. 12B). Analysis of distant metastases generated from grafted cells expressing C11*^{shVSIG1}* was problematic due to the decreased long-term kd of *VSIG1* in vivo. To palliate this issue, the inventors generated a biallelic KO of *VSIG1* through CRISPR/CAS9 in C11 cells. Since *VSIG1* is located on chX, only one allele was expressed harboring a 24bp deletion around the initiator ATG (Fig. 12C). The resulting clone, C11*^{VSIG1KO514}* lacked VSIG1 protein (Fig. 12D) and yielded a 2-fold transfilter efficiency increase vs. C11^{control}, phenocopying C11*^{shVSIG1}*. Importantly, C11^{*VSIG1*-*KO514*} produced a 3.3-fold increase in distant metastases from xenografts in vivo as compared with parental C11 cells (Fig.12E), reaching the levels seen with B2 cells.

Enrichment analyses of the transcriptomes of C11*^{shVSIG1}* vs. C11^{control} (Fig. 12F highlighted C11*^{shVSIG1}* downregulated clusters including those pertaining to the cell cycle, chromosome organization, mitochondria function and cholesterol biosynthesis (Fig.20O). In contrast, C11*^{shVSIG1}*cells displayed upregulated gene sets enriched for transcription regulation, cilium and cell projections (Fig.20O). Genes with altered expression included the upregulation of 8 cadherin/protocadherin family members and 4 axonemal dyneins concomitant with the repression of 11 kinesins. Whereas the latter two might suggest effects on the primary cilium, the inventors did not detect acetylated □TUBULIN⁺ or ARL13b⁺ cilia in primary colon cancer cells, their clones or in C11*^{shVSIG1}* cells (not shown). These changes may instead relate to vesicle trafficking and signaling events. Consistently, the inventors detected the upregulated expression of genes involved in these processes including those encoding TAZ, VIMENTIN, FIBRONECTIN and CAVEOLINS (CAV1), and the downregulation of those encoding COPINES, CLAUDINS and GAP junction proteins (Fig. 12).

Comparing enhanced genes in C11*^{shVSIG}* vs. C11^{control} with those in E3 vs. C11 and in E6 vs. F3 clones revealed a limited number of commonly upregulated genes, including those involved in vesicular trafficking and Ca⁺⁺ signaling along a cohort of zinc-finger genes. VSIG1-regulated genes differently modulated in E3 vs. C11 displayed an intermediate pattern in medium-metastatic B2 cells. Whereas a minority of genes of the upregulated cohort were so in all 'more vs. less' metastatic comparisons, the majority could be divided into two groups: one with enhancement in both B2 and E3 as compared with C11; and the other with enhancement in both B2 and C11 as compared with E3. A similar situation was evident for the repressed cohort.

### VSIG1 expression is sufficient to repress metastatic behavior from multiple colon cancer cells

Exogenous expression of VSIG1 in E3 cells, through lentiviral expression of either of two *VSIG1* cDNA forms, produced protein localized to the cell membrane (Fig.12G). The transcriptome of E3*^{VSIG1}* cells yielded few changes although enrichment analyses in E3*^{VSIG1}* vs. E3^{control} revealed alterations in metabolism, cell defense and signaling components (Fig. 12H). Comparison of these changes with those in C11*^{shVSIG1}* vs. control cells highlighted oppositely regulated genes (Fig. 12I): three with enhanced expression in E3 *^{VSIG1}* and four with the reverse pattern that include *VGLL1,* encoding a TEAD interacting TAZ-like cofactor. Given the strong upregulation of *TAZ* (*WWTR1*) by kd of *VSIG1* (Fig.12F), the inventors tested and found enhanced levels of both *VGLL1* and *TAZ* in E3 vs. C11 RNAseq data and by RT-qPCR (Fig.12J).

Expression of VSIG1 in E3 cells induced a 50% reduction in migratory behavior in vitro as compared with control E3 cells (Fig.12K). In vivo, tumors from grafted E3*^{VSIG1}* cells yielded a near 80% reduction in the number of distant lung metastases as compared with E3 tumor controls (Fig.12L). Both *VSIG1* cDNA isoforms- *VSIG1-V1* and *VSIG1-V2-* produced similar results (Fig.12L). VSIG1 gain-of-function also restricted metastatic behavior in E6 clone cells, in the primary cultures CC36 and mCC11 (which do not express *VSIG1,* not shown; Varnat et al., 2009), and in the DLD1 cell line (Fig.12M,N).

### VSIG1 globally promotes NONMETCL and represses METSP states

To investigate a possible relationship between VSIG1 function and the spiked-scRNAseq results the inventors first asked where genes upregulated in C11*^{shVSIG1}* vs. C11^{control} cells and also enhanced in more (E3, E6) vs. less (C11, F3) metastatic cells, localized in the CC14 map. Such genes were found to mark MET cells and conversely, genes with the opposite profiles were prominently expressed by NONMET cells. The restriction of metastatic behavior by VSIG1 (see above) thus appears to correlate with its restriction of pro-metastatic states.

The inventors then determined and analyzed the scRNAseq map of C11*^{shVSIG1}* and used it to localize the expression of genes differentially expressed in the C11*^{shVSIG1}* vs. control. In addition to cell cycle-dependent clustering of the bulk, hereafter the C11*^{shVSIGI}*CL, a side population (hereafter the C11*^{shVSIG}*SP) was detected in G1 (Fig.13A), paralleling the NONMETSP of CC14 (Fig.10A). Mapping the top fifty differentially enhanced genes from the C11*^{shVSIG1}* vs. C11^{control} RNAseq analysis revealed that nearly 90% of these were detected in the C11*^{shVSIG1}*SP, of which 35% were exclusively detected in this subpopulation. These results showed a high congruency of scRNAseq and RNAseq data although only a few genes showed a large percentage of positive cells in the former, likely due to its different coverage depth. The nature of VSIG1-dependent genes prominently expressed by the SP suggested that VSIG1 represses calcium binding (PRKCG, SYT17), microtubule/dendrite localization (MAP2, TUB1A1), signaling (GLI1, TAZ, PDGFA ligand and the WNT antagonist NKD1), migration/protein trafficking (TUB1A1, CD99) and vesicle trafficking (TUB1A1, PCLO, SYT17, KIF19, CAV1) functions.

Mapping the expression of genes prominent in the C11*^{shVSIG1}*SP revealed the common expression of NONMETSP markers (Figs.14B,17). Surprisingly, the expression of other genes revealed a split of this SP into two parts. One continued to express the Goblet differentiation markers *SPINK4* and *REG4* whereas the other displayed high levels of *TUB1A1* (Fig.13A,B). A search for genes tracking the differential expression of *TUB1A1* and *SPINK4* in the C11*^{svVSIG}*SP showed that the *TUB1A1*⁺ population contained cells expressing *MAP2, CD99* or *CAV1* for instance (Fig.13B). Since *MAP2* and *CD99* are also markers of the METSP in CC14, and *CD99* and *CAV1* are expressed in both the METCL and the METSP, kd of *VSIG1* might induce NONMETCL (C11) cells to acquire METSP characteristics.

### Interference with VSIG1 function in NONMET C11 cells engenders a METSP-like SP

To test if the C11*^{shVSIG1} TUB1A1*⁺ population might transfate to a CC14 METSP state the inventors asked if CC14 cells that migrate, which resemble the METSP, express *TUB1A1*⁺ markers (Fig.13C). All C11*^{shVSIG1} TUB1A1*⁺ markers tested but two were found at least equally represented and three - *HDAC9, KIF19* and *MAP2* - showed an enrichment >2-fold in migratory cells (Fig. 13C). In contrast, the C11*^{shVSIG1}SPINK4⁺* markers *SPINK4* and *REG4* were strongly underrepresented in the migratory population (Fig.13C). Whereas not enough C11 or C11*^{shVSIG1}* cells crossed the filter for analyses, *HDAC9, KIF19* and *MAP2* expression may thus mark SP migratory cell states that have acquired pro-metastatic characteristics following interference with VSIG1.

In this case, one would predict that global transcriptomic analyses should map C11*^{shVSIG1} TUB1A1*⁺ cells in the neighborhood of CC14 METSP cells. To test this possibility, the inventors merged the CC14 spike-scRNAseq (Fig.10) with the C11*^{shVSIG1}* scRNAseq (Fig. 13) data using the Scanorama software. The resulting map (Fig. 13D) showed that the bulk of C11*^{shVSIG1}* cells segregated from the NONMETCL and, importantly, from the C11 spike. However, they did not merge with the METCL where the E3 and B2 spikes home. The C11*^{shVSIG1}*SP split into two main populations separate from the bulk of C11*^{shVSIG1}* cells: one mapping close to NONMETSP cells and, importantly, the other one mapping close to METSP cells (Fig.13D). For example, *SPINK4*⁺ C11*^{shVSIG1}* cells mapped close to NONMETSP cells whereas rare *MAP2*⁺, *HDAC9*⁺ or *KIF19*⁺ C11*^{shVSIG1}* cells mapped close to the METSP (Fig. 13E,F).

These unsupervised transcriptomic analyses suggest that C11*^{shVSIG1}*SP^{*TUB1A1*+} cells, which derive from NONMETCL C11 cells, appear to acquire METCL/SP states. In addition to changes in the SP, genes widely upregulated in C11*^{shVSIG1}*CL cells included *CAV1* and *CAV2* (Fig. 13G), which normally mark the METCL/SP (Fig. 10E), further supporting the acquisition of metastatic traits by NONMETCL C11 cells with compromised VSIG1 function. Since MET and NONMET clusters are genetically distinguishable (Fig.9D,E), the results suggest that a subset of NONMET (C11*^{shVSIG1}*SP^{*TUB1A1*+}) cells transfate to become metastatic METCL/SP-like cells in the absence of VSIG1, and that VSIG1 acts as a global metastatic suppressor.

### Interconversion of VSIG1 positive and negative cells

The experiments presented above raised the question of how could a membrane-bound factor detected at any one point in a fraction of cells (Fig.11J-M) have a global impact (Figs. 12, 13). The inventors could envision two possibilities, a wider expression than so far detected and non-cell autonomous effects. To test the first possibility, the inventors performed FACS analyses, which revealed endogenous VSIG1 positive cells spanning from high to low expressors in CC14, C11 and F3 with 6, 20 and 12% VSIG1^{high} cells, respectively (Figs.14A,B,21A and not shown). Sorted positive (high) and negative populations were plated separately and cultured. Surprisingly, within a few days cells interconverted (Fig.14C,D,21A): VSIG1 positive CC14 cells rapidly engendered negative cells, and VSIG1 negative C11 cells quickly gave rise to positive progeny. After 1 week, the percentages of negative and positive cells generated seemed to stabilize with a combined profile (Fig.21C,D) resembling that of the starting population (Fig.14A,B), arguing that such profile is cell type-dependent. The data suggest most NONMETCL cells will express or have expressed VSIG1 at some point due to the existence of a temporal gradient.

To test for possible extrinsic influences by modifying two parameters: i- the recovery kinetics of VSIG1 expression in FACS sorted negative cells, and ii- the ratio of positive/negative cells at the end of the mixing experiments using cells with different levels of VSIG1. i- Tracing a 10% spike of FACS sorted C11^{GFP} VSIG1^{negative} cells co-cultured with cells with no detectable VSIG1 (C11*^{shVSIG1}* or E3 cells) showed that the kinetics of VSIG1 protein recovery were the same as in C11*^{control}* cells (Fig.21B). ii- Co-culture of cells with different levels of endogenous VSIG1, C11*^{lacZ}* with F3*^{GFP}* at 1/9 or 9/1 ratios, did not alter their respective stable frequencies of VSIG1⁺ vs. VSIG1⁻ cells (Fig.21C). Thus, the level of VSIG1 does not affect its overall expression in neighbors. Whereas sorted cells showed no difference in clonogenicity (Fig.21D), endogenous VSIG1^{negative} cells from CC14, C11 and F3 populations were 2-to-3-fold more migratory than their VSIG1^{high} counterparts (Fig. 14E), independently corroborating the functional data experimentally altering VSIG1 levels described above.

### Endogenous VSIG1 levels directly correlate with NONMETCL character and inversely with SP identity

To ascribe an identity to endogenous VSIG1 positive cells, finer FACS gating was implemented to separate negative, low, medium and high VSIG1 -expressing cells (Figs.14F,21E). The levels of *VSIG1* mRNA increased stepwise with overall differences of 100-fold in C11 and 500-fold in CC14 (Fig.21E). To track genes that exhibit a similar profile of expression as endogenous VSIG1 the inventors used the STEM (Short Time-series Expression Miner) software. STEM analyses of RNAseq performed on the four C11 FACS sorted populations identified one cluster with three statistically significant profiles and 223 genes inversely correlated with increasing VSIG1 levels (Figs.14G,22A. Enrichment analyses identified groups for membrane, vesicular and signaling processes, and a set of genes responding to YAP1 overexpression in colon cancer cells that included the putative YAP/TAZ functional homologue *VGLL1* (Fig.22B.

To extend these finding the inventors used 143 genes from the VSIG1^{high} vs. VSIG1^{negative} C11 cell comparison with >2 fold decreased expression in the former as compared with the latter. This yielded a much higher enrichment of the set of genes upregulated by YAP overexpression, raising the possibility that VSIG1 represses YAP/TAZ-TEAD signaling. Twenty-five genes were found enhanced >2-fold in the same comparison in addition to VSIG1 but were not enough to call significant predictions.

The gene set of inversely correlated genes with VSIG1 (underexpressed genes in VSIG1^{high} vs. VSIG1^{negative} C11 cells) was also compared with the sets of modulated genes in C11*^{shVSIG1}* vs. C11^{control} cells: fourteen inversely correlated genes included *HEPACAM2* and *PAX4,* whereas only four, including *CEACAM5*/*6,* showed directly correlated responses (Fig.14H).

The inventors next asked where genes upregulated >2-fold in VSIG1^{negative} vs. VSIG1^{high} localized in the scRNAseq maps. The overwhelming proportion of genes inversely tracking VSIG1 levels were found expressed by SP cells, notably in the *TUBA1A*⁺ subcluster (Fig 22C). More specifically, the inventors mapped also *shVSIG1*-modulated genes that track endogenous VSIG1 levels: all fourteen inversely correlated genes largely mapped to the C11*^{shVSIG1}*SP^{*TUB1A1*+} subcluster (Fig.14H and not shown), and those (7/14) that could be mapped in CC14 cells localized to the NONMETSP (not shown).

To test if these genes could mark NONMETSP cells that may acquire METSP-like character in the absence of VSIG1, the inventors further localized inversely and directly correlated genes onto the merged CC14-plus-C11*^{shVSIG1}* Scanorama map. All fourteen inversely correlated genes largely mapped to cells in the vicinity of the METSP, whereas the four directly correlated genes mapped to the NONMETCL (Figs.14J,K,22C and not shown). Similarly, comparison of high, medium and low VSIG1 expressing C11 cells vs. negative siblings, and vs. upregulated genes in *shVSIG1* C11 cells, identified a core set of 7 genes (Fig.14I) that localized to cells in the *TUB1A1*⁺ *shVSIG1*SP. All mapped to cells close to the Scanorama CC14 METSP, with *HEPACAM2* also labeling the NONMETSP (Fig. 14I-K; see Fig.13B). Together, the results of analyses of endogenous VSIG1 levels independently support the idea that VSIG1 acts globally to repress the differentiation of NONMETSP secretory cells, YAP/TAZ-TEAD signaling and METSP-like pro-metastatic states.

### Non-cell-autonomous suppression of high metastatic behavior involves VSIG1

To test if the negative influence of low-metastatic (VSIG1⁺) on high-metastatic (VSIG1⁻) cells described above (Fig.11E,F) could involve a non-cell-autonomous action of membrane-localized VSIG1, C11*^{sVSIG1}* and C11^{control} cells were mixed with E3*^{lacZ}* cells for 3 days before scoring them in transfilter assays tracking only the *lacZ*⁺ cells. Critically, the negative effect of C11 on high E3 metastatic behavior was abolished upon kd of *VSIG1* in C11 (Fig.15A), and the same effect was observed with kd of *VSIG1* in F3 cells (Fig.15B).

Lacking information on possible partners of VSIG1 except perhaps VSIG1 itself, the inventors investigated if low-metastatic cells could induce *VSIG1* expression in co-cultured high-metastatic cells as a plausible mechanism for repression of their high metastatic behavior. Isolation of E3*^{GFP}* cells from the E3*^{GFP}*+C11^{control} mix after co-culture for 3 days revealed that these E3*^{GFP}* cells now expressed detectable *VSIG1* mRNA (2.5-fold by RT-qPCR; Fig.15C). Confocal immunohistochemistry unequivocally identified VSIG1 protein in rare (<1/100) green fluorescent E3*^{GFP}* cells, whereas none was detected in E3*^{GFP}* cells cultured with E3 siblings (Fig. 15D and not shown).

### Partially overlapping VSIG1-dependent intrinsic and non-cell-autonomous programs

Enrichment analyses of the transcriptomes of E3*^{GFP}* cells from E3*^{GFP}*+C11*^{shVSIG1}* vs. E3*^{GFP}*+C11^{control} co-cultures after normalization by E3*^{GFP}*+E3 (Fig.15E), highlighted signaling and cell communication as the main biological processes for the upregulated genes, processes that repeatedly appear in various of the inventors' assays as associated with pro-metastatic states and negatively influenced by VSIG1. The top upregulated genes in E3 cells in these co-culture comparisons were *HDAC9, TAZ and TNC: HDAC9* maps to the METSP and is enhanced in migratory cells (Fig.13B,C,E), and TAZ (*WWTR1*) and *TNC* are two highly upregulated genes intrinsically in C11*^{shVSIG1}* (Fig. 12D). The inventors then compared the list of modulated E3 genes in E3*^{GFP}*+C11*^{shVSIG1}* vs. E3*^{GFP}*+C11^{control} co-cultures with the list of modulated genes in C11 cells with *VSIG1* kd vs. sibling controls: Five genes were upregulated and six genes downregulated in common in both intrinsic (in C11) and in non-cell-autonomous (in E3) VSIG1-dependent manners (Fig.15F,22D). Localizing these genes in the CC14-plus-C11*^{shVSIG1}* Scanorama map was hampered by the relatively low depth of scRNAseq, although the available data (e.g. for *HDAC9;* Fig. 13) supported the link between VSIG1 kd and NONMETCL downregulated genes as well as VSIG1 kd and METSP upregulated genes.

### VSIG1 regulates YAP/TAZ-TEAD signaling intrinsically and non-cell-autonomously

The inventors then inquired if YAP/TAZ/VGLL1 transcriptional output, as measured with a TEAD binding-site->luciferase reporter, could be correlated with higher metastatic behavior. Indeed, E3 cells had 5-fold higher TEAD reporter activity than C11 cells (Fig. 15G). The inventors then asked if increased VSIG1 expression in E3 cells (through expressing the *VSIG1-V1* cDNA) could reduce TEAD reporter activity and this was indeed the case (Fig.15H). Finally, the inventors tested if this effect could also be detected non-cell-autonomously by co-culturing E3 cells bearing the TEAD reporter (E3*^{TEADRep}*) along with E3^{control}, C11^{control} or C11*^{shVSIG1}* cells. Importantly, E3*^{TEADRep}* reporter activity was reduced in cells co-cultured with C11^{control} but not with C11*^{shVSIG1}* cells (Fig. 15I), arguing that YAP/TAZ-TEAD activity inversely correlates with and is responsive to VSIG1 function. As TAZ/TEAD activity has been linked with cell junctions and metastases, the inventors' data places VGLL1/TAZ-TEAD activity as an important target of the repressive action of VSIG1 on pro-metastatic states. Consistently, 9/22 YAP/TAZ targets in various cancers were upregulated in C11*^{shVSIG1}* cells by >1.5-fold in RNAseq analyses, including 2/4 direct targets . Similarly, 5/7 YAP/TAZ targets in other contexts tested by rt-qPCR (*AREG, AXL, CYR61, UCA1, BMP4;* but not *CTGF* or *GDF15*) were also enhanced >1.5-fold by *VSIG1* kd.

### High YAP/TAZ-TEAD activity rescues the repression of E3 cell migration by VSIG1

In this case, activation of TEAD signaling would be predicted to be epistatic to VSIG1 and revert the repressive effect of the latter on E3 cell migration. To enhance TEAD activity the inventors used an activated form of YAP (YAP^{S127A}). Its expression in E3 cells enhanced endogenous TEAD activity 20 times as assessed by a TEAD->Luciferase reporter (Fig.15J). When co-expressed, YAP^{S127A} rescued the restriction on E3 cell migration in transfilter assays imposed by VSIG1 (Fig.15J).

### Discussion

The inventors have developed *spiked-scRNAseq,* a system that links expressed genotype and function taking advantage of clones that exhibit stable phenotypes, in the present case metastatic behavior. Data from this approach, together with the analysis of tumor cell interactions and of VSIG1 function, reveal that the imposition of extrinsic (cell interactions) influences on intrinsic (genetic/transcriptomic) variety is a driving force for the emergent construction of pro-metastatic heterogeneity. These studies build on a two-state interactive cellular model for the control of metastatic proclivities and show the dynamic origin of pro-metastatic states and therefore of metastases.

The data obtained by the inventors indicates that VSIG1 acts both cell-autonomously and non-cell-autonomously to inhibit the development of metastatic states. The inventors envision a cell-autonomous and cell-type-specific ebb and flow of VSIG1 where most if not all tumor cells will express this protein at different times. Indeed, the stochastic interconversions and cell-type specific frequencies of conversion of VSIG1 positive and negative cells fit a Markov model, which predicts the appearance of intrinsic equilibrium.

This scenario with high VSIG1 expression dynamics may resolve an apparent contradiction: whereas some inversely related genes (also enhanced by VSIG1 kd) have anti-metastatic function in different contexts others have pro-metastatic activities. The inventors suggest that as VSIG1 levels fluctuate, the expression of VSIG1 -responsive pro- and anti-metastatic genes follow diverse kinetics. Visually, this may be akin to punctually sampling overlapping waves of expression that have different periods and amplitudes. It could also explain an increase in *VSIG1* levels upon metastatic reprogramming (not shown) where it might act as a feedback inhibitor.

VSIG1 function from the membrane may involve various factors similarly localized that transcriptionally respond to alterations in VSIG1 levels, including several protocadherins, Ig-domain containing membrane components and other members of the VSIG family (VSIG2, GPA33). It may also involve intracellular vesicular trafficking molecules such as multiple CAVEOLINS and RABs and changes in DYNEINS and KINESINS. This, together with the requirement for CAV1, provides a functional link between pro-metastatic states and vesicular trafficking. As VSIG1 and other family members can suppress T cell activation, it remains possible VSIG1 may also affect stromal or immune system components in a growing cancer.

More specifically, the data support the idea that VSIG1 controls pro-metastatic states in part by repressing YAP/TAZ-TEAD membrane-to-nucleus signaling. This is in line with the ability of different plasma membrane and junctional components to regulate YAP/TAZ-TEAD function. It is also consistent with the regulation of *CAV1, BMP4* and other, but not all, YAP/TAZ-TEAD targets by VSIG1.

Beyond YAP/TAZ-TEAD, however, additional signals would appear required to drive high metastases, for instance in E3 vs. B2 cells, as both lack VSIG1 and have similar TEAD->Luciferase reporter levels (not shown). In this sense, TMED9-TGFα-GLI1 signaling has been implicated in colon cancer metastases (Varnat et al., 2009), *TMED9* is prominent in the METSP, *GLI1* is enhanced in migratory cells and can upregulate *CAV1.* However, alteration of VSIG1 does not affect *GLI1* levels or reporter activity (not shown). As TEAD⁺/GLI⁺ cancer states have been described, the inventors suggest that a dual YAP/TAZ/VGLL1-TEAD⁺/TMED9-TGFα-GLI1⁺ state is responsible for high metastatic behavior, consistent with the requirement of both YAP and GLI1 (Varnat et al., 2009) for CC14 metastases.

The inventors find that VSIG1 participates in the restriction of colon cancer metastases and that its redeployment is sufficient to hamper metastatic behavior from multiple colon cancer cell types. Unlike in gastric cancer, it does not affect colon cancer cell proliferation or tumor growth. Inducing the expression of VSIG1, mimicking its function or modulating downstream effectors thus holds promise to reduce metastases by inhibiting pro-metastatic states.

Generally, spiked-scRNAseq, linking single-cell omics to stable or traceable phenotypes, can be applied to a wealth of systems: from cancer clones to post-mitotic neurons and from endogenous to perturbation (e.g. gene alteration, pathogen- or drug-induced) phenotypes. The inventors note that this strategy does not require a priori knowledge of molecular identifiers. For instance, the inventors' proof of principle with Vemurafenib opens the possibility to use spiked-scRNAseq to better understand drug effects in individual cell subpopulations aiming to select effective and personalized treatments.

### Example 4

### Key Resource Table

| REAGENT or RESOURCE | SOURCE | IDENTIFIER |
|---|---|---|
| Antibodies | | |
| VSIG1 monoclonal antibody | R&D | Cat# MAB4818 |
| E-cadherin antibody | Cell Signaling Technology | Cat# 3195 |
| EZRIN antibody | Cell Signaling Technology | Cat# 3145 |
| ZO-1 antibody | Cell Signaling Technology | Cat# 13663 |
| VSIG1 polyclonal antibody | Sigma Aldrich | Cat# H PA03631 0 |
| Secondary antibodies | Jackson Immuno-Research, Life Technologies | Cat# 111-485-003 |
| | | Cat# A31570 |

| Chemicals, Peptides, and Recombinant Proteins | | |
|---|---|---|
| DMEM/F12 | Sigma Aldrich | Cat# D8062 |
| Puromycin | Gibco | Cat# A 11138-03 |
| TRIzol | Life technologies | Cat# 15596018 |
| X-gal | AppliChem | Cat# A1007,0001 |
| Alkaline phosphatase Substrate | Sigma Aldrich | Cat# B5655 |
| BMP4 ligand | Signal Chem | Cat# B853-40N |
| EREG ligand | Sigma Aldrich | Cat# SRP3033 |
| B27 | Gibco | Cat# 17504-044 |
| Propidium iodide | Sigma Aldrich | Cat# P4170 |
| Vemurafenib | Sellenchem | Cat# S1267 |
| DMSO | Sigma Aldrich | Cat# G1640 |
| Lipofectamine 2000 | Invitrogen | Cat# 11668-019 |
| Immu-Mount | Thermo Fisher | Cat# 9990402 |
| TritonX-100 | Sigma | Cat# T-8532 |
| Tween 20 | Applichem | Cat# A1389,0500 |
| Opti-MEM | Gibco | Cat# 31985-062 |
| HBSS | Gibco | Cat# 14175-053 |
| Collagenase | Worthington | Cat# 4176 |
| Trypsine-EDTA | Sigma Aldrich | Cat# T3924 |
| Hyaluronidase | Sigma Aldrich | Cat# H-3884 |
| DNAse I | Roche | Cat# 10104159001 |
| RNAse A | Thermofisher | Cat#12091021 |

| Critical Commercial Assays | | |
|---|---|---|
| iQ SYBR super Mix | BioRad | Cat#1708882 |
| DNeasy Blood & Tissue Kit | Qiagen | Cat# 69504 |
| Go Taq G2 DNA polymerase | Promega | Cat# M7841 |
| Dual-Luciferase Reporter Assay System | Promega | Cat# E1910 |
| ChromiumTM Single Cell 3' Library and Gel Bead Kit v2 | 10xGenomic | Cat# 120267 |
| ChromiumTM Single Cell A Chip Kit | 10xGenomic | Cat# 1000009 |
| Mouse Cell Depletion kit | Miltenyi Biotec | Cat# 130-104-694 |
| Superscript II | Invitrogen | Cat# 18064-014 |
| Truecut CAS9 protein V2 | Thermofisher | A36498 |
| | | |
| Rainbow beads calibration 8peaks | Biolegend | Cat# 422903 |

| Experimental Models: Cell Lines | | |
|---|---|---|
| DLD1 | ATCC | ATCC^{®} CCL-221 |
| CC14 and derived clones | Primary culture made in the lab (Varnat et al., 2009) | N/A |
| CC36 and derived clones | Primary culture made in the lab (Varnat et al., 2009) | N/A |
| mCC11 | Primary culture made in the lab (Varnat et al., 2009) | N/A |

| Experimental Models: Organisms/Strains | | |
|---|---|---|
| NUDE (NMRI-foxn1 nu/nu) female mice | Janvier Laboratories | N/A |
| Oligonucleotides | | |
| Primers for genomic PCR and sequening (5'-3') | | |
| XRCC5 fw: CAGAGTTGGGCTGTCCGATT | Herein | N/A |
| XRCC5 rv: CCTAGCCCCAACCAACAACT | Herein | N/A |
| GBF1 fw: CTGCAGTCAGAGGCGATCC | Herein | N/A |
| GBF1 rv: GACTCTTTTTGATAAAGCTGGGC | Herein | N/A |
| GDF11fw: CTTATCAGCGGACCCAGCAG | Herein | N/A |
| GDF11 rv: TATCTCCTCAGGGCCAGGTTA | Herein | N/A |
| AXL fw: CTTGCATGCAGTGAACAGCC | Herein | N/A |
| AXL rv: ACACATGTGCACGTAACTCAC | Herein | N/A |
| NEO1fw:TTTTTGTAGGAGCCAGCATTCG | Herein | N/A |
| NEO1 rv:AACAGTGGCCACACACTGATAA | Herein | N/A |
| ROR2 fw: CTCACATTGCTCACTGGGCT | Herein | N/A |
| ROR2 rv: CGGCAAGTTCTCCATCGACT | Herein | N/A |
| DNAH5 fw: CACAGGGAATAAAAGGAGAGAAACT | Herein | N/A |
| DNAH5 rv: TTGCCTAACCCAGCCTACAC | Herein | N/A |
| CH9 fw: TCCTTCAGCACACTTCCACA | Herein | N/A |
| CH9 rv: TGGGGTTTAGGATATGCTGAGT | Herein | N/A |
| Primers for RT-qPCR (5'-3') | | |
| AKAP12 FW: CCGCTAAGCTGATCTCCTGT | | N/A |
| AKAP12 RV: CATCTTCAGAGTCTCTCTGTCCAA | Herein | N/A |
| CAV1 FW:CAGGCTTGTAACCTTTACAGGAC | Herein | N/A |
| CAV1 RV:CATAGATGCTTAGTCCCTCATGC | Herein | N/A |
| GLI1 FW:AGCGTGAGCCTGAATCTGTG | (Varnat et al., 2009) | N/A |
| GLI1 RV:CAGCATGTACTGGGCTTTGAA | (Varnat et al., 2009) | N/A |
| IL18 FW:TCTTCATTGACCAAGGAAATCGG | Herein | N/A |
| IL18 RV:TCCGGGGTGCATTATCTCTAC | Herein | N/A |
| TGFB2 FW: CAGCACACTCGATATGGACCA | Herein | N/A |
| TGFB2 RV:CCTCGGGCTCAGGATAGTCT | Herein | N/A |
| CAV2 FW:GCCCTCTTTGAAATCAGC | Herein | N/A |
| CAV2 RV:CAAGTATTCAATCCTGGCTC | Herein | N/A |
| CSAG1 FW:ACACTGGTCTGGTGAAGATGTC | Herein | N/A |
| CSAG1 RV:TGTTGATGGGTGGTTGTTGG | Herein | N/A |
| MAGEA2 FW:TCCAGCAACCAAGAAGAGGAG | Herein | N/A |
| MAGEA2 RV:TGATTGCTGCTTGGAACTCG | Herein | N/A |
| MAGEA12 FW: GTGGTCCTAAGATCTACCAAGCA | Herein | N/A |
| MAGEA12 RV:AGGGCAGCAGGTAGGAGTG | Herein | N/A |
| LGR5 FW:GGAGCATTCACTGGCCTTTA | (Varnat et al., 2009) | N/A |
| LGR5 RV:CTGGACGGGGATTTCTGTTA | (Varnat et al., 2009) | N/A |
| MAGEA3 FW:AGAAATGCTGGGGAGTGTCG | Herein | N/A |
| MAGEA3 RV:AAGACCAGCTGCAAGGAACT | Herein | N/A |
| IL8 FW:GTGCAGTTTTGCCAAGGAGT | Herein | N/A |
| IL8 RV:CTCTGCACCCAGTTTTCCTT | Herein | N/A |
| SCEL FW:GTGCTCAACCGACATAATTCCC | Herein | N/A |
| SCEL RV:TGTCATCAGAACTGTACCGACTA | Herein | N/A |
| SDCBP2 FW:GGGGCTCTTTGTGCAGTTG | Herein | N/A |
| SDCBP2 RV:AGTCACGCCCGTCAATCTG | Herein | N/A |
| ADM2 FW:CTGAGCCCCATCTGAAGCC | Herein | N/A |
| ADM2 RV:CAGCACTGCGTGTAGACCAG | Herein | N/A |
| RBPM2 FW:AAGACAGCCTGTTGGTTTTGT | Herein | N/A |
| RBPM2 RV:CGAATACCGTTCAGCGCATT | Herein | N/A |
| sXBP1 FW:CTGAGTCCGAATCAGGTGCAG | Herein | N/A |
| sXBP1 RV:ATCCATGGGGAGATGTTCTGG | Herein | N/A |
| IL32 FW:GAAGACTGCGTGCAGAAGGT | Herein | N/A |
| IL32RV:CTCTGCCAGGCTCGACAT | Herein | N/A |
| EREG FW:TTGTTTGCATGGACAGTGCAT | Herein | N/A |
| EREG RV: CCCACTTCACACCTGCAGTAGTT | Herein | N/A |
| ANXA13 FW: GCTAAAGCGAGCAGTCCTCAG | Herein | N/A |
| ANXA13 RV: GTCCTGCCCGATAAGATTTCAA | Herein | N/A |
| VGLL1 FW: CCAAAGGCAAACAGAAGCCTA | Herein | N/A |
| VGLL1 RV: CATCACACCTTCACTCTGACTC | Herein | N/A |
| WWTR1 FW: ATTCGAATGCGCCAAGAG | Herein | N/A |
| WWTR1 RV: AACTGGGGCAAGAGTCTCAG | Herein | N/A |
| HSPA5 FW:CATCACGCCGTCCTATGTCG | Herein | N/A |
| HSPA5 RV:CGTCAAAGACCGTGTTCTCG | Herein | N/A |
| DNAJC15 FW:TTGCAGGTCGCTACGCATTT | Herein | N/A |
| DNAJC15 RV:CCAGCTTCTCGCCTACTCATTT | Herein | N/A |
| BGN FW:ACACACCGGACAGATAGACG | Herein | N/A |
| BGN RV:CAGGGTGAAGTCCCAGAAGC | Herein | N/A |
| VSIG1 FW:AGCAGATAAGCCAGGCAAAC | Herein | N/A |
| VSIG1 RV:GCAAGGCAGCTTAGGATCAG | Herein | N/A |
| VSIG1_V1 FW: GCTGGACCTCTGAGATTTACTT | Herein | N/A |
| VSIG1_V1 RV: TACCTGGATCGTTGGACCCT | Herein | N/A |
| VSIG1_V2 FW: AGGAGATGGAGCCAATTTCTATTTAC | Herein | N/A |
| VSIG1_V2 RV: TACCTGGATCGTTGGACCCT | Herein | N/A |
| HES6 FW:AACTTTTAGGGACCCTGCAG | Herein | N/A |
| HES6 RV:CGCTGGCAGCTCTACAAAATTC | Herein | N/A |
| SPINK4 FW:CAGTGGGTAATCGCCCTGG | Herein | N/A |
| SPINK4 RV:CACAGATGGGCATTCTTGAGAAA | Herein | N/A |
| TFF3 FW:CTCCAGCTCTGCTGAGGAGT | Herein | N/A |
| TFF3 RV:CTCCAGCTCTGCTGAGGAGT | Herein | N/A |
| HDAC9 FW:AGTAGAGAGGCATCGCAGAGA | Herein | N/A |
| HDAC9 RV:GGAGTGTCTTTCGTTGCTGAT | Herein | N/A |
| KIF19 FW:ACCCTCATCGCCCATAAAGTG | Herein | N/A |
| KIF19 RV:TCGAACAGGTAGGACTTCTCC | Herein | N/A |
| MAP2 FW:CCAATGGATTCCCATACAGG | Herein | N/A |
| MAP2 RV:CTGCTACAGCCTCAGCAGTG | Herein | N/A |
| ENOX FW:CGATAGACACGACCCAGCTC | Herein | N/A |
| ENOX RV:CGCATCCTCTGCTTGCATTC | Herein | N/A |
| PDGFA FW:GCAAGACCAGGACGGTCATTT | Herein | N/A |
| PDGFA RV:GGCACTTGACACTGCTCGT | Herein | N/A |
| NKD1 FW:ACTTTCGGCTGGAAGTGGC | Herein | N/A |
| NKD1 RV:CAGGGTTCGCTCACTCTCTC | Herein | N/A |
| TUBA1A FW:GCAACAACCTCTCCTCTTCG | Herein | N/A |
| TUBA1A RV:GAATCATCTCCTCCCCCAAT | Herein | N/A |
| REG4 FW:CCTTGCACTAGCTACATCCTCA | Herein | N/A |
| REG4 RV:AAAACCATCCAGGAGCACAG | Herein | N/A |
| RCOR2 FW:CACTCGCACGACAGCATGAT | Herein | N/A |
| RCOR2 RV:CATCGCAATGTACTTGTCAAGC | Herein | N/A |
| ZEB1 FW:TTACACCTTTGCATACAGAACCC | Herein | N/A |
| ZEB1 RV:TTTACGATTACACCCAGACTGC | Herein | N/A |
| SYT2L FW:GGAGGTCTTCGTATTGGCTTTG | Herein | N/A |
| SYT2L RV:CCATCTTCTCCCAGAGAGCAA | Herein | N/A |
| MSX1 FW:GGAACCATATCTTCACCTGCGT | Herein | N/A |
| MSX1 RV:AGTTCTCCAGCTCGCTCAGC | Herein | N/A |
| BMP4 FW:GATCCACAGCACTGGTCTTG | Herein | N/A |
| BMP4 RV:GGGATGCTGCTGAGGTTAAA | Herein | N/A |
| GLRX FW:CCCATCAAACAAGGGCTTCTG | Herein | N/A |
| GLRX RV:CTGCATCCGCCTATACAATCTT | Herein | N/A |
| DKK1 FW:TCCGAGGAGAAATTGAGGAA | Herein | N/A |
| DKK1 RV:CCTGAGGCACAGTCTGATGA | Herein | N/A |
| EPCAM FW:AATCGTCAATGCCAGTGTACTT | Herein | N/A |
| EPCAM RV:TCTCATCGCAGTCAGGATCATAA | Herein | N/A |
| S100A14 FW:GAGACGCTGACCCCTTCTG | Herein | N/A |
| S100A14 RV:CTTGGCCGCTTCTCCAATCA | Herein | N/A |
| RAB25 FW:ATCGGCGAATCAGGTGTGG | Herein | N/A |
| RAB25 RV:TAGGTCTGGTGCTTGGTTAGG | Herein | N/A |

| Recombinant DNA | | |
|---|---|---|
| Phosphatase-expressing retrovector (AP) (CMV-PLAP) | Addgene | Cat# 15164 |
| *lac*Z-expressing lentivector | Varnat et al., 2009 | N/A |
| shVSIG1 (26) | Sigma Aldrich | TRCN0000432026 |
| shVSIG1 (95) | Sigma Aldrich | TRCN0000158295 |
| shCAV1 | Sigma Aldrich | TRCN0000008002 |
| pLKO.1-puromycin | Addgene | Cat# 8453 |
| *VSIG1*variant1-*GFP* | Origene | Cat# RC230056L2 |
| *VSIG1*variant2-*GFP* | Origene | Cat# RC207391 L2 |
| 8XGTIIC-luciferase | Addgene | Cat# 34615 |
| S127A YAP | Addgene | Cat# 27370 |
| Renilla pGL4.74 | Promega | Cat# E6921 |
| pBOB-EF1-FastFUCCI-Puro | Addgene | Cat# 86849 |

| CRISPR/CAS9 | | |
|---|---|---|
| Truecut CAS9 protein V2 | Thermofisher | A36498 |
| Geneart genome clivage detection ki | Thermofisher | A24372 |
| Trueguide TracrRNA | Thermofisher | A35506 |
| Trueguide crRNA | Thermofisher | A35512 |

### SEQUENCES

SEQ ID NO: 115
ATGGTGTTCGCATTTTGGAAGGTCTTTCTGATCCTAAGCTGCCTTGCAGGTCAGGTTAGTGTGGTGCAAG
TGACCATCCCAGACGGTTTCGTGAACGTGACTGTTGGATCTAATGTCACTCTCATCTGCATCTACACCAC
CACTGTGGCCTCCCGAGAACAGCTTTCCATCCAGTGGTCTTTCTTCCATAAGAAGGAGATGGAGCCAATT
TCTCACAGCTCGTGCCTCAGTACTGAGGGTATGGAGGAAAAGGCAGTCAGTCAGTGTCTAAAAATGACGC

ACGCAAGAGACGCTCGGGGAAGATGTAGCTGGACCTCTGAGATTTACTTTTCTCAAGGTGGACAAGCTGT
AGCCATCGGGCAATTTAAAGATCGAATTACAGGGTCCAACGATCCAGGTAATGCATCTATCACTATCTCG
CATATGCAGCCAGCAGACAGTGGAATTTACATCTGCGATGTTAACAACCCCCCAGACTTTCTCGGCCAAA
ACCAAGGCATCCTCAACGTCAGTGTGTTAGTGAAACCTTCTAAGCCCCTTTGTAGCGTTCAAGGAAGACC
AGAAACTGGCCACACTATTTCCCTTTCCTGTCTCTCTGCGCTTGGAACACCTTCCCCTGTGTACTACTGG
CATAAACTTGAGGGAAGAGACATCGTGCCAGTGAAAGAAAACTTCAACCCAACCACCGGGATTTTGGTCA
TTGGAAATCTGACAAATTTTGAACAAGGTTATTACCAGTGTACTGCCATCAACAGACTTGGCAATAGTTC
CTGCGAAATCGATCTCACTTCTTCACATCCAGAAGTTGGAATCATTGTTGGGGCCTTGATTGGTAGCCTG
GTAGGTGCCGCCATCATCATCTCTGTTGTGTGCTTCGCAAGGAATAAGGCAAAAGCAAAGGCAAAAGAAA
GAAATTCTAAGACCATCGCGGAACTTGAGCCAATGACAAAGATAAACCCAAGGGGAGAAAGCGAAGCAAT
GCCAAGAGAAGACGCTACCCAACTAGAAGTAACTCTACCATCTTCCATTCATGAGACTGGCCCTGATACC
ATCCAAGAACCAGACTATGAGCCAAAGCCTACTCAGGAGCCTGCCCCAGAGCCTGCCCCAGGATCAGAGC
CTATGGCAGTGCCTGACCTTGACATCGAGCTGGAGCTGGAGCCAGAAACGCAGTCGGAATTGGAGCCAGA
GCCAGAGCCAGAGCCAGAGTCAGAGCCTGGGGTTGTAGTTGAGCCCTTAAGTGAAGATGAAAAGGGAGTG
GTTAAGGCA

SEQ ID NO: 116
ATGGTGTTCGCATTTTGGAAGGTCTTTCTGATCCTAAGCTGCCTTGCAGGTCAGGTTAGTGTGGTGCAAG
TGACCATCCCAGACGGTTTCGTGAACGTGACTGTTGGATCTAATGTCACTCTCATCTGCATCTACACCAC
CACTGTGGCCTCCCGAGAACAGCTTTCCATCCAGTGGTCTTTCTTCCATAAGAAGGAGATGGAGCCAATT
TCTATTTACTTTTCTCAAGGTGGACAAGCTGTAGCCATCGGGCAATTTAAAGATCGAATTACAGGGTCCA
ACGATCCAGGTAATGCATCTATCACTATCTCGCATATGCAGCCAGCAGACAGTGGAATTTACATCTGCGA
TGTTAACAACCCCCCAGACTTTCTCGGCCAAAACCAAGGCATCCTCAACGTCAGTGTGTTAGTGAAACCT
TCTAAGCCCCTTTGTAGCGTTCAAGGAAGACCAGAAACTGGCCACACTATTTCCCTTTCCTGTCTCTCTG
CGCTTGGAACACCTTCCCCTGTGTACTACTGGCATAAACTTGAGGGAAGAGACATCGTGCCAGTGAAAGA
AAACTTCAACCCAACCACCGGGATTTTGGTCATTGGAAATCTGACAAATTTTGAACAAGGTTATTACCAG
TGTACTGCCATCAACAGACTTGGCAATAGTTCCTGCGAAATCGATCTCACTTCTTCACATCCAGAAGTTG
GAATCATTGTTGGGGCCTTGATTGGTAGCCTGGTAGGTGCCGCCATCATCATCTCTGTTGTGTGCTTCGC
AAGGAATAAGGCAAAAGCAAAGGCAAAAGAAAGAAATTCTAAGACCATCGCGGAACTTGAGCCAATGACA
AAGATAAACCCAAGGGGAGAAAGCGAAGCAATGCCAAGAGAAGACGCTACCCAACTAGAAGTAACTCTAC
CATCTTCCATTCATGAGACTGGCCCTGATACCATCCAAGAACCAGACTATGAGCCAAAGCCTACTCAGGA
GCCTGCCCCAGAGCCTGCCCCAGGATCAGAGCCTATGGCAGTGCCTGACCTTGACATCGAGCTGGAGCTG
GAGCCAGAAACGCAGTCGGAATTGGAGCCAGAGCCAGAGCCAGAGCCAGAGTCAGAGCCTGGGGTTGTAG
TTGAGCCCTTAAGTGAAGATGAAAAGGGAGTGGTTAAGGCA

## Claims

1. A method for identifying a cluster of cells associated with a phenotype, wherein the method comprises:
a. spiking a heterogeneous population of cells with a tagged phenotypically characterised single cell; and
b. analysing single cell omics data from said spiked population of cells, wherein presence of the tagged single cell within a cluster of cells in the population identifies said cluster as associated with said phenotype.

2. A method according to claim 1, wherein the single cell omics data is derived from any one or more of genomics, transcriptomics, proteomics, metabolomics and epigenomics analysis.

3. A method according to claim 1 or claim 2, wherein the single cell omics data is derived from transcriptomics analysis.

4. A method according to claim 3, wherein the clusters of cells are determined according to variable gene expression.

5. A method according to any one of claims 1 to 4, wherein:
(a) the heterogeneous population of cells is derived from diseased tissue, optionally
wherein the diseased tissue is cancer; and/or
(b) the single cell is tagged by the incorporation of a heterologous transgene.

6. A method according to any one of claims 1 to 5, wherein the phenotypically characterised cell has a determined:
i) metastatic potential;
ii) cell motility;
iii) invasiveness;
iv) responsiveness to a medicament.

7. A method according to any one of claims 1 to 6, wherein prior to step a. , the method comprises:
i. isolating a single cell from the heterogeneous population of cells; and
ii. phenotypically characterising and tagging the single cell.

8. A method according to any one of claims 1 to 7, wherein the method comprises spiking the heterogeneous population of cells with a first and second single cell each with distinct tags, wherein the first and second single cell have been subject to the same phenotypic characterisation assay to determine a particular phenotype, and wherein the first and second cell are characterised as having different phenotypes based on their behaviour in the phenotypic characterisation assay, and wherein step d) thereby involves the identification of distinct clusters associated with each of the different phenotypes.

9. A method according to claim 8, wherein the phenotype is metastatic potential, and wherein the first cell is characterised as metastatic and the second cell is characterised as non-metastatic, and optionally wherein the cluster identified by the second cell has increased VSIG1 expression relative to the cluster identified by the first cell.

10. A method according to any one of claims 1 to 7, wherein the method comprises spiking the heterogeneous population of cells with a first and second single cell each with distinct tags, wherein the first and second single cell have been subject to a different phenotypic characterisation assay to determine distinct phenotypes, and wherein step d) involves the identification of one or more clusters of cells associated with the distinct phenotypes.

11. A method for preventing cancer metastasis in an individual, wherein the method comprises administering to the individual an agent for inducing VSIG1.

12. A method according to claim 11, wherein:
(i) the administration is intratumoral;
(ii) wherein the metastasis is a primary or secondary metastasis; and/or
(iii) wherein the agent is a polynucleotide encoding VSIG1.

13. A method for determining the efficacy of a medicament, wherein method comprises the identification of one or more clusters of cells in accordance with the method of any one of claims 1 to 10, and wherein the method further comprises spiking and clustering a heterogeneous population of cells which has been subject to treatment with a medicament and assessing whether the proportion of a cluster of cells associated with a particular phenotype changes after treatment with said medicament.

14. A heterogeneous population of cells spiked with a phenotypically characterised and tagged single cell.

15. A heterogeneous population of cells according to claim 14, wherein:
i) the heterogeneous population of cells have been derived from a tumour biopsy, and optionally wherein the tagged single cell has metastatic potential;
ii) the tagged single cell is autologous to the population of cells; and/or
iii) the single cell is tagged with a heterologous transgene.
